# EUROPEAN PATENT APPLICATION

(11) **EP 4 538 280 A1**
(43) Date of publication of application: **16.04.2025**
(21) Application number: 23819260.3
(22) Date of filing: 09.06.2023
(51) Int. Cl.: C07J 43/00, A61K 31/58, A61P 25/00

(54) **19-NOR-C3,3-DISUBSTITUTED C21-AZACYCLO-SUBSTITUTED STEROID AND METHOD FOR USING SAME**

(30) Priority: 09.06.2022 CN 202210646194; 01.10.2022 CN 202211217180; 09.01.2023 CN 202310026184
(71) Applicant: Shandong Luye Pharmaceutical Co., Ltd., Yantai, Shandong 264670 (CN)
(72) Inventor: TIAN, Jingwei, Yantai, Shandong 264670 (CN); MA, Mingxu, Yantai, Shandong 264670 (CN); YE, Liang, Yantai, Shandong 264670 (CN); LI, Chunmei, Yantai, Shandong 264670 (CN); XU, Hengwei, Yantai, Shandong 264670 (CN); WANG, Wenyan, Yantai, Shandong 264670 (CN); ZHANG, Rui, Yantai, Shandong 264670 (CN)
(74) Representative: karo IP
(86) International application number: PCT/CN2023/099369
(87) International publication number: WO 2023/237097

(57) **Abstract**

The present invention provides a 19-nor-C3,3-disubstituted C21-azacyclo-substituted steroid and a method for using same. In particular, the present invention relates to a compound represented by general formula (I), a method for preparing same, a pharmaceutical composition containing the compound, and use thereof as a GABA_{A}R positive allosteric modulator in prevention and/or treatment of various CNS-related diseases, such as treatment of sleep disorder, mood disorder, insomnia, anxiety, depression, traumatic brain injury (TBI), stress and epilepsy, etc. Substituents in the general formula (I) are defined the same as in the description.

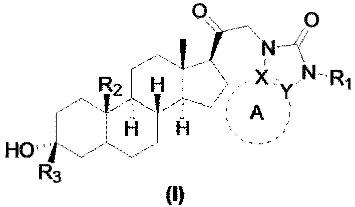

## Description

### TECHNICAL FIELD

The present disclosure relates to a class of 19-nor C3,3-disubstituted C21-azacyclo-substituted steroids and a preparation method therefor, as well as use thereof in the field of GABA_{A} receptor-related diseases. Specifically, the present disclosure relates to compounds of formula (I) and pharmaceutically acceptable salts thereof.

### BACKGROUND

Depression is a common psychological disease clinically characterized mainly by persistent and long-lasting low mood. It is the most important type of psychological disease in modern people. While it is not unusual to occasionally feel sad, these feelings are typically short-lived and disappear after a few days for most people. When depression manifests as a disorder, the patient's symptoms can interfere with daily life and normal functioning. Depression is a leading cause of disability and can result in a variety of symptoms, including not only low mood but also subsequent changes in endocrine and immune functions, thereby increasing susceptibility to physical illnesses. In the worst case, depression can lead to suicide. Depression can be categorized into major depressive disorder (MDD), persistent depressive disorder (PDD, also known as dysthymic disorder), psychotic depression, postpartum depression (PPD), and seasonal affective disorder (SAD).

In 2018, there were 223.1 million prevalent cases of MDD worldwide, with projections suggesting that the number will increase to 248.9 million by 2027, showing an 11.6% increase. It is estimated that in 2018, Asia had the highest number of prevalent cases (130.7 million), while Oceania had the fewest (1.5 million).

PPD is one of the most common neurobiological complications following childbirth, affecting 13% of women after delivery. Some women may experience it as early as the late pregnancy period. Severe cases occur in about 5-10% of patients and are a leading cause of maternal mortality. Given the potentially devastating impact of PPD on both patients and their children and families, this unmet need is particularly high.

Neuroactive steroids (NAS) are the most effective regulators of neuronal excitability. Neurosteroids have been shown to influence the functioning of the central nervous system (CNS) primarily through allosteric regulation of the GABA_{A} receptor (GABA_{A}R). GABA receptors include GABA_{A}, GABA_{B}, and GABA_{C}. The GABA_{A} and GABA_{B} receptors are generally associated with mood disorders. The GABA_{A} receptor is a pentameric chloride ion channel receptor consisting mainly of two α subunits (α1-α6), two β subunits (β1-β3), and an additional subunit (γ1-γ3, δ, ε, π, or 0). After the GABA_{A} receptor on the neuron cell membrane binds to GABA, it causes the chloride ion channel to open (the influx of chloride ions into the membrane), hyperpolarizing the neuron cell membrane and causing inhibition of neurons. The unique subunit composition determines the biophysical and pharmacological characteristics of the channel and influences its location at synapses or extrasynaptic sites. In MDD patients, reduced levels of GABA have been observed in plasma, cerebrospinal fluid, or cortical tissue, along with decreased GABA synthetase expression, altered GABA_{A}R subunit expression, and reduced numbers of GABAergic interneurons. In women at risk of developing PPD, the perinatal GABA levels have been shown to be relatively low, and GABA levels are negatively correlated with depression scores. Positive allosteric regulators of GABA_{A}R can facilitate the binding of GABA to the GABA_{A} receptor, enhancing GABA's inhibitory effect.

The 19-nor-C3,3-disubstituted C21-azacyclo-substituted steroids, as improved steroids, have a relatively good effect of regulating brain excitability and can be used to prevent and treat depression and other CNS-related diseases. The compounds, compositions, and methods described herein relate to this purpose.

### SUMMARY

In one embodiment of the present disclosure, provided is a compound of formula (I) or a pharmaceutically acceptable salt thereof: 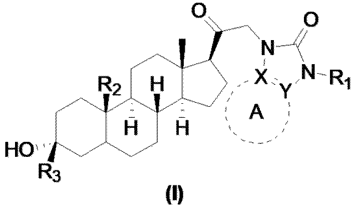 wherein,
(1) X is C, and Y is N; or X is N, and Y is C; or X and Y are simultaneously C;
   R₁ is selected from the group consisting of H, C₁₋₆ alkyl, C₃₋₆ haloalkyl, C₃₋₆ cycloalkyl, C₃₋₆ cycloalkyl C₁₋₃ alkyl, C₁₋₆ alkoxy C₁₋₃ alkyl, halogenated C₁₋₆ alkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups; R₂ is selected from the group consisting of H, halogen, C₁₋₆ alkyl, or C₁₋₆ alkoxy C₁₋₃ alkyl;
   R₃ is selected from the group consisting of H, C₁₋₆ alkyl, or C₁₋₆ alkoxy C₁₋₃ alkyl; or
(2) X and Y are simultaneously C, and X and Y, together with the atoms to which they are attached, form a ring A; the ring A is 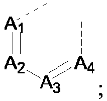
   wherein A₁, A₂, A₃, and A₄ are each independently selected from the group consisting of CR₄ or N;
   R₁ is selected from the group consisting of H, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
   R₂ is selected from the group consisting of H, halogen, C₁₋₆ alkyl, or C₁₋₆ alkoxy C₁₋₃ alkyl;
   R₃ is selected from the group consisting of H, C₁₋₆ alkyl, or C₁₋₆ alkoxy C₁₋₃ alkyl;
   each R₄ is independently selected from the group consisting of H, halogen, C₁₋₆ alkyl, C₁₋₆ alkoxy, C₁₋₆ haloalkoxy, C₁₋₆ alkoxy C₁₋₃ alkyl, or halogenated C₁₋₆ alkoxy C₁₋₃ alkyl.

In another embodiment of the present disclosure, provided is a compound of formula (IA) or (IB) or a pharmaceutically acceptable salt thereof: 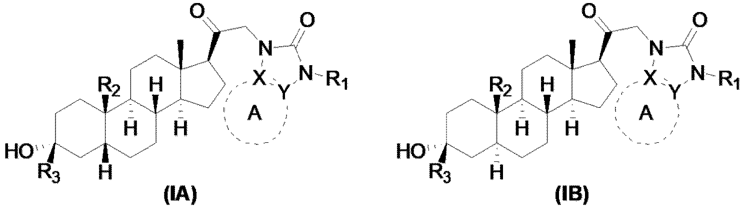 wherein,
(1) X is C, and Y is N; or X is N, and Y is C; or X and Y are simultaneously C;
R₁ is selected from the group consisting of H, C₁₋₆ alkyl, C₁₋₆ haloalkyl, C₃₋₆ cycloalkyl, C₃₋₆ cycloalkyl C₁₋₃ alkyl,
C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl,
C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl; or
(2) X and Y are simultaneously C, and X and Y, together with the atoms to which they are attached, form a ring A; the ring A is 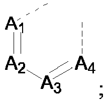 wherein A₁, A₂, A₃, and A₄ are each independently selected from the group consisting of CR₄ or N;
R₁ is selected from the group consisting of H, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl; each R₄ is independently selected from the group consisting of H, halogen, C₁₋₆ alkyl, C₁₋₆ alkoxy, C₁₋₆ haloalkoxy, C₁₋₆ alkoxy C₁₋₃ alkyl, or halogenated C₁₋₆ alkoxy C₁₋₃ alkyl.

In some embodiments of the present disclosure, in the compound of formula (I), formula (IA), or formula (1B) or the pharmaceutically acceptable salt thereof,
(1) X is C, and Y is N; X is N, and Y is C; or X and Y are both C atoms;
R₁ is selected from the group consisting of H, C₁₋₃ alkyl, C₁₋₃ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₃ alkyl,
C₁₋₃ haloalkyl, C₁₋₃ alkoxy, C₁₋₃ haloalkoxy, -(C=O)NH₂, -NH₂, or cyano groups;
R₁ is further preferably selected from the group consisting of H, methyl, ethyl, n-propyl, isopropyl, n-butyl, isobutyl, s-butyl, t-butyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, pentachloroethyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, cyclohexylethyl, methoxymethyl, ethoxymethyl, methoxyethyl, ethoxyethyl, trifluoromethoxymethyl, trifluoromethoxyethyl, phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl; the phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups;
R₁ is more preferably selected from the group consisting of methyl, ethyl, cyclopentyl, cyclohexyl, cyclopropylmethyl, 2,2,2-trifluoroethyl, methoxyethyl, trifluoromethoxyethyl, phenyl, oxazolyl, thiazolyl, pyridinyl, benzodioxolyl, benzopyrazolyl, or indolyl; the phenyl, oxazolyl, thiazolyl, pyridinyl, benzodioxolyl, benzopyrazolyl, or indolyl is optionally substituted with one or more F, Cl, Br, I, methyl, ethyl, difluoromethyl, trifluoromethyl, methoxy, trifluoromethoxy, 2,2,2-trifluoroethoxy, -(C=O)NH₂, -NH₂, or cyano groups;
R₂ is selected from the group consisting of H, F, Cl, Br, I, methyl, ethyl, n-propyl, isopropyl, methoxymethyl, or ethoxymethyl;
R₃ is selected from the group consisting of H, methyl, ethyl, n-propyl, isopropyl, methoxymethyl, or ethoxymethyl; or
(2) X and Y are both C atoms, and X and Y, together with the atoms to which they are attached, form a ring A; the ring A is selected from the group consisting of 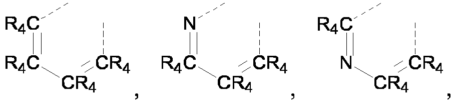 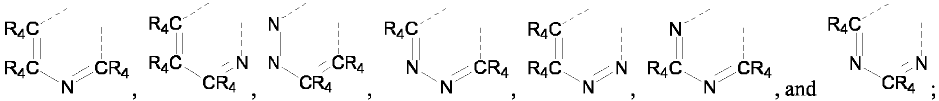
R₁ is selected from the group consisting of H, C₁₋₃ haloalkyl, C₃₋₆ cycloalkyl C₁₋₃ alkyl, C₁₋₆ alkoxy C₁₋₃ alkyl, halogenated C₁₋₆ alkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₃ alkyl, C₁₋₃ haloalkyl, C₁₋₃ alkoxy, C₁₋₃ haloalkoxy, -(C=O)NH₂, -NH₂, or cyano groups;
R₁ is further preferably selected from the group consisting of H, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, pentachloroethyl, cyclopropylmethyl, cyclopropylethyl, cyclobutylmethyl, cyclobutylethyl, cyclopentylmethyl, cyclopentylethyl, cyclohexylmethyl, cyclohexylethyl, methoxymethyl, ethoxymethyl, methoxyethyl, ethoxyethyl, trifluoromethoxymethyl, trifluoromethoxyethyl, phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl; the phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, methoxy, ethoxy, n-propoxy, isopropoxy, trifluoromethoxy, trichloromethoxy, -(C=O)NH₂, -NH₂, or cyano groups;
R₁ is more preferably selected from the group consisting of cyclopropylmethyl, 2,2,2-trifluoroethyl, methoxyethyl, trifluoromethoxyethyl, phenyl, oxazolyl, thiazolyl, pyridinyl, benzodioxolyl, benzopyrazolyl, or indolyl; the phenyl, oxazolyl, thiazolyl, pyridinyl, benzodioxolyl, benzopyrazolyl, or indolyl is optionally substituted with one or more F, Cl, Br, I, methyl, ethyl, trifluoromethyl, methoxy, trifluoromethoxy, -(C=O)NH₂, -NH₂, or cyano groups;
R₂ is selected from the group consisting of H, F, Cl, Br, I, methyl, ethyl, n-propyl, isopropyl, methoxymethyl, or ethoxymethyl;
R₃ is selected from the group consisting of H, methyl, ethyl, n-propyl, isopropyl, methoxymethyl, or ethoxymethyl; each R₄ is independently selected from the group consisting of H, F, Cl, Br, I, methyl, ethyl, n-propyl, isopropyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, methoxymethyl, ethoxymethyl, methoxyethyl, fluoromethoxymethyl, chloromethoxymethyl, difluoromethoxymethyl, dichloromethoxymethyl, trifluoromethoxymethyl, trichloromethoxymethyl, 2,2-difluoroethoxymethyl, 2,2-dichloroethoxymethyl, 2,2,2-trifluoroethoxymethyl, 2,2,2-trichloroethoxymethyl, fluoromethoxyethyl, chloromethoxyethyl, difluoromethoxyethyl, dichloromethoxyethyl, trifluoromethoxyethyl, trichloromethoxyethyl, 2,2-difluoroethoxyethyl, 2,2-dichloroethoxyethyl, 2,2,2-trifluoroethoxyethyl, or 2,2,2-trichloroethoxyethyl.

In another embodiment of the present disclosure, provided is a compound of formula (IIA), (IIB), (IIC), or (IID) or a pharmaceutically acceptable salt thereof: 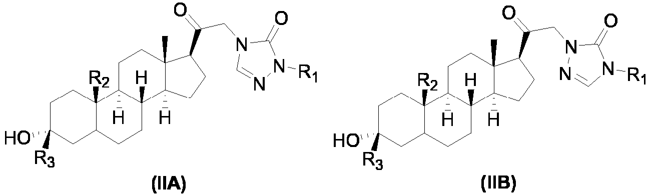 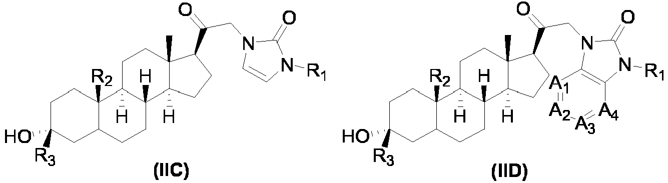 wherein,
A₁, A₂, A₃, and A₄ are each independently selected from the group consisting of CR₄ or N;
R₁ is selected from the group consisting of H, C₁₋₆ alkyl, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl;
each R₄ is independently selected from the group consisting of H, halogen, C₁₋₆ alkyl, C₁₋₆ alkoxy, C₁₋₆ haloalkoxy, C₁₋₆ alkoxy C₁₋₃ alkyl, or halogenated C₁₋₆ alkoxy C₁₋₃ alkyl.

In another embodiment of the present disclosure, provided is a compound of formula (IIA-1), (IIA-2), (IIB-1), (IIB-2), (IIC-1), (IIC-2), (IID-1), or (IID-2) or a pharmaceutically acceptable salt thereof: 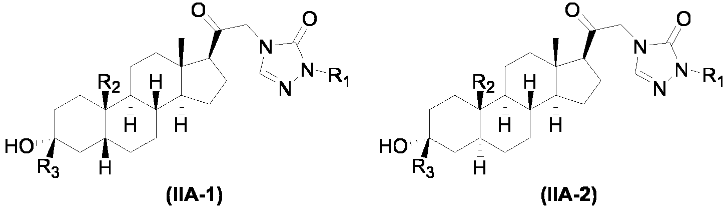 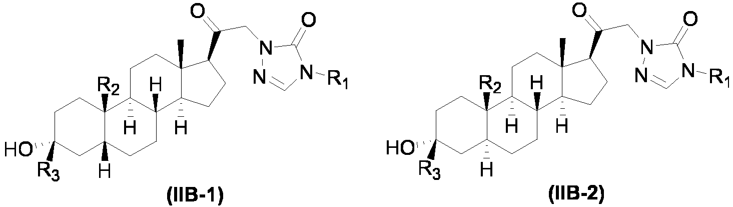 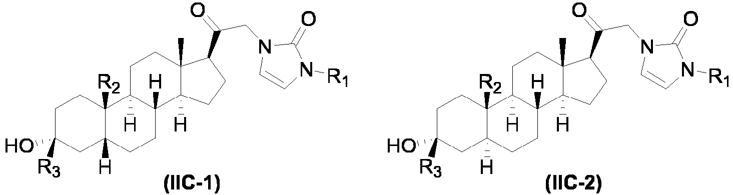 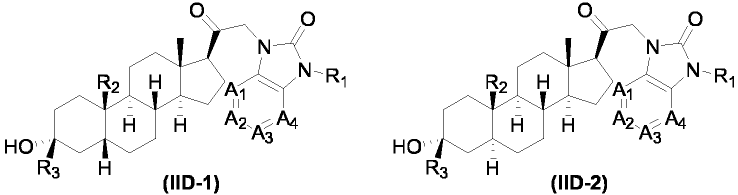 wherein,
A₁, A₂, A₃, and A₄ are each independently selected from the group consisting of CR₄ or N;
R₁ is selected from the group consisting of H, C₁₋₆ alkyl, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl;
each R₄ is independently selected from the group consisting of H, halogen, C₁₋₆ alkyl, C₁₋₆ alkoxy, C₁₋₆ haloalkoxy, C₁₋₆ alkoxy C₁₋₃ alkyl, or halogenated C₁₋₆ alkoxy C₁₋₃ alkyl.

In some embodiments of the present disclosure, in the compound of formula (IIA), (IIB), (IIC), (IIA-1), (IIA-2), (IIB-1), (IIB-2), (IIC-1) or (IIC-2) or the pharmaceutically acceptable salt thereof, R₁ is selected from the group consisting of H, methyl, ethyl, n-propyl, isopropyl, n-butyl, isobutyl, s-butyl, t-butyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, pentachloroethyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, cyclohexylethyl, methoxymethyl, ethoxymethyl, methoxyethyl, ethoxyethyl, trifluoromethoxymethyl, trifluoromethoxyethyl, phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl; the phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups, and the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₁ is selected from the group consisting of H, methyl, ethyl, cyclopropylmethyl, 2,2,2-trifluoroethyl, methoxyethyl, trifluoromethoxyethyl, phenyl, oxazolyl, thiazolyl, pyridinyl, benzodioxolyl, benzopyrazolyl, and indolyl; the phenyl, oxazolyl, thiazolyl, pyridinyl, benzodioxolyl, benzopyrazolyl, or indolyl is optionally substituted with one or more F, Cl, Br, I, methyl, ethyl, trifluoromethyl, methoxy, trifluoromethoxy, or cyano groups, and the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₁ is selected from the group consisting of H, methyl, ethyl, t-butyl, cyclopropylmethyl, 2,2,2-trifluoroethyl, cyclopentyl, cyclohexyl, methoxymethyl, ethoxymethyl, methoxyethyl, ethoxyethyl, trifluoromethoxymethyl, trifluoromethoxyethyl, 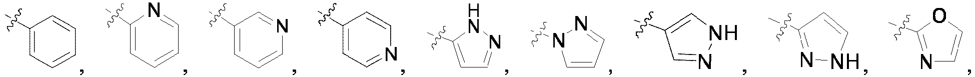 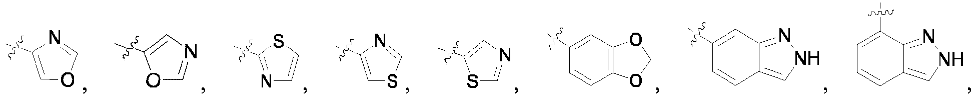 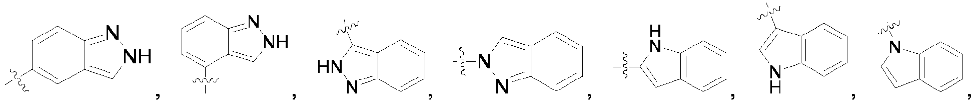 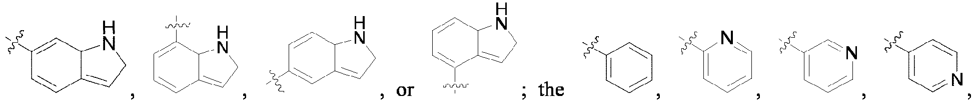 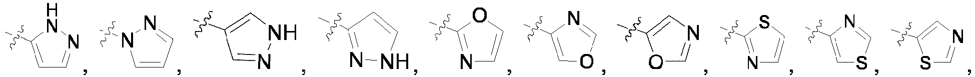 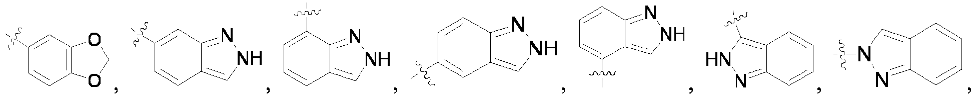 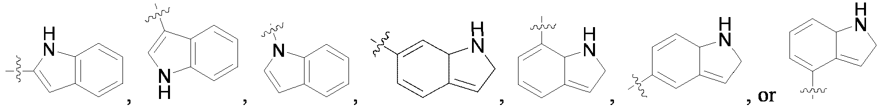 is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups, and the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₁ is selected from the group consisting of H, methyl, ethyl, cyclopropylmethyl, 2,2,2-trifluoroethyl, methoxyethyl, trifluoromethoxyethyl, 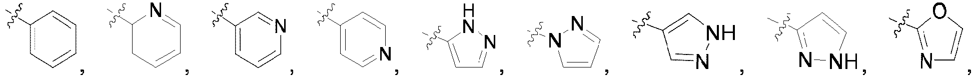 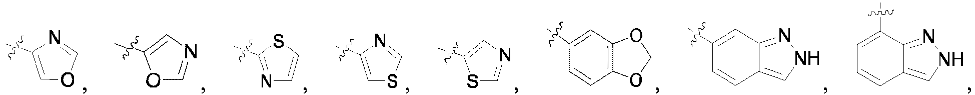 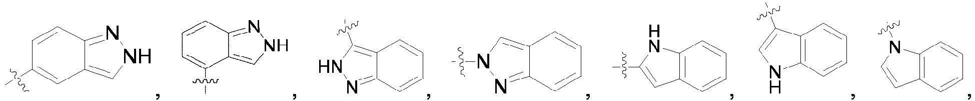 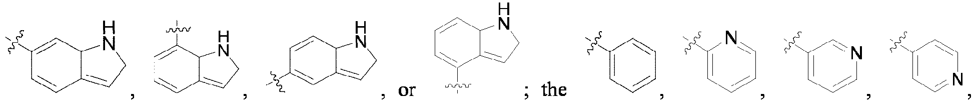 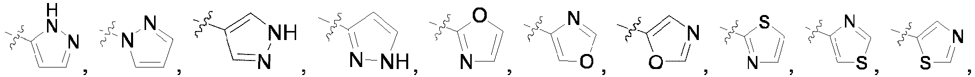 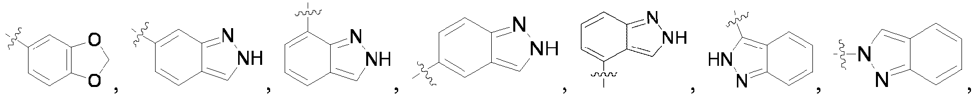 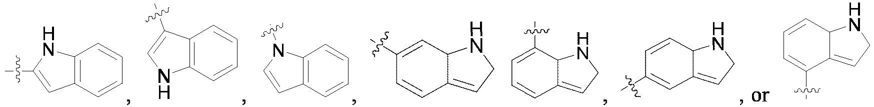 is optionally substituted with one or more F, Cl, Br, I, methyl, ethyl, trifluoromethyl, methoxy, trifluoromethoxy, or cyano groups, and the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₂ is selected from the group consisting of H, F, Cl, Br, I, methyl, ethyl, n-propyl, isopropyl, methoxymethyl, or ethoxymethyl, and the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₂ is selected from the group consisting of H or methyl and is preferably H; the other variables are as defined in the present disclosure.

In some embodiments of the present disclosure, in the compound of formula (IIA), (IIB), (IIC), (IIA-1), (IIA-2), (IIB-1), (IIB-2), (IIC-1), or (IIC-2) or the pharmaceutically acceptable salt thereof, R₃ is selected from the group consisting of H, methyl, ethyl, n-propyl, isopropyl, methoxymethyl, or ethoxymethyl and is preferably methyl; the other variables are as defined in the present disclosure.

In some embodiments of the present disclosure, in the compound of formula (IIA), (IIA-1), or (IIA-2) or the pharmaceutically acceptable salt thereof, R₁ is selected from the group consisting of phenyl, m-fluorophenyl, m-chlorophenyl, m-bromophenyl, m-iodophenyl, m-methoxyphenyl, or m-ethoxyphenyl, and the other variables are as defined in the present disclosure.

In some embodiments of the present disclosure, in the compound of formula (IIA), (IIA-1), or (IIA-2) or the pharmaceutically acceptable salt thereof, R₁ is selected from the group consisting of phenyl, m-fluorophenyl, or m-methoxyphenyl, and the other variables are as defined in the present disclosure.

In some embodiments of the present disclosure, in the compound of formula (IID), (IID-1), or (IID-2) or the pharmaceutically acceptable salt thereof, R₁ is selected from the group consisting of H, cyclopropylmethyl, 2,2,2-trifluoroethyl, methoxyethyl, trifluoromethoxyethyl, phenyl, oxazolyl, thiazolyl, pyridinyl, benzodioxolyl, benzopyrazolyl, and indolyl; the phenyl, oxazolyl, thiazolyl, pyridinyl, benzodioxolyl, benzopyrazolyl, or indolyl is optionally substituted with one or more F, Cl, Br, I, methyl, ethyl, trifluoromethyl, methoxy, trifluoromethoxy, or cyano groups, and the other variables are as defined in the present disclosure.

In some embodiments of the present disclosure, in the compound of formula (IID), (IID-1), or (IID-2) or the pharmaceutically acceptable salt thereof, R₁ is selected from the group consisting of H, cyclopropylmethyl, 2,2,2-trifluoroethyl, methoxyethyl, trifluoromethoxyethyl, 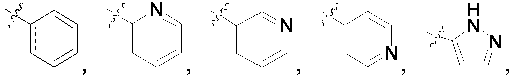 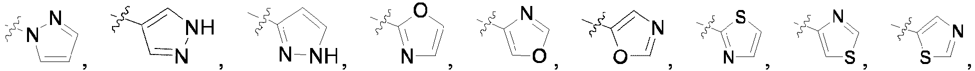 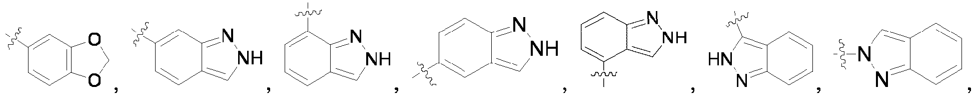 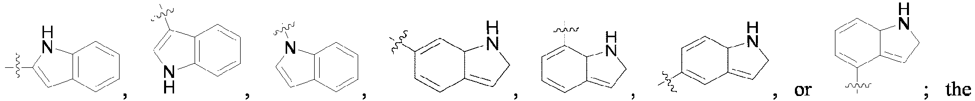 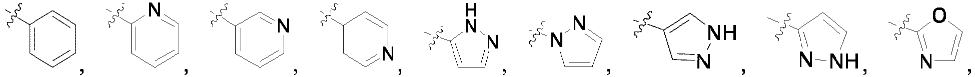 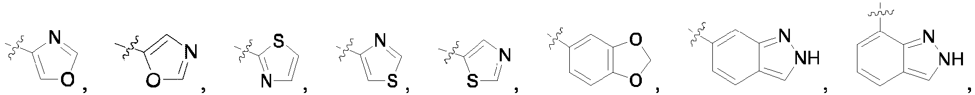 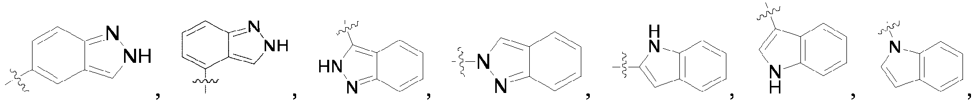 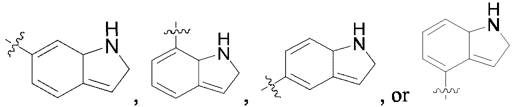 is optionally substituted with one or more F, Cl, Br, I, methyl, ethyl, trifluoromethyl, methoxy, trifluoromethoxy, or cyano groups, and the other variables are as defined in the present disclosure.

In some embodiments of the present disclosure, in the compound of formula (IID), (IID-1), or (IID-2) or the pharmaceutically acceptable salt thereof, R₂ is selected from the group consisting of H, F, Cl, Br, I, methyl, ethyl, n-propyl, isopropyl, methoxymethyl, or ethoxymethyl, and the other variables are as defined in the present disclosure.

In some embodiments of the present disclosure, in the compound of formula (IID), (IID-1), or (IID-2) or the pharmaceutically acceptable salt thereof, R₃ is selected from the group consisting of H, methyl, ethyl, n-propyl, isopropyl, methoxymethyl, or ethoxymethyl, and the other variables are as defined in the present disclosure.

In some embodiments of the present disclosure, in the compound of formula (IID), (IID-1), or (IID-2) or the pharmaceutically acceptable salt thereof, each R₄ is independently selected from the group consisting of H, F, Cl, Br, I, methyl, ethyl, n-propyl, isopropyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, methoxymethyl, ethoxymethyl, methoxyethyl, fluoromethoxymethyl, chloromethoxymethyl, difluoromethoxymethyl, dichloromethoxymethyl, trifluoromethoxymethyl, trichloromethoxymethyl, 2,2-difluoroethoxymethyl, 2,2-dichloroethoxymethyl, 2,2,2-trifluoroethoxymethyl, 2,2,2-trichloroethoxymethyl, fluoromethoxyethyl, chloromethoxyethyl, difluoromethoxyethyl, dichloromethoxyethyl, trifluoromethoxyethyl, trichloromethoxyethyl, 2,2-difluoroethoxyethyl, 2,2-dichloroethoxyethyl, 2,2,2-trifluoroethoxyethyl, or 2,2,2-trichloroethoxyethyl, and the other variables are as defined in the present disclosure.

In one embodiment of the present disclosure, in the compound of formula (IIA), (IIA-1), or (IIA-2) or the pharmaceutically acceptable salt thereof,
R₁ is selected from the group consisting of C₁₋₆ alkyl, C₃₋₆ cycloalkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with 1, 2, 3, 4, or 5 halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl.

In some embodiments of the present disclosure, in the compound of formula (IIA), (IIA-1), or (IIA-2) or the pharmaceutically acceptable salt thereof, R₁ is selected from the group consisting of t-butyl, cyclopentyl, cyclohexyl, methoxymethyl, ethoxymethyl, methoxyethyl, ethoxyethyl, trifluoromethoxymethyl, trifluoromethoxyethyl, 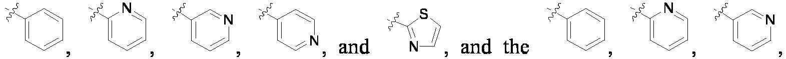 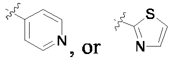 is optionally substituted with 1, 2, 3, 4, or 5 F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups. The other variables are as defined in the present disclosure.

In some embodiments of the present disclosure, in the compound of formula (IIA), (IIA-1), or (IIA-2) or the pharmaceutically acceptable salt thereof, R₁ is selected from the group consisting of 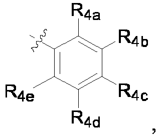 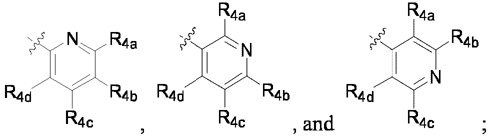 R₄ₐ, R_{4b}, R_{4c}, R₄ₐ, and R₄ₑ are each independently selected from the group consisting of H, F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, - (C=O)NH₂, -NH₂, or cyano; the other variables are as defined in the present disclosure.

In some embodiments of the present disclosure, in the compound of formula (IIA), (IIA-1), or (IIA-2) or the pharmaceutically acceptable salt thereof, R₂ is selected from the group consisting of H, F, Cl, Br, I, methyl, ethyl, n-propyl, isopropyl, methoxymethyl, or ethoxymethyl and is preferably H or methyl; the other variables are as defined in the present disclosure.

In some embodiments of the present disclosure, in the compound of formula (IIA), (IIA-1), or (IIA-2) or the pharmaceutically acceptable salt thereof, R₃ is selected from the group consisting of H, methyl, ethyl, n-propyl, isopropyl, methoxymethyl, or ethoxymethyl and is preferably methyl; the other variables are as defined in the present disclosure.

In some embodiments of the present disclosure, provided is the compound of formula (IIA), (IIA-1), or (IIA-2) or the pharmaceutically acceptable salt thereof, wherein,
R₁ is 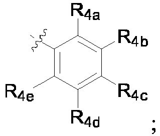
R₄ₐ, R_{4b}, R_{4c}, R_{4d}, and R₄ₑ are each independently selected from the group consisting of H, F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, -
(C=O)NH₂, -NH₂, or cyano;
R₂ is selected from the group consisting of H, F, Cl, Br, I, methyl, ethyl, n-propyl, isopropyl, methoxymethyl, or ethoxymethyl and is preferably H or methyl;
R₃ is selected from the group consisting of H, methyl, ethyl, n-propyl, isopropyl, methoxymethyl, or ethoxymethyl and is preferably methyl.

In some embodiments of the present disclosure, provided is the compound of formula (IIA), (IIA-1), or (IIA-2) or the pharmaceutically acceptable salt thereof, wherein,
R₁ is selected from the group consisting of 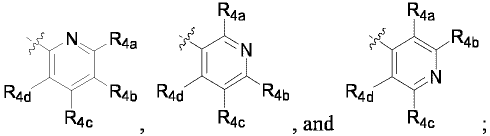
R₄ₐ, R_{4b}, R_{4c}, and R_{4d} are each independently selected from the group consisting of H, F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, - (C=O)NH₂, -NH₂, or cyano;
R₂ is selected from the group consisting of H, F, Cl, Br, I, methyl, ethyl, n-propyl, isopropyl, methoxymethyl, or ethoxymethyl and is preferably H or methyl;
R₃ is selected from the group consisting of H, methyl, ethyl, n-propyl, isopropyl, methoxymethyl, or ethoxymethyl and is preferably methyl.

In another embodiment of the present disclosure, provided are the following compounds or pharmaceutically acceptable salts thereof:

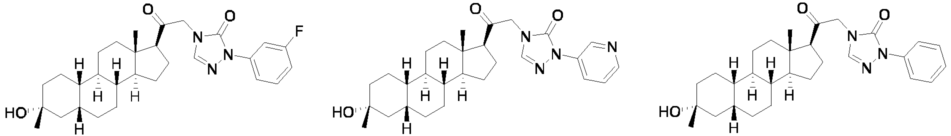

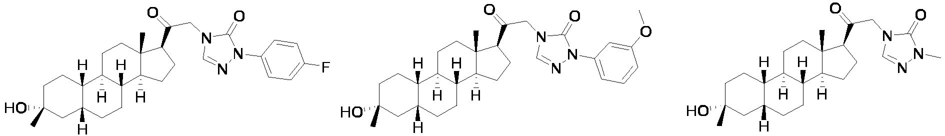

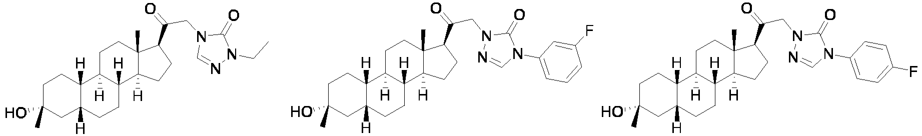

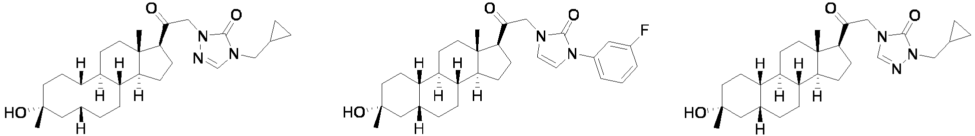

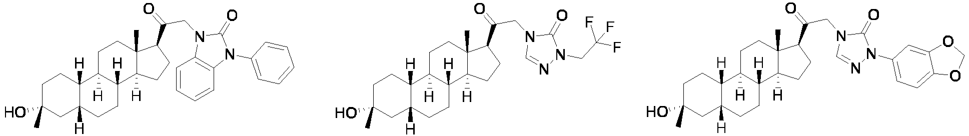

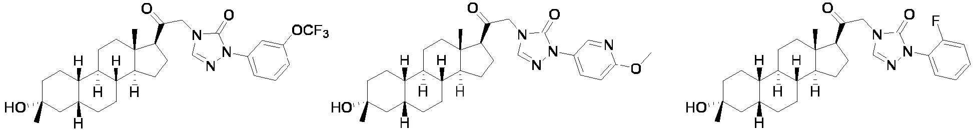

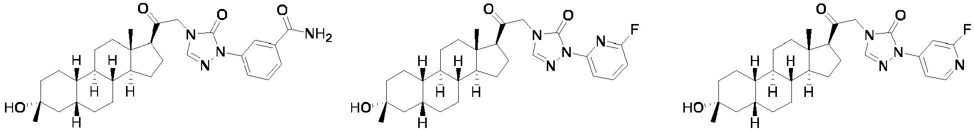

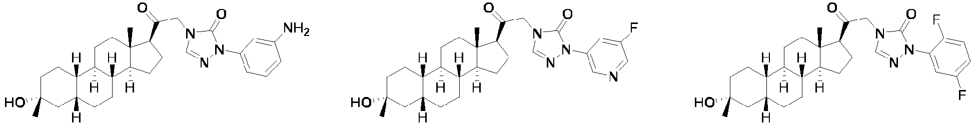

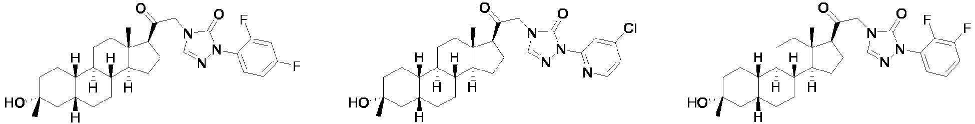

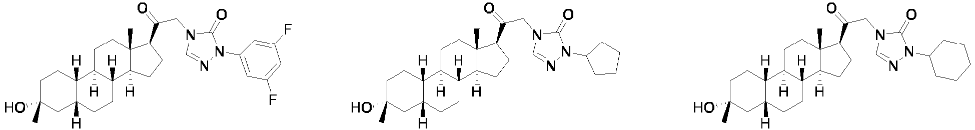

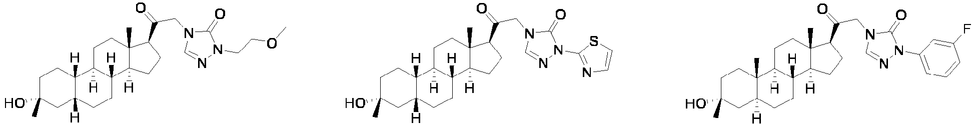

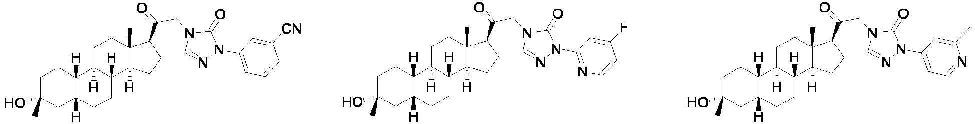

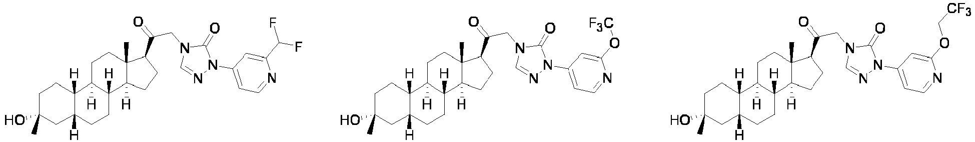

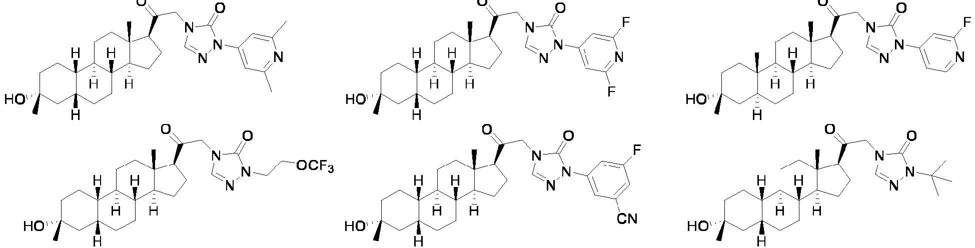

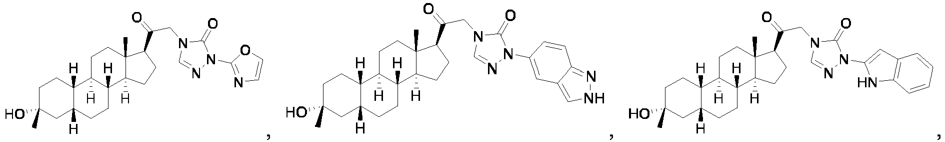

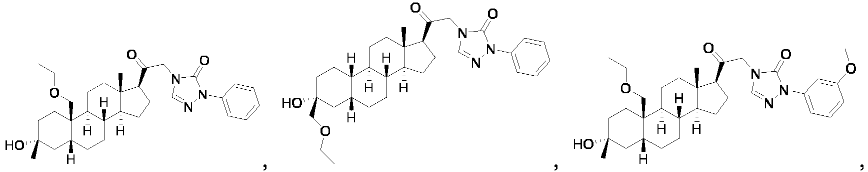

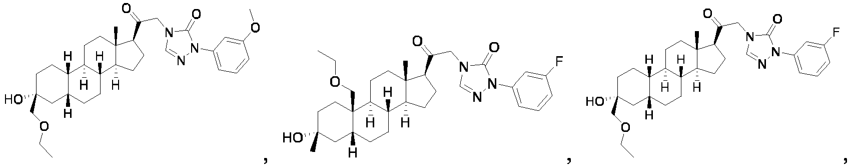

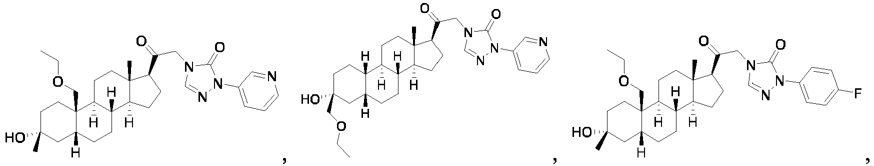

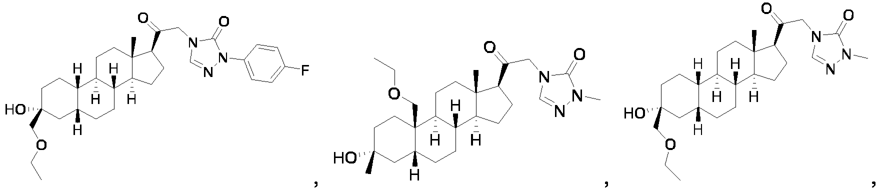

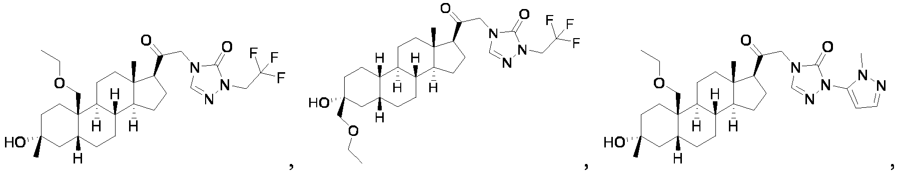

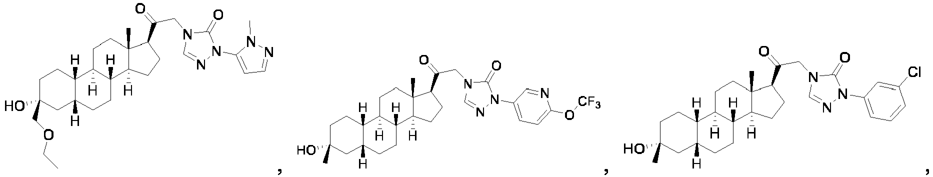

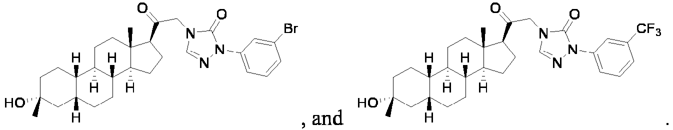

The present disclosure also provides a pharmaceutical composition comprising any one of the compounds or pharmaceutically acceptable salts thereof described above and a pharmaceutically acceptable carrier. The pharmaceutical composition can be prepared into various pharmaceutically acceptable dosage forms, such as tablets, capsules, oral liquid, granules, and injections. The pharmaceutical composition can be administered orally or parenterally (e.g., intravenously, subcutaneously, topically, etc.). The administration dose may be suitably adjusted depending on the age, sex, and type of disease of the patient, and is generally about 1-200 mg per day. The present disclosure also provides use of the compounds or the pharmaceutically acceptable salts thereof described above or the pharmaceutical composition in the manufacture of a GABA_{A} receptor regulator medicament.

The present disclosure further provides use of the compounds or the pharmaceutically acceptable salts thereof described above or the pharmaceutical composition thereof in the manufacture of a medicament for treating CNS-related diseases. The CNS-related diseases include, but are not limited to, sleep disorder (e.g., insomnia), mood disorder (e.g., depression (e.g., major depressive disorder (MDD)), mania, dysthymic disorder (e.g., minor depressive disorder), bipolar disorder (e.g., type I and/or type II), anxiety disorder (e.g., generalized anxiety disorder (GAD) and social anxiety disorder), stress, post-traumatic stress disorder (PTSD), compulsive disorder (e.g., obsessive-compulsive disorder (OCD))), schizophrenia spectrum disorder (e.g., schizophrenia and schizoaffective disorder), convulsive disorder (e.g., epilepsy (e.g., status epilepticus (SE)), epileptic seizures), disorders of memory and/or cognition (e.g., attention disorders (e.g., attention deficit hyperactivity disorder (ADHD)), dementia (e.g., dementia of the Alzheimer type, dementia with Lewy bodies, and vascular dementia), movement disorder (e.g., Huntington's disease, Parkinson's disease, and essential tremor), personality disorder (e.g., antisocial personality disorder and obsessive-compulsive personality disorder), autism spectrum disorder (ASD) (e.g., autism and monogenetic causes of autism, e.g., synaptophathy), e.g., Rett syndrome, fragile X syndrome, and Angelman syndrome), pain (e.g., neuropathic pain, injury-related pain syndrome, acute pain, and chronic pain), traumatic brain injury (TBI), vascular diseases (e.g., stroke, ischemia, and vascular malformation), substance abuse disorder and/or withdrawal syndrome (e.g., addiction to opiates, cocaine, and/or alcohol), and tinnitus.

The present disclosure further provides use of any one of the compounds or the pharmaceutically acceptable salts thereof described above, or the pharmaceutical composition thereof in the manufacture of a medicament for treating depression. The depression is selected from the group consisting of minor depression, major depressive disorder (MDD), persistent depressive disorder (PDD), psychotic depression, postpartum depression (PPD), or seasonal affective disorder.

### DEFINITION AND DESCRIPTION

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof .

The term "pharmaceutically acceptable" is described herein in terms of the compounds, compositions, and/or dosage forms which are suitable for use in contact with human and animal tissues within the scope of reliable medical judgment, with no excessive toxicity, irritation, allergic response, or other problems or complications, and commensurate with a reasonable benefit/risk ratio.

The term "pharmaceutically acceptable salt" described herein refers to a salt of the compound of the present disclosure, which is prepared by reacting the compound having a specific substituent described herein with a relatively nontoxic acid or base. When the compound of the present disclosure contains a relatively basic functional group, an acid addition salt can be obtained by contacting the neutral form of such a compound with a sufficient amount of an acid in a pure solution or a suitable inert solvent. Examples of the pharmaceutically acceptable acid addition salts include inorganic acid salts and organic acid salts, and also include salts of amino acids such as arginine, and salts of organic acids such as glucuronic acid. Certain specific compounds of the present disclosure contain basic functional group that allow the compounds to be converted into any acid addition salt.

Certain compounds of the present disclosure may have asymmetric carbon atoms (optical centers) or double bonds. Racemates, diastereoisomers, geometric isomers, and individual isomers are all included within the scope of the present disclosure.

The compounds disclosed herein may be present in a specific geometric isomer or stereoisomer form. All such compounds are contemplated herein, including cis and trans isomers, (-)- and (+)-enantiomers, (R)- and (S)-enantiomers, diastereoisomers, (D)-isomers, (L)-isomers, and racemic mixtures and other mixtures thereof, such as a mixture enriched in enantiomer or diastereoisomer, all of which are encompassed within the scope of the present disclosure. Substituents such as alkyl may have an additional asymmetric carbon atom. All such isomers and mixtures thereof are included within the scope of the present disclosure.

The pharmaceutically acceptable salt of the present disclosure can be synthesized from the parent compound containing an acid or a basic moiety by conventional chemical methods. In general, the salt is prepared by reacting the free acid or base form of the compound with a stoichiometric amount of anappropriate base or acid in water or an organic solvent or a mixture thereof.

The term "pharmaceutically acceptable carrier" refers to any preparation or carrier medium representative of a carrier that is capable of delivering an effective amount of an active substance of the present disclosure, does not interfere with the biological activity of the active substance, and has no toxic side effects on the host or patient, including, but not limited to: binders, fillers, lubricants, disintegrants, wetting agents, dispersing agents, solubilizers, suspending agents, and the like.

The present disclosure is intended to include all isotopes of atoms present in the compounds of the present disclosure. Isotopes include those atoms having the same number of atoms but different mass numbers. As a general example and not by way of limitation, isotopes of hydrogen include deuterium and tritium. Isotopes of carbon include ¹³C and ¹⁴C. Isotopically-labeled compounds of the present disclosure can generally be prepared by conventional techniques known to those skilled in the art or by processes analogous to those described herein using an appropriate isotopically-labeled reagent in place of the non-labeled reagent otherwise used.

"Optionally substituted with one or more..." means that the group is unsubstituted or at least one and at most five hydrogen atoms, e.g., 1, 2, 3, 4, or 5 hydrogen atoms, in the group are independently substituted with a corresponding number (1, 2, 3, 4, or 5) of substituents. For example: "the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups" means that the 6-10 membered aryl and 5-10 membered heteroaryl are unsubstituted or 1, 2, 3, 4, or 5 hydrogen atoms in the 6-10 membered aryl and 5-10 membered heteroaryl are independently substituted with a corresponding number (1, 2, 3, 4, or 5) of halogen, C₁₋₆ alkyl, C₁₋₆ haloalkyl, C₁₋₆ alkoxy, C₁₋₆ haloalkoxy, -(C=O)NH₂, -NH₂, or cyano groups.

Unless otherwise specified, the term "alkyl" is used to refer to linear or branched saturated hydrocarbyl, which may be monosubstituted (e.g., -CH₂F) or polysubstituted (e.g., -CF₃), and may be monovalent (e.g., methyl), divalent (e.g., methylene), or multivalent (e.g., methine). For example, C₁-C₆ represents 1 to 6 carbons, C₁₋₆ is selected from the group consisting of C₁, C₂, C₃, C₄, C₅, and C₆. Examples of alkyl include methyl (Me), ethyl (Et), propyl (e.g., n-propyl and isopropyl), butyl (e.g., n-butyl, isobutyl, s-butyl, and t-butyl), pentyl (e.g., n-pentyl, isopentyl, neopentyl, and 1-ethylpropyl), hexyl (e.g., n-hexyl, isohexyl, 1,1-dimethylbutyl, 2,2-dimethylbutyl, 3,3-dimethylbutyl, and 2-ethylbutyl), and the like.

Unless otherwise specified, the term "halo" or "halogen" by itself or as part of another substituent refers to a fluorine, chlorine, bromine, or iodine atom.

Unless otherwise specified, "haloalkyl" is used to refer to monohalogenated/polyhalogenated linear or branched alkyl. Typical haloalkyl groups include C₁₋₆ haloalkyl, such as haloalkyl of C₁, C₂, C₃, C₄, C₅, and C₆. For example, examples of C₁-C₆ haloalkyl include, but are not limited to: fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, and pentachloroethyl.

Unless otherwise specified, "alkoxy" represents the above alkyl groups (including cycloalkyl or haloalkyl) having the specified number of carbon atoms connected through an oxygen bridge. Typical alkoxy groups include C₁₋₆ alkoxy, e.g., alkoxy of C₁, C₂, C₃, C₄, C₅, and C₆, cycloalkoxy of C₃, C₄, C₅, and C₆, and haloalkoxy of C₁, C₂, C₃, C₄, C₅, and C₆. Examples of alkoxy include, but are not limited to: methoxy, ethoxy, n-propoxy, isopropoxy, n-butoxy, s-butoxy, t-butoxy, n-pentyloxy, S-pentyloxy, hexyloxy, and 2-ethylbutoxy. Examples of cycloalkoxy include, but are not limited to: cyclopropoxy, cyclobutoxy, cyclopentyloxy, and cyclohexyloxy. Examples of haloalkoxy include, but are not limited to: fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, pentafluoroethoxy, and pentachloroethoxy.

Unless otherwise specified, "cycloalkyl" refers to a saturated or partially unsaturated monocyclic or polycyclic hydrocarbon substituent, which may be monosubstituted or polysubstituted and may be monovalent, divalent, or polyvalent. Examples of these cycloalkyl groups include, but are not limited to, cyclopropyl, cyclobutyl, cyclopentyl, cyclohexyl, and the like.

Unless otherwise specified, "aryl" refers to a 6- to 10-membered carbon monocyclic or fused polycyclic (rings sharing a pair of adjacent carbon atoms) group having a conjugated π-electron system such as phenyl and naphthyl, preferably phenyl.

Unless otherwise specified, "heteroaryl" refers to a group of a 5-10 membered monocyclic or bicyclic 4n+2 aromatic ring system (e.g., having 6 or 10 π electrons shared in a cyclic array) having ring carbon atoms and 1-4 ring heteroatoms provided in the aromatic ring system, wherein each heteroatom is independently selected from the group consisting of nitrogen, oxygen, and sulfur ("5-10 membered heteroaryl"). In heteroaryl groups that contain one or more nitrogen atoms, the point of attachment can be a carbon or nitrogen atom, as long as valency permits. Heteroaryl bicyclic ring systems can include one or more heteroatoms in one or both rings. "Heteroaryl" includes ring systems in which the heteroaryl ring, as defined above, is fused with one or more carbocyclyl or heterocyclyl groups, wherein the point of attachment is on the heteroaryl ring, and in such cases, the number of ring members continues to represent the number of ring members in the heteroaryl ring system.

Examples of 5-membered heteroaryl containing one heteroatom include, but are not limited to: pyrrolyl, furanyl, and thienyl. Examples of 5-membered heteroaryl containing two heteroatoms include, but are not limited to: imidazolyl, pyrazolyl, oxazolyl, isoxazolyl, thiazolyl, and isothiazolyl. Examples of 5-membered heteroaryl containing three heteroatoms include, but are not limited to: triazolyl, oxadiazolyl, and thiadiazolyl. Examples of 5-membered heteroaryl containing four heteroatoms include, but are not limited to: tetrazolyl. Examples of 6-membered heteroaryl containing one heteroatom include, but are not limited to: pyridinyl. Examples of 6-membered heteroaryl containing two heteroatoms include, but are not limited to: pyridazinyl, pyrimidinyl, and pyrazinyl. Examples of 6-membered heteroaryl containing three or four heteroatoms include, but are not limited to: triazinyl and tetrazinyl. Examples of 7-membered heteroaryl containing one heteroatom include, but are not limited to: azepinyl, oxepinyl, and thiepinyl. Examples of 5,6-bicyclic heteroaryl include, but are not limited to: indolyl, isoindolyl, indazolyl, benzotriazolyl, benzothienyl, isobenzothienyl, benzofuranyl, benzisofuranyl, benzodioxolyl, benzimidazolyl, benzoxazolyl, benzisoxazolyl, benzooxadiazolyl, benzothiazolyl, benzisothiazolyl, benzothiadiazolyl, indolizinyl, and purinyl. Examples of 6,6-bicyclic heteroaryl include, but are not limited to: naphthyridinyl, pteridinyl, quinolyl, isoquinolyl, cinnolinyl, quinoxalinyl, phthalazinyl, and quinazolinyl.

Compounds are named either manually or by ChemDraw^{®} software, and supplier's catalog names are given for commercially available compounds.

### DETAILED DESCRIPTION

The present disclosure will be further illustrated with reference to specific examples and test examples, but the scope of the present disclosure is not limited in any way.

### Example 1

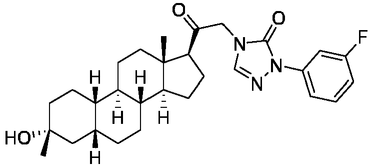

### Synthetic route:

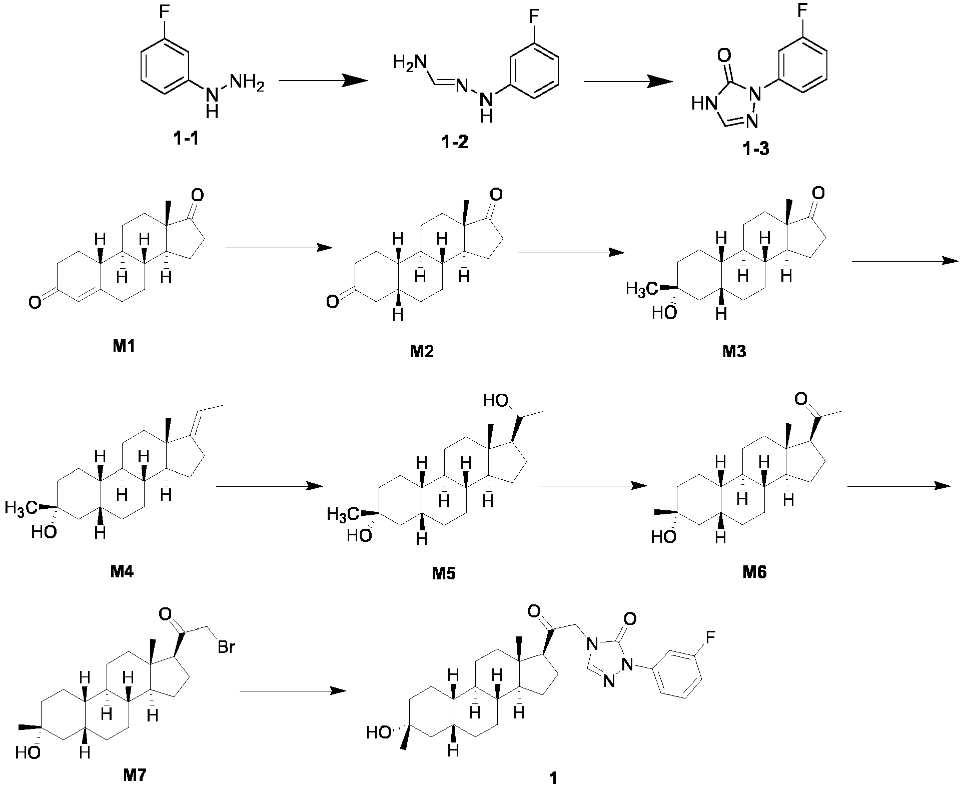

1. **M1** (200 g, 734 mmol, 1.00 *eq)* and HBr (5.96 g, 35.4 mmol, 4.00 mL, 0.048 *eq)* were added to THF (1400 mL), and then Pd/C (10.0 g, 9.43 mmol, 0.013 *eq)* was added. The system was stirred at 25 °C under H₂ (1.00 MPa) for 24 h. After the system was filtered and concentrated, the solid was ground and dissolved in 600 mL of acetone. After filtration, a while crude product was obtained. The crude product was separated and purified by column chromatography (dichloromethane/methanol (10:1-5:1)) to give **M2** (180 g, white solid).
2. **M2** (100 g, 364 mmol, 1.00 *eq)* was added to 1 L of toluene, and then methylaluminum bis(2,6-di-t-butyl-4-methylphenoxide) (525 g, 1.09 mol, 571 mL, 3.00 *eq)* was added. The system was stirred at -60 °C for 1 h, and then methylmagnesium bromide (3 M, 364 mL, 3.00 *eq)* was added at -60°C. The system was stirred at -60 °C for 3 h, and then saturated NH₄Cl (500 mL) was added. Then the mixture was filtered, and the filter cake was washed with ethyl acetate (200 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (100:1-10:1)) to give **M3** (46 g, white solid, 98 g of **M2** recovered).
3. Ethyltriphenylphosphonium bromide (102 g, 275 mmol, 4.00 *eq)* was added to THF (120 mL) at 0 °C, and then t-BuOK (30.9 g, 275 mmol, 4.00 *eq)* in THF (200 mL) was added. Then the system was stirred at 60 °C for 1 h. Then **M3** (20.0 g, 68.9 mmol, 1.00 *eq)* was dissolved in THF (120 mL), and then the solution was added to the system. The system was stirred at 60 °C for 13 h, quenched with an NH₄Cl solution (150 mL) under an ice bath, and then extracted with ethyl acetate (150 mL × 2). The organic phases were combined and then washed with saturated brine (50 mL), dried over anhydrous sodium sulfate, and filtered. The organic phase was concentrated under reduced pressure and separated and purified by column chromatography (dichloromethane/methanol (100:1-3:1)) to give **M4** (18.3 g, 60.6 mmol, yield: 88.0%).
4. **M4** (18.3 g, 60.6 mmol, 1.00 *eq)* was dissolved in THF (180 mL), and then BH₃·Me₂S (10.0 M, 30.3 mL, 5.00 *eq)* was added at 0 °C. The system was stirred at 25 °C for 3 h, and then 3.00 M NaOH (100 mL) and H₂O₂ (86.5 g, 763 mmol, 73.3 mL, 12.6 *eq)* were added dropwise at 0 °C. The system was left to react at 25 °C for 4 h and filtered, and the filtrate was quenched with a Na₂SO₃ solution (60.0 mL × 2) and extracted with ethyl acetate (150 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (100:1-3:1)) to give **M5** (13.0 g, 40.6 mmol, yield: 66.9%).
5. **M5** (13.0 g, 40.6 mmol, 1.00 *eq)* was dissolved in dichloromethane (130 mL), and then PCC (17.5 g, 81.1 mmol, *2.00 eq)* was added at 0 °C. The system was stirred at 25 °C for 3 h and filtered, and the filtrate was quenched with a Na₂SO₃ solution (100 mL) and extracted with dichloromethane (100 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (100:1-3:1)) to give **M6** (9.03 g, 28.3 mmol, yield: 69.9%).
6. **M6** (9.03 g, 28.3 mmol, 1.00 *eq)* was dissolved in MeOH (90.0 mL), and then Br₂ (4.53 g, 28.3 mmol, 1.46 mL, *1.00 eq)* and HBr (927 mg, 5.50 mmol, 622 µL, 0.190 *eq)* were added at 0 °C. Then the system was stirred at 25 °C for 12 h, quenched with a NaHCO₃ solution (50 mL), and extracted with ethyl acetate (50.0 mL × 2). The organic phases were combined and then washed with saturated brine (50 mL), dried over anhydrous sodium sulfate, and filtered. The organic phase was concentrated under reduced pressure and separated and purified by column chromatography (dichloromethane/methanol (100:1-1:1)) to give **M7** as a white solid (7.1 g, 17.8 mmol, yield: 63.0%). ¹H NMR (400 MHz, CDCl₃)δ 3.99 - 3.85 (m, 2H), 2.92 - 2.69 (m, 1H), 2.25 - 2.12 (m, 1H), 1.98 - 1.58 (m, 9H), 1.53 - 1.28 (m, 11H), 1.27 - 1.18 (m, 3H), 1.15 - 1.03 (m, 3H), 0.64 (s, 3H).
**7. 1-1** (1.00 g, 6.15 mmol, 1 *eq,* HCl) and glyoxylic acid (683.02 mg, 9.23 mmol, 513.55 µL, 1.5 *eq)* were added to HCl (12 M, 1 mL, 1.95 *eq)* and H₂O (30 mL). The system was stirred at 25 °C for 5 h, filtered, and concentrated to give crude **1-2** as a yellow solid (1.20 g).
**8. 1-2** (200 mg, 1.10 mmol, 1.00 *eq)* and Et₃N (111 mg, 1.10 mmol, 152 µL, 1.00 *eq)* were added to toluene (10.0 mL) at room temperature, and then DPPA (302 mg, 1.10 mmol, 237 µL, 1.00 *eq)* was added. The system was stirred at 90 °C for 1 h, quenched with water (20 mL), and extracted with ethyl acetate (30.0 mL). The organic phases were combined and then washed with saturated brine (30 mL), dried over anhydrous sodium sulfate, and filtered. The organic phase was concentrated under reduced pressure and separated and purified by column chromatography (dichloromethane/methanol (100:1-10:1)) to give **1-3** as a white solid (90 mg, 0.483 mmol, yield: 44%). MS (ESI) m/z = 180.2.
9. K₂CO₃ (1.04 g, 7.55 mmol, 10.0 *eq),* **M7** (300 mg, 755 µmol, 1.00 *eq),* and **1-3** (541 mg, 3.02 mmol, 4.00 *eq)* were added to THF (18.0 mL). Then the system was stirred at 30 °C for 19 h, quenched with water (150 mL), and extracted with ethyl acetate (80.0 mL × 2). The organic phases were combined and then washed with saturated brine (100 mL), dried over anhydrous sodium sulfate, and filtered. The organic phase was concentrated under reduced pressure, and then the crude product was separated and purified by pre-HPLC (column: YMC Triart 30 × 150 mm × 7 µm; mobile phase: [water(HCl)-ACN]; B%: 30%-90%, 40 min) to give **compound 1** (110 mg, 208 µmol, yield: 27.6%). MS (ESI) m/z = 478.5 [M-17] ⁺, ¹H NMR (400 MHz, CDCl₃) δ 7.79 - 7.63 (m, 2H) 7.53 (s, 1H) 7.30 (br d, J = 6.52 Hz, 1H) 6.85 (br d, J = 1.64 Hz, 1H) 4.66 - 4.43 (m, 1H) 4.40 - 4.22 (m, 1H) 2.63 - 2.44 (m, 1H) 2.40 - 1.90 (m, 2H) 1.87 - 1.56 (m, 6H) 1.54 - 1.15 (m, 16H) 1.12 - 0.93 (m, 3H) 0.61 (s, 3H).

### Example 2

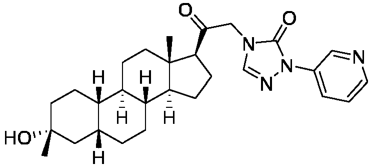

### Synthetic route:

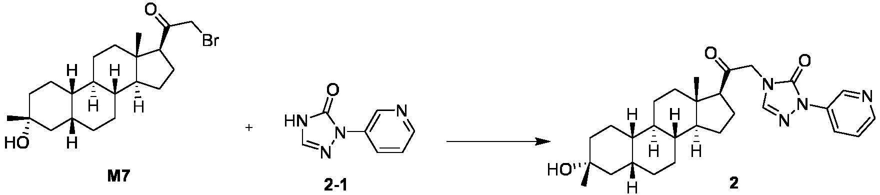

1. K₂CO₃ (852 mg, 6.17 mmol, 10.0 *eq),* **M7** (294 mg, 740 µmol, 1.20 *eq),* and **2-1** (100 mg, 617 µmol, 1.00 *eq)* (2-1 was prepared with reference to the preparation method for 1-3) were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **2** as a white solid (70.0 mg, 141 µmol, yield: 22.8%). MS (ESI) m/z = 479.5 [M + H] ⁺, ¹H NMR (400 MHz, DMSO-*d₆*) δ 9.11 (d, J = 2.12 Hz, 1H), 8.45 - 8.48 (m, 1H), 8.22 - 8.27 (m, 2H), 7.52 (dd, J = 8.38, 4.63 Hz, 1H), 4.69 - 4.76 (m, 1H), 4.54 - 4.62 (m, 1H), 4.25 (s, 1H), 2.77 (br t, J = 8.82 Hz, 1H), 2.02 - 2.12 (m, 2H), 1.56 - 1.78 (m, 7H), 1.13 - 1.39 (m, 10H), 0.97 - 1.12 (m, 7H), 0.59 (s, 3H).

### Example 3

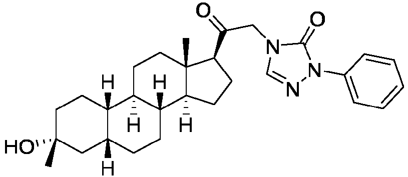

### Synthetic route:

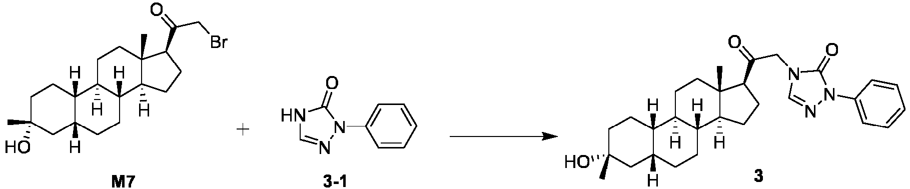

1. K₂CO₃ (386 mg, 2.79 mmol, 10.0 *eq),* **M7** (111 mg, 279 µmol, 1.00 *eq),* and **3-1** (180 mg, 1.12 mmol, 4.00 *eq)* **(3-1** was prepared with reference to the preparation method for **1-3)** were added to THF (20 mL). Then the system was stirred at 50 °C for 6 h, quenched with water (10 mL), and extracted with 2-methyltetrahydrofuran (10.0 mL × 2). The organic phases were combined and then washed with saturated brine (50 mL), dried over anhydrous sodium sulfate, and filtered. The organic phase was concentrated under reduced pressure, and the crude product was separated by SFC (column: DAICEL CHIRALCEL OJ (250 mm × 30 mm, 10 µm); mobile phase: [0.1% NH₃H₂O ETOH]; B%: 40%-40%, min) to give a crude product as a yellow liquid. The crude product was separated and purified by column chromatography (dichloromethane/methanol (0-10%)) to give **3** as a yellow solid (100 mg, 202 µmol, yield: 72.2%). MS (ESI) m/z = 478.4 [M+H]⁺, ¹H NMR (400 MHz, CDCl₃) δ 7.99 - 7.92 (m, 2H), 7.63 - 7.58 (m, 1H), 7.47 - 7.40 (m, 2H), 7.25 - 7.20 (m, 1H), 4.66 - 4.37 (m, 2H), 2.67 - 2.59 (m, 1H), 2.28 - 2.11 (m, 2H), 1.91 - 1.71 (m, 7H), 1.70 - 1.61 (m, 2H), 1.50 - 1.12 (m, 16H), 0.61(s, 3H).

### Example 4

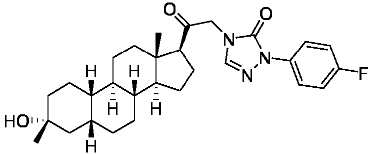

### Synthetic route:

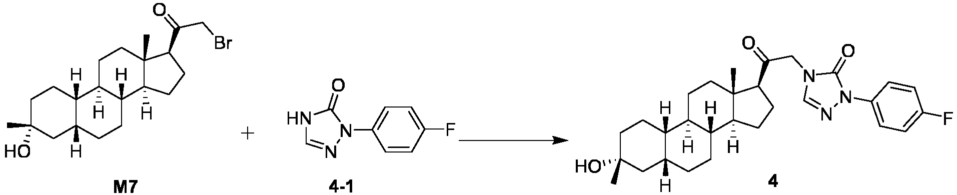

1. K₂CO₃ (386 mg, 2.79 mmol, 10.0 *eq),* **M7** (112 mg, 279 µmol, 1.00 *eq),* and **4-1** (200 mg, 1.12 mmol, 4.00 *eq)* **(4-1** was prepared with reference to the preparation method for **1-3)** were added to THF (20 mL). Then the system was stirred at 50 °C for 6 h, quenched with water (100 mL), and extracted with 2-methyltetrahydrofuran (20.0 mL × 2). The organic phases were combined and then washed with saturated brine (50 mL), dried over anhydrous sodium sulfate, and filtered. The organic phase was concentrated under reduced pressure, and the crude product was separated by SFC (column: DAICEL CHIRALCEL OJ (250 mm × 30 mm, 10 µm); mobile phase: [0.1% NH₃H₂O ETOH]; B%: 35%-35%, min) to give a crude product as a yellow liquid. The crude product was purified by pre-HPLC (column: Phenomenex luna C18 250 × 50 mm × 10 µm; mobile phase: [water (HCl)-ACN]; B%: 30%-70%, 20 min) to give 4 as a yellow solid. Then the solid was purified by SFC (EB6914-18-P1S2, RT = 2.589 min) (column: DAICEL CHIRALPAK IC (250 mm × 30 mm,10 µm); mobile phase: [0.1% NH₃H₂O ETOH]; B%: 50%-50%, min) to give 4 as a white solid (80 mg, 161 µmol, yield: 57.8%). MS (ESI) m/z = 478.4 [M-OH]⁺, ¹H NMR (400 MHz, CDCl₃) δ 7.99 - 7.87 (m, 2H), 7.63 - 7.53 (m, 1H), 7.17 - 7.06 (m, 2H), 4.67 - 4.32 (m, 2H), 2.72 - 2.57 (m, 1H), 2.30 - 2.09 (m, 2H), 1.96 - 1.66 (m, 9H), 1.52 - 1.39 (m, 14H), 1.18 - 1.07 (m, 2H), 0.61 (s, 3H).

### Example 5

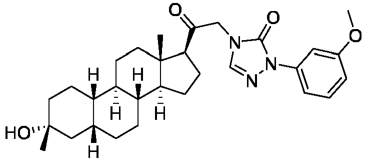

### Synthetic route:

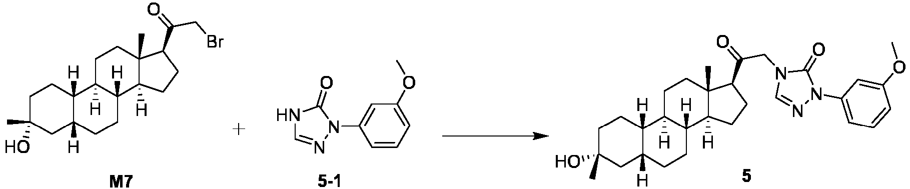

1. K₂CO₃ (580 mg, 4.19 mmol, 10.0 *eq),* **M7** (200 mg, 503 µmol, 1.2 *eq),* and **5-1** (80.2 mg, 0.419 mmol, 1.00 *eq)* (5-1 was prepared with reference to the preparation method for 1-3) were added to THF (15 mL). Then the system was stirred at 50 °C for 14 h, quenched with water (50 mL), and extracted with ethyl acetate (50.0 mL × 2). The organic phases were combined and then washed with saturated brine (50 mL), dried over anhydrous sodium sulfate, and filtered. The organic phase was concentrated under reduced pressure, and the crude product was separated and purified by SFC (column: DAICEL CHIRALCEL OJ (250 mm × 30 mm, 10 µm); mobile phase: [0.1% NH₃H₂O ETOH]; B%: 45%-45%, min) to give 5 as a yellow solid (81 mg, 152 µmol, yield: 36.3%). MS (ESI) m/z = 508 [M+1]⁺, ¹H NMR (400 MHz, DMSO-*d₆*) δ 8.14 (s, 1H), 7.43 - 7.54 (m, 2H), 7.37 (t, J = 8.32 Hz, 1H), 6.77 - 6.88 (m, 1H), 4.51 - 4.76 (m, 2H), 4.25 (s, 1H), 3.78 (s, 3H), 2.76 (br t, J = 8.76 Hz, 1H), 2.01 - 2.15 (m, 2H), 1.82 - 1.14 (m, 21H), 0.59 (s, 3H).

### Example 6

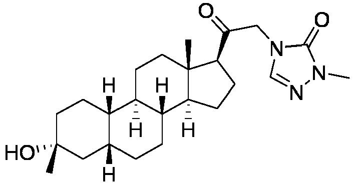

### Synthetic route:

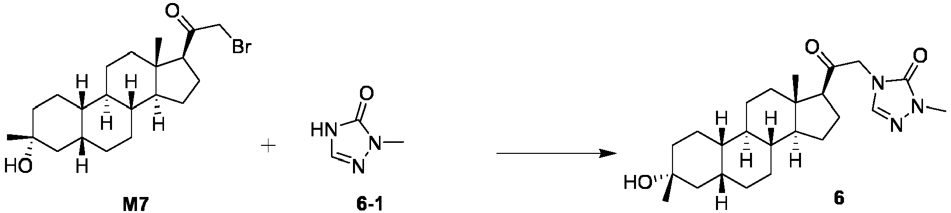

1. K₂CO₃ (4.46 g, 32.3 mmol, 1.0 *eq),* **M7** (1.41 g, 3.55 mmol, 1.1 *eq),* and **6-1** (780 mg, 3.23 mmol, 1.00 *eq) **(6-***1 was prepared with reference to the preparation method for **1-3)** were added to THF (50 mL). Then the system was stirred at 50 °C for 16 h, quenched with water (50 mL), and extracted with 2-methyltetrahydrofuran (50.0 mL × 2). The organic phases were combined and then washed with saturated brine (50 mL), dried over anhydrous sodium sulfate, and filtered. The organic phase was concentrated under reduced pressure, and the crude product was ground and dissolved in MTBE (10 mL). The solution was stirred at 25 °C for 1 h and filtered to give a filter cake of **6** as a white solid (200 mg, 478 µmol, yield: 14.8%). MS (ESI) m/z = 416.3 [M+1]⁺, ¹H NMR (400 MHz, DMSO-*d₆*) δ 7.88 - 7.75 (m, 1H), 4.59 - 4.41 (m, 2H), 4.25 - 4.21 (m, 1H), 3.31 - 3.28 (m, 3H), 2.74 - 2.67 (m, 1H), 2.10 - 2.00 (m, 2H), 1.80 - 1.42 (m, 9H), 1.39 - 1.23 (m, 7H), 1.20 - 1.06 (m, 6H), 1.05 - 0.98 (m, 2H), 0.62 - 0.48 (m, 3H).

### Example 7

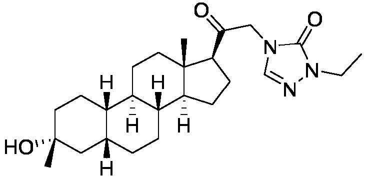

### Synthetic route:

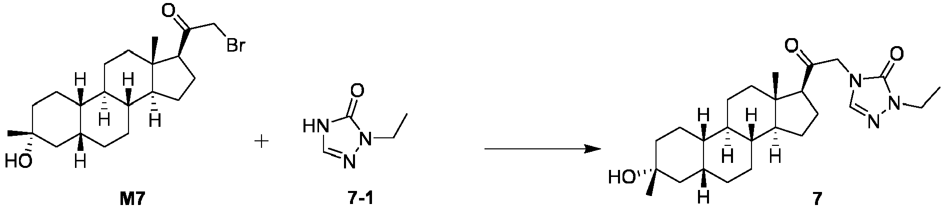

1. K₂CO₃ (1.20 g, 8.66 mmol, 10.0 *eq),* **M7** (378 mg, 0.952 mmol, 1.1 *eq),* and **7-1** (98 mg, 0.866 mmol, 1.00 *eq)* (7-1 was prepared with reference to the preparation method for 1-3) were added to THF (50 mL). Then the system was stirred at 50 °C for 12 h, quenched with water (20 mL), and extracted with ethyl acetate (20.0 mL × 2). The organic phases were combined and then washed with saturated brine (20 mL), dried over anhydrous sodium sulfate, and filtered. The organic phase was concentrated under reduced pressure and separated and purified by column chromatography (dichloromethane/methanol (100:1-10:1)) to give 7 (100 mg, 221 µmol, yield: 25.5%). MS (ESI) m/z = 430.5 [M+1]⁺, ¹H NMR (400 MHz, CDCl₃) δ 7.43 (s, 1H), 4.66 - 4.26 (m, 2H), 3.85 (q, J = 7.2 Hz, 2H), 2.73 - 2.51 (m, 1H), 2.29 - 2.07 (m, 3H), 1.99 - 1.23 (m, 23H), 1.17 - 1.02 (m, 4H), 0.67 (s, 3H).

### Example 8

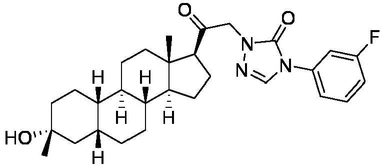

### Synthetic route:

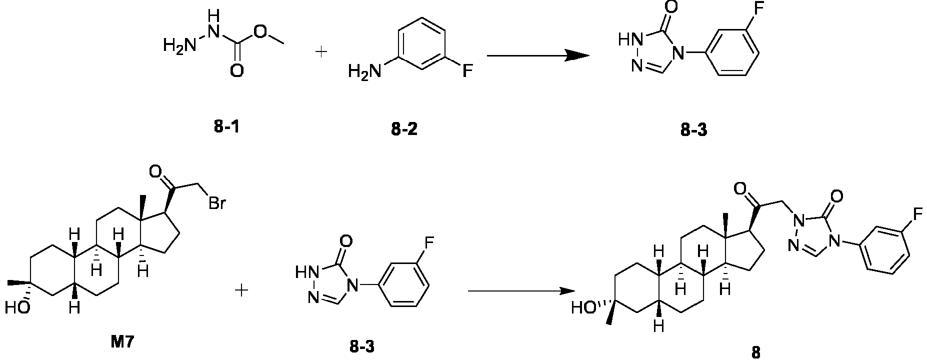

1. Trimethyl orthoformate (4.78 g, 45.0 mmol, 4.93 mL, 1.00 *eq),* **8-2** (5.00 g, 45.0 mmol, 4.31 mL, 1.00 *eq),* and **8-1** (4.05 g, 45.0 mmol, 1.00 *eq)* were added to MeOH (50.0 mL). The system was stirred at 70 °C for 6 h, and CH₃ONa (7.29 g, 135 mmol, 3.00 *eq)* was added to the system. Then the system was stirred at 70 °C for 13 h. The pH was adjusted to ~1 with HCl (1 M, 80.0 mL), and extraction was performed with ethyl acetate (70.0 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (0-7%)) to give **8-3** as a pale yellow solid (560 mg, 3.13 mmol, yield: 6.95%). MS (ESI) m/z = 179.0 [M+1]⁺.
2. K₂CO₃ (1.39 g, 10.0 mmol, 10.0 *eq),* **M7** (399 mg, 1.00 mmol, 1.00 *eq),* and **8-3** (98 mg, 0.866 mmol, 1.00 *eq)* were added to THF (15 mL). Then the system was stirred at 50 °C for 18 h, quenched with water (40 mL), and extracted with ethyl acetate (50.0 mL × 2). The organic phases were combined and then washed with saturated brine (60 mL), dried over anhydrous sodium sulfate, and filtered. The organic phase was concentrated under reduced pressure and separated and purified by column chromatography (dichloromethane/methanol (0-7%)) to give **8** as a white solid (200 mg, 400 µmol, yield: 39.8%). MS (ESI) m/z = 496.2 [M+1]⁺, ¹H NMR (400 MHz, DMSO-*d₆*) δ 8.60 (s, 1H), 7.86 - 7.43 (m, 3H), 7.37 - 7.05 (m, 1H), 4.94 - 4.48 (m, 2H), 4.24 (s, 1H), 2.76 (br t, J = 8.8 Hz, 1H), 2.18 - 1.93 (m, 2H), 1.82 - 1.54 (m, 7H), 1.44 - 0.95 (m, 17H), 0.59 (s, 3H).

### Example 9

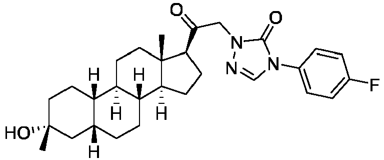

### Synthetic route:

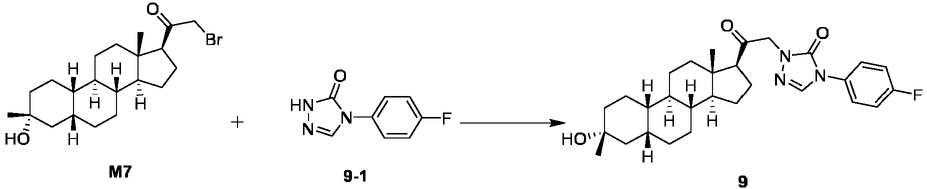

1. K₂CO₃ (1.16 g, 8.42 mmol, 10.0 *eq),* M7 (400 mg, 1.01 mmol, 1.20 *eq),* and **9-1** (151 mg, 0.842 mmol, 1.00 *eq)* **(9-1** was prepared with reference to the preparation method for **8-3)** were added to THF (15 mL). Then the system was stirred at 50 °C for 16 h, quenched with water (100 mL), and extracted with ethyl acetate (50.0 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (100/0-90/10)) to give 9 as a white solid (200 mg, 404 µmol, yield: 47.9%). MS (ESI) m/z = 496.8 [M+1]⁺, ¹H NMR (400 MHz, DMSO-*d₆*) δ 8.49 (s, 1H), 7.71 - 7.77 (m, 2H), 7.38 (t, J = 8.88 Hz, 2H), 4.73 - 4.81 (m, 1H), 4.58 (d, J = 18.4 Hz, 1H), 4.22 - 4.27 (m, 1H), 2.75 (s, 1H), 2.00 - 2.08 (m, 3H), 1.57 - 1.77 (m, 8H), 1.28 - 1.41 (m, 6H), 0.93 - 1.12 (m, 9H), 0.58 (s, 3H).

### Example 10

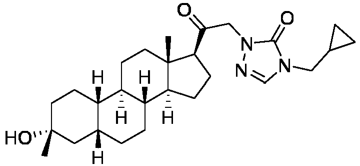

### Synthetic route:

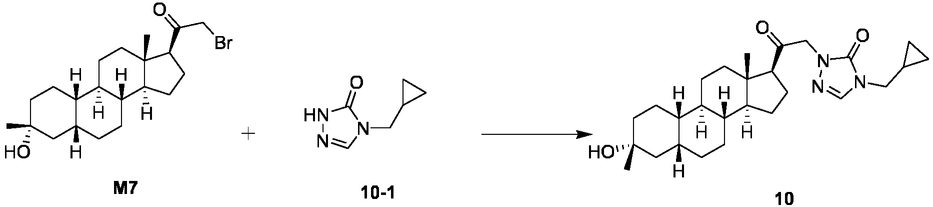

1. K₂CO₃ (894 mg, 6.47 mmol, 10.0 *eq),* **M7** (257 mg, 0.647 mmol, 1.20 *eq),* and **10-1** (90.0 mg, 0.647 mmol, 1.00 *eq)* **(10-1** was prepared with reference to the preparation method for **8-3)** were added to THF (10.0 mL). Then the system was stirred at 50 °C for 18 h, quenched with water (20 mL), and extracted with ethyl acetate (40.0 mL × 2). The organic phases were combined and then washed with saturated brine (50 mL), dried over anhydrous sodium sulfate, and filtered. The organic phase was concentrated under reduced pressure, separated and purified by column chromatography (dichloromethane/methanol (0-5%)), and further purified by pre-HPLC (column: Welch Xtimate C18 150 × 30 mm × 5 µm; mobile phase: water (NH₄HCO₃)-ACN; B%: 0%-90%, 14 min) to give 10 as a white solid (80.0 mg, 175 µmol, yield: 27.1%). MS (ESI) m/z = 455.3 [M+1]⁺, ¹H NMR (400 MHz, DMSO-*d₆*) δ 8.02 (s, 1H), 4.76 - 4.57 (m, 1H), 4.55 - 4.37 (m, 1H), 3.43 (br d, J = 7.2 Hz, 2H), 2.68 (br s, 1H), 2.22-2.09(m, 2H), 1.82 - 1.08 (m, 26H), 0.61 - 0.44 (m, 5H), 0.38 - 0.28 (m, 2H).

### Example 11

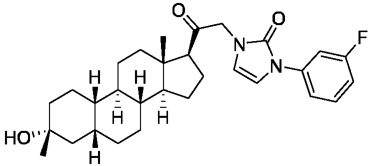

### Synthetic route:

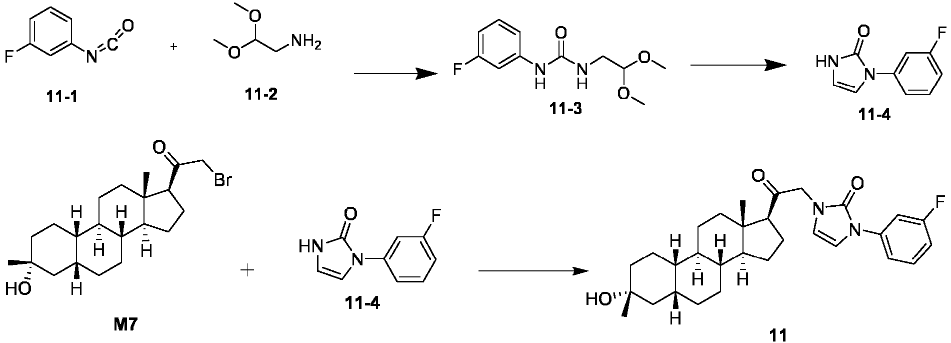

1. **11-2** (2.30 g, 21.9 mmol, 2.38 mL, 1.00 *eq)* was dissolved in toluene (30.0 mL). Then the system was left at 5 °C, and **11-1** (3.00 g, 21.9 mmol, 2.50 mL, 1.00 *eq)* was added. Then the system was stirred at 25 °C for 12 h and concentrated to give **11-3** as a colorless oil (5.05 g, 20.8 mmol, yield: 95%). ¹H NMR (400 MHz, CDCl₃) δ 8.09 (s, 1H), 7.20 (td, J = 2.0, 11.2 Hz, 1H), 7.14 - 7.05 (m, 1H), 6.92 (dd, J = 1.2, 8.4 Hz, 1H), 6.68 - 6.54 (m, 1H), 6.14 (br s, 1H), 4.39 (t, J = 5.2 Hz, 1H), 3.43 - 3.32 (m, 8H).
2. HCl (12.0 M, 3.00 mL, 4.36 *eq)* was added to H₂O (20.0 mL), and then the mixture was added dropwise to **11-3** (2.00 g, 8.26 mmol, 1.00 *eq).* The system was stirred at 25 °C for 10 h, quenched with water (20 mL), and extracted with ethyl acetate (10.0 mL × 2). The organic phases were combined and then washed with saturated brine (20 mL), dried over anhydrous sodium sulfate, and filtered. The organic phase was concentrated under reduced pressure and separated by column chromatography (petroleum ether/ethyl acetate (100/1-1/1)) to give **11-4** as a white solid (900 mg, 5.05 mmol, yield: 61.2%). ¹H NMR (400 MHz, CDCl₃) δ 10.40 (br s, 1H), 7.49 - 7.32 (m, 3H), 7.02 - 6.92 (m, 1H), 6.59 - 6.52 (m, 1H), 6.45 (t, J = 2.8 Hz, 1H).
3. K₂CO₃ (1.26 g, 9.29 mmol, 10.0 *eq),* **M7** (397 mg, 1.00 mmol, 1.10 *eq),* and 11-4 (162 mg, 0.909 mmol, 1.00 *eq)* were added to THF (10.0 mL). Then the system was stirred at 50 °C for 12 h, quenched with water (20 mL), and extracted with ethyl acetate (20.0 mL × 2). The organic phases were combined and then washed with saturated brine (50 mL), dried over anhydrous sodium sulfate, and filtered. The organic phase was concentrated under reduced pressure and separated and purified by column chromatography (dichloromethane/methanol (100/1-3/1)) to give 11 as an off-white solid (200 mg, 0.401 mmol, yield: 44.1%). MS (ESI) m/z = 495.5 [M + 1] ⁺, ¹H NMR (400 MHz, CDCl₃) δ 7.47 (td, J = 1.6, 10.4 Hz, 1H), 7.42 - 7.32 (m, 2H), 6.99 - 6.88 (m, 1H), 6.63 (d, J = 3.2 Hz, 1H), 6.37 (d, J = 3.2 Hz, 1H), 4.59 (d, J = 18.4 Hz, 1H), 4.35 (d, J = 18.4 Hz, 1H), 2.62 (s, 1H), 2.29 - 2.09 (m, 2H), 1.94 - 1.02 (m, 25H), 0.69 (s, 3H).

### Example 12

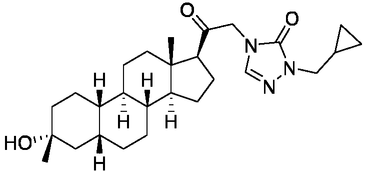

### Synthetic route:

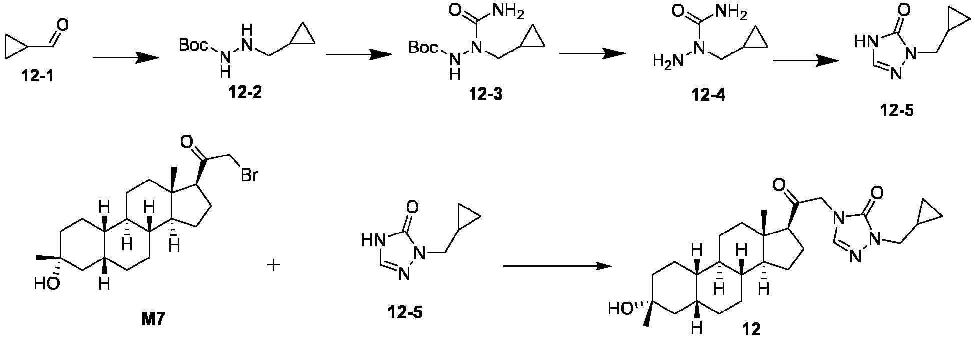

1. **12-1** (5.00 g, 71.3 mmol, 5.33 mL, 1.00 *eq*), BocNHNH₂ (9.43 g, 71.3 mmol, 1.00 *eq),* and NaBH₃CN (8.97 g, 142.7 mmol, 2.00 *eq)* were added to MeOH (50.0 mL), and then AcOH (14.0 mL) was added at 25 °C. The system was stirred at 25 °C for 16 h and concentrated to give a crude product, and the crude product was separated and purified by column chromatography (dichloromethane/methanol (100/1-1/1)) to give **12-2** as a white solid (2.26 g, 12.1 mmol, yield: 17.0%). ¹H NMR (400 MHz, CDCl₃) δ 7.09 (br s, 1H), 4.31 - 3.73 (m, 1H), 2.44 (br d, J = 7.2 Hz, 2H), 1.33 - 1.10 (m, 9H), 0.79 - 0.52 (m, 1H), 0.29 - 0.10 (m, 2H), 0.00 -0.21 (m, 2H).
**2. 12-2** (1.00 g, 5.37 mmol, 1.00 *eq)* was added to i-PrOH (10.0 mL), and then TMSNCO (1.24 g, 10.7 mmol, 1.43 mL, 2.00 *eq)* was added. The system was stirred at 25 °C for 2 h and concentrated to give **12-3** as a white solid (1.19 g, 5.19 mmol, yield: 96.7%). ¹H NMR (400 MHz, CDCl₃) δ 6.90 - 6.51 (m, 1H), 5.18 (br s, 2H), 3.96 - 2.96 (m, 2H), 1.48 (s, 8H), 0.98 - 0.82 (m, 1H), 0.57 - 0.45 (m, 2H), 0.19 (br d, J = 4.4 Hz, 2H).
3. **12-3** (500 mg, 2.18 mmol, 1.00 *eq)* and HCl/EtOAc (4.00 M, 545 µL, 1.00 *eq)* were stirred at 25 °C for 1 h, and the system was concentrated to give **12-4** (350 mg, crude). ¹H NMR (400 MHz, DMSO-*d₆*) δ 10.55 - 9.74 (m, 2H), 6.93 (br s, 1H), 6.73 - 6.51 (m, 1H), 3.47 (d, J = 7.2 Hz, 2H), 1.13 - 0.87 (m, 1H), 0.50 - 0.43 (m, 2H), 0.36 - 0.26 (m, 2H).
4. **12-4** (350 mg, 2.71 mmol, 1.00 *eq)* was added to EtOH (4.00 mL), and then CH(OCH₃)₃ (718 mg, 6.77 mmol, 742 µL, 2.50 *eq)* was added. The system was stirred at 25 °C for 2 h and concentrated to give a crude product, and the crude product was stirred in diisopropyl ether (5.00 mL) at 25 °C for 30 min to give **12-5** as a white solid (160 mg, 1.15 mmol, yield: 42.4%). ¹H NMR (400 MHz, DMSO-*d₆*) δ 11.46 (br s, 1H), 7.79 (s, 1H), 3.49 (d, J = 6.8 Hz, 2H), 1.20 - 0.91 (m, 1H), 0.60 - -0.21 (m, 4H).
5. K₂CO₃ (695 mg, 5.03 mmol, 5.0 *eq),* **M7** (399 mg, 1.01 mmol, 1.00 *eq),* and **12-5** (140 mg, 1.01 mmol, 1.00 *eq)* were added to THF (10.0 mL). Then the system was stirred at 50 °C for 12 h, quenched with water (20 mL), and extracted with ethyl acetate (20.0 mL × 2). The organic phases were combined and then washed with saturated brine (20 mL), dried over anhydrous sodium sulfate, and filtered. The organic phase was concentrated under reduced pressure and separated and purified by column chromatography (dichloromethane/methanol (100/1-10/1)) to give **12** as a white solid (300 mg, 0.655 mmol, yield: 65.1%). MS (ESI) m/z = 456.5 [M + 1] ⁺, ¹H NMR (400 MHz, CDCl₃) δ 7.44 (s, 1H), 4.58 - 4.47 (m, 1H), 4.42 - 4.31 (m, 1H), 3.67 (d, J = 7.2 Hz, 2H), 2.67 - 2.55 (m, 1H), 2.28 - 2.06 (m, 2H), 1.82 - 1.04 (m, 26H), 0.67 (s, 3H), 0.61 - 0.48 (m, 2H), 0.38 (q, J = 5.2 Hz, 2H).

### Example 13

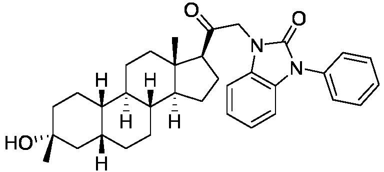

### Synthetic route:

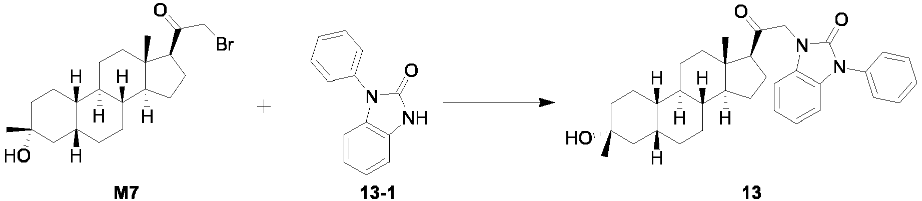

1. K₂CO₃ (657 mg, 4.76 mmol, 10.0 *eq),* **M7** (208 mg, 0.523 mmol, 1.10 *eq),* and **13-1** (100 mg, 0.476 mmol, 1.00 *eq)* (purchased from Shanghai Bide) were added to THF (10.0 mL). Then the system was stirred at 50 °C for 16 h, quenched with water (20 mL), and extracted with ethyl acetate (20.0 mL × 3). The organic phases were combined and then washed with saturated brine (50 mL), dried over anhydrous sodium sulfate, and filtered. The organic phase was concentrated under reduced pressure and separated and purified by column chromatography (dichloromethane/methanol 10:1) to give 13 as a pale yellow solid (88 mg, 0.166 mmol, yield: 34.8%). MS (ESI) m/z = 527.2 [M + 1] ⁺, ¹H NMR (400 MHz, DMSO-*d₆*) δ 7.71 - 7.39 (m, 5H), 7.22 - 6.98 (m, 4H), 5.06 - 4.61 (m, 2H), 4.24 (s, 1H), 2.23 - 2.04 (m, 3H), 1.74 - 1.11 (m, 24H), 0.61 (s, 3H).

### Example 14

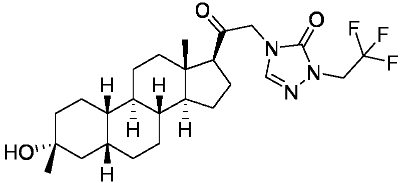

### Synthetic route:

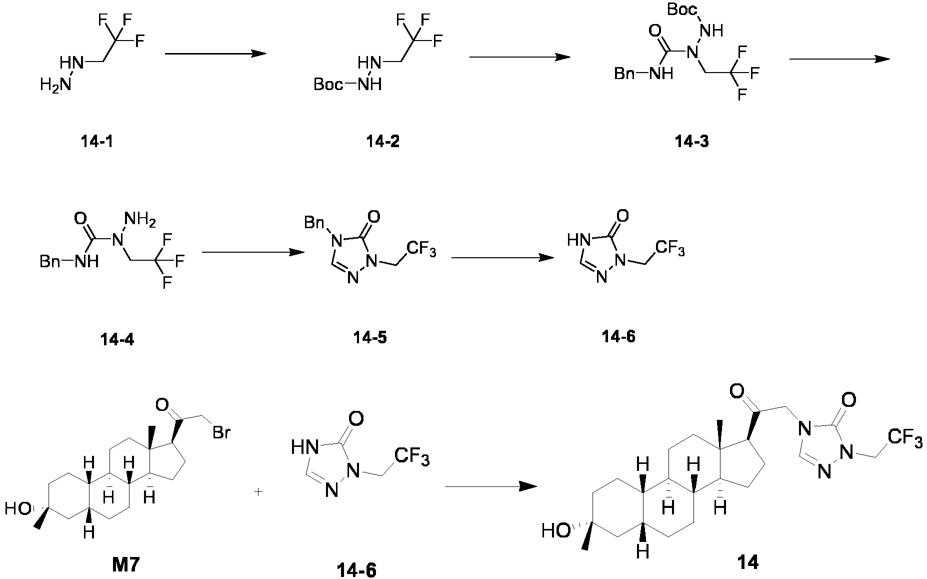

1. **14-1** (7.00 g, 39.9 mmol, 1.00 *eq)* and (Boc)₂O (8.71 g, 39.9 mmol, 9.16 mL, 1.00 *eq)* were added to MeOH (100 mL), and then BnNCO (1.37 g, 10.3 mmol, 1.25 mL, 1.10 *eq)* was added. Then the system was stirred at 25 °C for 12 h and concentrated to give a crude product, and the crude product was separated and purified by column chromatography (petroleum ether/ethyl acetate (100/1-1/1)) to give **14-2** as a white solid (5.00 g, 23.3 mmol, yield: 58.5%). ¹H NMR (400 MHz, CDCl₃)δ 6.35 (br s, 1H), 4.62 - 3.79 (m, 1H), 3.52 - 3.32 (m, 2H), 1.48 (br s, 9H).
**2. 14-2** (2 g, 9.34 mmol, 1.00 *eq)* and DMAP (11.4 mg, 93.4 µmol, 0.01 *eq)* were added to pyridine (20 mL). Then the system was stirred at 60 °C for 12 h, quenched with citric acid (30 mL), and extracted with ethyl acetate (20 mL). The organic phases were combined and then washed with citric acid (30 mL) and saturated brine (20 mL), dried over anhydrous sodium sulfate, and filtered. The organic phase was concentrated under reduced pressure and separated and purified by column chromatography (petroleum ether/ethyl acetate (100/1-1/1)) to give **14-3** as a colorless oily liquid (3.3 g, crude).
3. **14-3** (3.33 g, 9.59 mmol, 1.00 *eq)* was added to a 40 mL sealed tube, and HCl/EtOAc (4.00 M, 2.40 mL, 1.00 *eq)* was added. The system was stirred at 25 °C for 1 h and concentrated to give **14-4** as a colorless oily liquid (2.8 g, crude). ¹H NMR (400 MHz, DMSO-*d₆*) δ 7.68 (br s, 1H), 7.40 - 7.13 (m, 5H), 6.38 - 5.98 (m, 2H), 4.38 - 4.15 (m, 4H).
4. **14-4** (2.80 g, 11.3 mmol, 1.00 *eq)* was dissolved in EtOH (10.0 mL) in a 40 mL sealed tube. Then 10 mL of ethanol was added, and CH(OEt)₃ (3.00 g, 28.3 mmol, 3.10 mL, 2.50 *eq)* was added. The system was stirred at 25 °C for 5 h and concentrated to give a crude product, and the crude product was separated and purified by column chromatography (petroleum ether/ethyl acetate (100/1-1/1)) to give **14-5** as a white solid (1.30 g, 5.05 mmol, yield: 44.6%). ¹H NMR (400 MHz, DMSO-*d₆*)δ 8.20 (s, 1H), 7.53 - 7.14 (m, 5H), 4.83 (s, 2H), 4.68 - 4.52 (m, 2H).
5. **14-5** (800 mg, 3.11 mmol, 1.00 *eq)* was added to a 100 mL hydrogenation flask with a N₂ balloon. Then 5 mL of tetrahydrofuran and 15 mL of ethanol were added, and then Pd/C (10.0 mg, 9.43 µmol, 0.001 *eq)* and Pd(OH)₂ (10.0 mg, 7.12 µmol, 0.001 *eq)* were added. The system was stirred at 25 °C under a H₂ atmosphere (50 Psi) for 12 h and concentrated to give a crude product, and the crude product was purified by Pre-HPLC (column: Welch Xtimate C18 150 × 30 mm × 5 µm; mobile phase: [water (HCl)-ACN]; B%: 0%-24%, 36 min) to give **14-6** as a white solid (170 mg, 1.02 mmol, yield: 21.2%). ¹H NMR (400 MHz, DMSO-*d₆*) δ 11.80 (br s, 1H), 7.95 (d, *J=* 0.8 Hz, 1H), 4.52 (q, *J* = 9.2 Hz, 2H).
6. **14-6** (100 mg, 598 µmol, 1.00 *eq)* was dissolved in 10 mL of tetrahydrofuran, and then the solution was added to a 40 mL sealed tube. M7 (237 mg, 598 µmol, 1.00 *eq)* and K₂CO₃ (827 mg, 5.98 mmol, 10.0 *eq)* were added. The system was stirred at 50 °C for 12 h, quenched with water (20 mL), and extracted with ethyl acetate (20 mL). The organic phases were combined and then washed with saturated brine (20 mL), dried over anhydrous sodium sulfate, and filtered. The organic phase was concentrated under reduced pressure and separated and purified by column chromatography (petroleum ether/ethyl acetate (100/1-1/1)) to give **14** as a white solid (40.0 mg, 77.5 µmol, yield: 12.9%). ¹H NMR (400 MHz, DMSO-*d₆*) δ 7.97 (s, 1H), 4.67 - 4.55 (m, 3H), 4.54 - 4.46 (m, 1H), 4.26 (s, 1H), 2.72 (br t, J = 8.8 Hz, 1H), 2.10 - 2.02 (m, 2H), 1.77 - 1.62 (m, 6H), 1.37 - 1.22 (m, 9H), 1.14 - 0.98 (m, 9H), 0.56 (s, 3H).

### Example 15

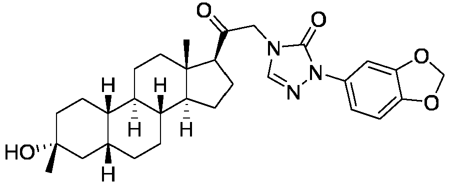

### Synthetic route:

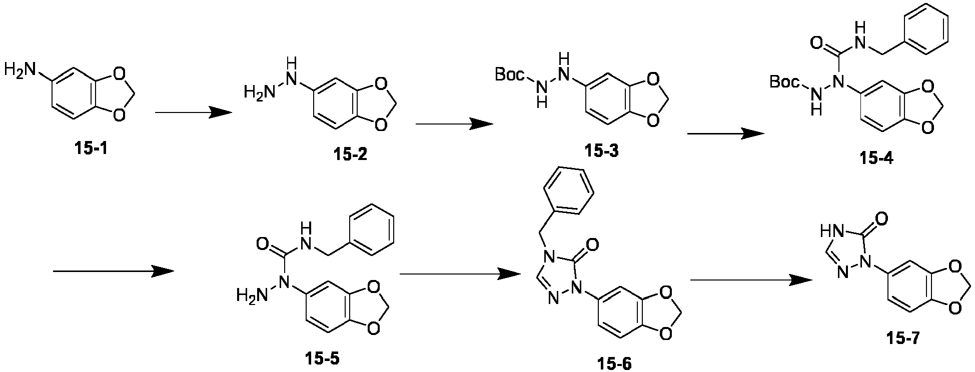

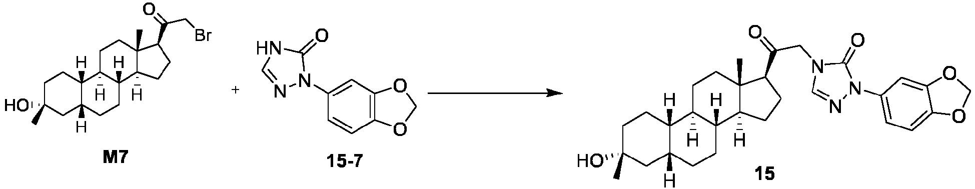

1. **15-1** (10.0 g, 72.9 mmol, 1.00 *eq)* was dissolved in HCl (150 mL). The system was cooled to 0 °C, and then NaNO₂ (5.53 g, 80.2 mmol, 1.10 *eq)* and H₂O (150 mL) were added to the system. The system was stirred at 0 °C for 30 min, and then SnCl₂·2H₂O (49.4 g, 219 mmol, 3.00 *eq)* and HCl (300 mL) were added to the system. The system was stirred at 0 °C for 24 h and filtered, and the filter cake was washed with H₂O (200 mL) and dried to give **15-2** as a dark brown solid (8.40 g, 44.5 mmol, yield: 61.1%, HCl). ¹H NMR (400 MHz, DMSO-*d₆*)δ 10.22 - 9.90 (m, 3H), 6.90 - 6.79 (m, 1H), 6.74 - 6.62 (m, 1H), 6.53 - 6.40 (m, 1H), 6.06 - 5.91 (m, 2H).
**2. 15-2** (5.00 g, 26.5 mmol, 1.00 *eq,* HCl) was dissolved in ACN (45.0 mL), and then DIEA (3.43 g, 26.5 mmol, 4.62 mL, 1.00 *eq)* was added at room temperature. The pH of the system was adjusted to about 8, and (Boc)₂O (5.79 g, 26.5 mmol, 6.09 mL, 1.00 *eq)* was added. The system was stirred at 25 °C for 16 h and concentrated to give a crude product, and the crude product was purified with (ethyl acetate:petroleum ether = 0-10%) to give **15-**3 as a dark brown solid (4.56 g, 18.1 mmol, yield: 68.2%). ¹H NMR (400 MHz, DMSO-*d₆*)δ 8.78 - 8.65 (m, 1H), 7.39 - 7.23 (m, 1H), 6.74 - 6.63 (m, 1H), 6.34 - 6.24 (m, 1H), 6.16 - 6.08 (m, 1H), 5.92 - 5.81 (m, 2H), 1.48 - 1.33 (m, 9H).
**3. 15-3** (4.56 g, 18.1 mmol, 1.00 *eq)* and DMAP (22.1 mg, 181 µmol, 0.01 *eq)* were dissolved in pyridine (50.0 mL), and then benzyl isocyanate (2.65 g, 19.9 mmol, 2.43 mL, 1.10 *eq)* was added. The system was stirred at 60 °C for 16 h, quenched with citric acid (200 mL), and extracted with ethyl acetate (200 mL). The organic phases were combined and then washed with citric acid (300 mL) and saturated brine (200 mL), dried over anhydrous sodium sulfate, and filtered. The organic phase was concentrated under reduced pressure to give a crude product, and the crude product was purified by column chromatography (THF:dichloromethane = 0-10%) to give **15-4** as a yellow solid (5.00 g, 12.7 mmol, yield: 70.0%). ¹H NMR (400 MHz, DMSO-*d₆*)δ = 9.57 (s, 1H), 7.33 - 7.16 (m, 5H), 6.97 - 6.77 (m, 3H), 6.09 - 5.92 (m, 2H), 4.26 (brd, *J* = 5.6 Hz, 2H), 1.46 - 1.26 (m, 9H).
**4. 15-4** (2.00 g, 5.19 mmol, 1.00 *eq)* was added to HCl/dioxane (15.0 mL), and then the system was stirred at 25 °C for 24 h and concentrated to give **15-5** as a green solid (2.00 g, crude). MS (ESI) m/z = 286.0 [M+1]⁺.
5. **15-5** (2.00 g, 7.01 mmol, 1.00 *eq)* was added to EtOH (20.0 mL), and then trimethoxymethane (1.86 g, 17.5 mmol, 1.92 mL, 2.50 *eq)* was added. The system was stirred at 40 °C for 16 h and concentrated to give a crude product, and the crude product was purified by column chromatography (ethyl acetate:petroleum ether = 0-10%) to give **15-6** as a yellow solid (990 mg, 2.73 mmol, yield: 38.9%). MS (ESI) m/z = 295.9 [M+1]⁺, ¹H NMR (400 MHz, DMSO-*d₆*)*δ* = 8.38 - 8.26 (m, 1H), 7.53 - 7.20 (m, 7H), 7.02 - 6.94 (m, 1H), 6.10 - 6.00 (m, 2H), 4.91 - 4.82 (m, 2H).
6. **15-6** (100 mg, 339 µmol, 1.00 *eq)* was added to EtOH (6.00 mL) and THF (2.00 mL), and then Pd(OH)₂ (23.8 mg, 16.9 µmol, 0.05 *eq)* and Pd/C (18.0 mg, 16.9 µmol, 10.0% purity, 0.05 *eq)* were added. The system was stirred at 25 °C for 16 h, then stirred at 60 °C for 16 h, and concentrated to give a crude product, and then the crude product was purified by pre-HPLC (column: Welch Xtimate C18 150 × 30 mm × 5 µm; mobile phase: [water (HCl)-ACN]; B%: 0%-90%, 36 min) to give **15-7** as a white solid (60 mg, 284 µmol, yield: 83.8%). MS (ESI) m/z = 205.9 [M+1]⁺.
7. K₂CO₃ (404 mg, 2.92 mmol, 10.0 *eq),* **M7** (128 mg, 0.322 mmol, 1.10 *eq),* and **15-7** (60 mg, 0.292 mmol, 1.00 *eq)* were added to THF (6 mL). Then the system was stirred at 50 °C for 16 h, quenched with water (20 mL), and extracted with ethyl acetate (20.0 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (0-10%)) to give **15** as a white solid (40 mg, 76.7 µmol, yield: 24.6%). MS (ESI) m/z = 522.2 [M+1]⁺, ¹H NMR (400 MHz, DMSO-*d₆*)δ 8.09 (s, 1H), 7.44 - 7.30 (m, 2H), 6.99 (d, *J =* 8.4 Hz, 1H), 6.06 (s, 2H), 4.73 - 4.49 (m, 2H), 4.28 (s, 1H), 2.78 - 2.72 (m, 1H), 2.13 - 2.03 (m, 2H), 1.82 - 1.56 (m, 6H), 1.43 - 1.26 (m, 7H), 1.17 (br t, J = 7.2 Hz, 3H), 1.13 - 1.01 (m, 8H), 0.58 (s, 3H).

### Example 16

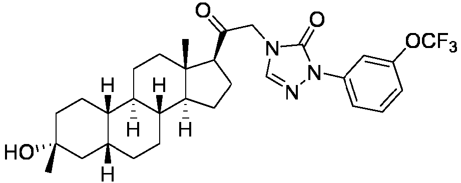

### Synthetic route:

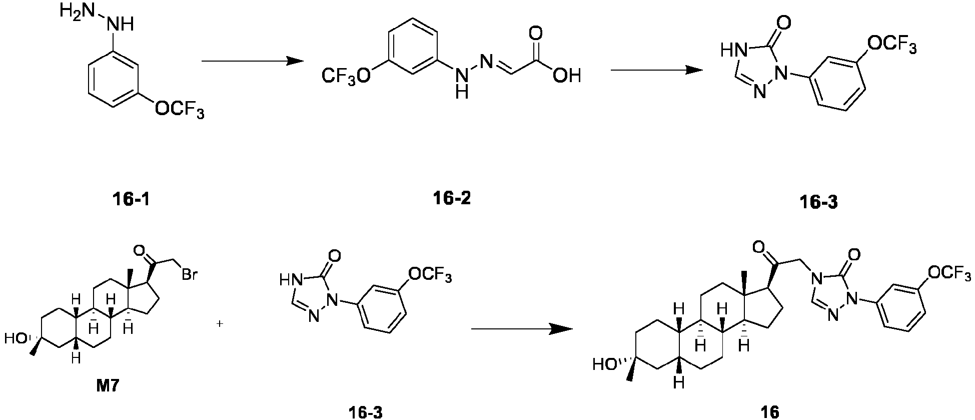

1. **16-1** (2.00 g, 8.75 mmol, 1.00 *eq,* HCl) was added to a round-bottom flask. H₂O (20.0 mL) was added under an ice bath, and then glyoxylic acid (1.94 g, 13.1 mmol, 1.46 mL, 50% purity, 1.50 *eq)* and HCl (12 M, 1.42 mL, 1.95 *eq)* were added. The system was stirred at 25 °C for 2 h and concentrated to give crude **16-2** as a yellow solid (2.4 g). The crude product was directly used in the next step. ¹H NMR (400 MHz, DMSO-*d₆*)δ 12.56 - 12.34 (m, 1H), 11.36 (s, 1H), 7.39 (t, *J* = 8.0 Hz, 1H), 7.16 (s, 1H), 7.10 - 7.03 (m, 2H), 6.85 (br d, *J* = 8.0 Hz, 1H).
**2. 16-2** (500 mg, 2.01 mmol, 1.00 *eq)* was added to a round-bottom flask, and toluene (25.0 mL) and TEA (203.9 mg, 2.01 mmol, 280 µL, 1.00 *eq)* were added. The system was stirred at 25 °C for 0.5 h, and then DPPA (554 mg, 2.01 mmol, 437 µL, 1.00 *eq)* was added. Then the system was stirred at 90 °C for 16 h, quenched with water (20 mL), and extracted with ethyl acetate (20.0 mL × 2). The organic phases were combined and then washed with saturated brine (100 mL), dried over anhydrous sodium sulfate, and filtered. The organic phase was concentrated under reduced pressure and separated and purified by column chromatography (dichloromethane/methanol (0-10%)) to give **16-3** as a yellow solid (290 mg, 1.18 mmol, yield: 58.7%). MS (ESI) m/z = 445.9 [M+1]⁺, ¹H NMR (400 MHz, DMSO-*d₆*)δ = 12.13 (br s, 1H), 8.19 (s, 1H), 7.96 - 7.93 (m, 2H), 7.60 (t, *J* = 8.8 Hz, 1H), 7.22 (br d, *J* = 8.4 Hz, 1H).
3. K₂CO₃ (1.07 g, 7.75 mmol, 10.0 *eq),* **M7** (339 mg, 0.853 mmol, 1.10 *eq),* and **16-3** (190 mg, 0.775 mmol, 1.00 *eq)* were added to THF (20 mL). Then the system was stirred at 50 °C for 16 h, quenched with water (20 mL), and extracted with ethyl acetate (20.0 mL × 2). The organic phases were combined and then washed with saturated brine (20 mL), dried over anhydrous sodium sulfate, and filtered. The organic phase was concentrated under reduced pressure and separated and purified by column chromatography (dichloromethane/methanol (0-10%)) to give **16** as a yellow solid (230 mg, 0.403 mmol, yield: 52.1%). MS (ESI) m/z = 544.2 [M-H₂O]⁺, ¹H NMR (400 MHz, DMSO-*d₆*)*δ* = 8.21 (s, 1H), 7.99 - 7.86 (m, 2H), 7.62 (t, *J =* 8.4 Hz, 1H), 7.25 (br d, *J =* 8.0 Hz, 1H), 4.78 - 4.53 (m, 2H), 4.26 (s, 1H), 2.76 (br t, *J =* 8.8 Hz, 1H), 2.14 - 2.02 (m, 2H), 1.78 - 1.62 (m, 7H), 1.55 - 1.28 (m, 7H), 1.27 - 1.13 (m, 4H), 1.11 (s, 4H), 1.05 (br d, *J =* 10.0 Hz, 2H), 0.59 (s, 3H).

### Example 17

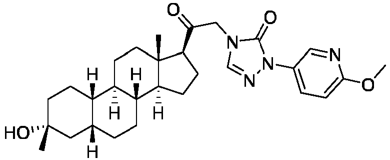

### Synthetic route:

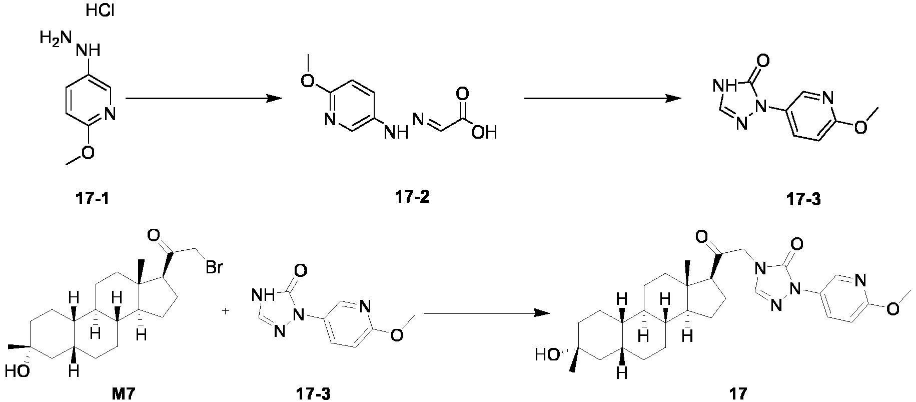

Compound M7 was synthesized using the same method as that in Example 1.
1. 17-1 (1.00 g, 5.69 mmol, 1.00 *eq)* was weighed into a 40 mL sealed tube, and 10 mL of H₂O and HCl (12.0 M, 925 µL, 1.95 *eq)* were added at room temperature. Glyoxylic acid (632 mg, 8.54 mmol, 475 µL, 1.50 *eq)* was added at room temperature. The system was stirred at room temperature for 12 h and lyophilized to give a dark brown solid. The solid was slurried with 10 mL of acetonitrile, and then the slurry was filtered. The filter cake was concentrated and dried to give **17-2** (1.07 g, 5.48 mmol, yield: 96.3%). ¹H NMR (400 MHz, DMSO-*d₆*) δ = 11.52 (br s, 1H), 7.96 (d, *J* = 2.4 Hz, 1H), 7.65 (dd, *J =* 2.8, 9.2 Hz, 1H), 7.17 (s, 1H), 6.90 (d, *J* = 9.2 Hz, 1H), 3.83 (s, 3H).
**2. 17-2** (500 mg, 2.56 mmol, 1.00 *eq)* was weighed into a 40 mL sealed tube, and 10 mL of toluene was at room temperature. DPPA (705 mg, 2.56 mmol, 555 µL, 1.00 *eq)* and TEA (259 mg, 2.56 mmol, 356 µL, 1.00 *eq)* were added at room temperature. The system was stirred at room temperature for 12 h, quenched with water (10.0 mL), and washed with ethyl acetate (10.0 mL × 2). The organic layers were mixed, washed with brine (20.0 mL), dried over Na2SO4, filtered, and concentrated to give a crude product, and the crude product was purified by column chromatography (petroleum ether/ethyl acetate = 100/1-1/1) to give **17-3** as a dark brown solid (100 mg, 520 µmol, yield: 20.3%). ¹H NMR (400 MHz, DMSO-*d₆*)δ = 12.00 (br s, 1H), 10.26 (s, 1H), 8.61 (d, *J* = 2.8 Hz, 1H), 8.16 - 8.10 (m, 2H), 7.84 (d, *J* = 2.8 Hz, 1H), 7.61 (s, 1H), 7.44 - 7.33 (m, 1H), 6.93 (d, *J* = 8.8 Hz, 1H), 6.73 (d, *J* = 8.8 Hz, 1H), 3.87 (s, 3H).
3. K₂CO₃ (863 mg, 6.24 mmol, 10.0 *eq),* **M7** (297 mg, 749 µmol, 1.20 *eq),* and **17-3** (120 mg, 624 µmol, *1 eq)* were added to THF (4.0 mL). Then the system was stirred at 30 °C for 19 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by pre-HPLC (column: YMC Triart 30 × 150 mm × 7 µm; mobile phase: [water(HCl)-ACN]; B%: 44%-84%, 36 min) to give **compound 17** (100 mg, 194 µmol, yield: 31.1%). MS (ESI) m/z = 509.1 [M+H] ⁺, ¹H NMR (400 MHz, DMSO-*d₆*).δ = 8.61 (d, *J=* 2.4 Hz, 1H), 8.22 - 8.04 (m, 2H), 6.95 (d, *J* = 8.8 Hz, 1H), 4.76 - 4.48 (m, 2H), 3.87 (s, 3H), 2.76 (s, 2H), 2.12 - 2.03 (m, 2H), 1.80 - 1.60 (m, 7H), 1.44 - 1.40 (m, 1H), 1.44 - 1.23 (m, 8H), 1.18 - 0.97 (m, 8H), 0.59 (s, 3H).

### Example 18

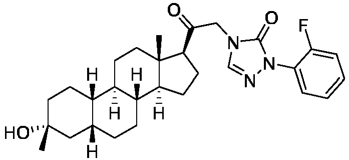

### Synthetic route:

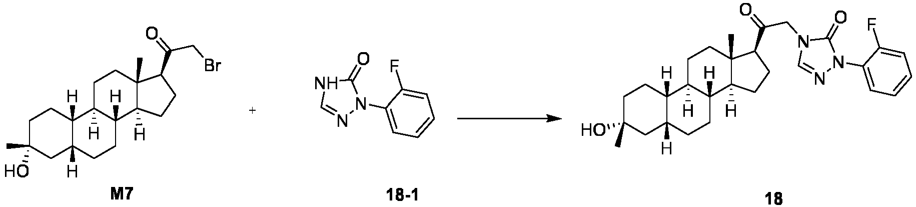

1. K₂CO₃ (870 mg, 6.29 mmol, 10.0 *eq),* **M7** (300 mg, 755 µmol, 1.20 *eq),* and 18-1 (113 mg, 629 µmol, 1.00 *eq)* (18-1 was prepared with reference to the preparation method for 17-3) were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 18** as a white solid (150 mg, 298.1 µmol, yield: 47.39%). MS (ESI) m/z = 478.3 [M-H₂O + H] ⁺, ¹H NMR(400 MHz, DMSO-*d₆*) *δ* = 8.13 (s, 1H), 7.58 - 7.50 (m, 1H), 7.49 - 7.38 (m, 2H), 7.38 - 7.29 (m, 1H), 4.72 - 4.65 (m, 1H), 4.59 - 4.51 (m, 1H), 4.26 (s, 1H), 2.76 (br t, *J* = 8.68 Hz, 1H), 2.14 - 2.02 (m, 2H), 1.79 - 1.64 (m, 6H), 1.61 - 1.44 (m, 2H), 1.43 - 1.21 (m, 9H), 1.17 - 1.02 (m, 7H), 0.58 (s, 3H).

### Example 19

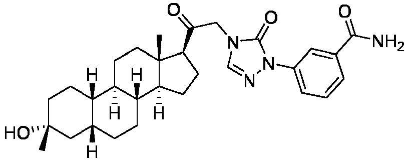

### Synthetic route:

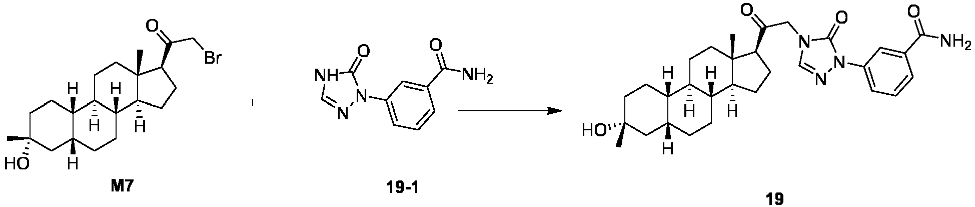

1. K₂CO₃ (609 mg, 4.41 mmol, 10.0 *eq),* **M7** (193 mg, 485 µmol, 1.10 *eq),* and **19-1** (90 mg, 441 µmol, 1.00 *eq)* **(19-1** was prepared with reference to the preparation method for 17-3) were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 19** as a white solid (135 mg, 259 µmol, yield: 58.8%). MS (ESI) m/z = 503.1 [M-H₂O + H] ⁺, ¹H NMR (400 MHz, DMSO-*d₆*) *δ* = 8.41 - 8.35 (m, 1H), 8.19 (s, 1H), 8.09 - 8.03 (m, 1H), 8.11 - 8.03 (m, 1H), 7.77 - 7.70 (m, 1H), 7.55 (t, *J* = 8.0 Hz, 1H), 7.46 (br s, 1H), 4.76 - 4.55 (m, 2H), 4.26 (s, 1H), 2.82 - 2.72 (m, 1H), 2.16 - 2.03 (m, 2H), 1.80 - 1.62 (m, 7H), 1.35 (br d, *J* = 9.6 Hz, 7H), 1.30 - 1.16 (m, 4H), 1.12 (s, 4H), 1.06 (br d, *J =* 10.8 Hz, 2H), 0.60 (s, 3H).

### Example 20

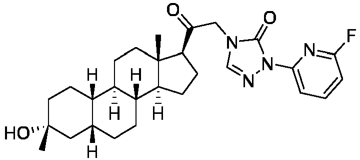

### Synthetic route:

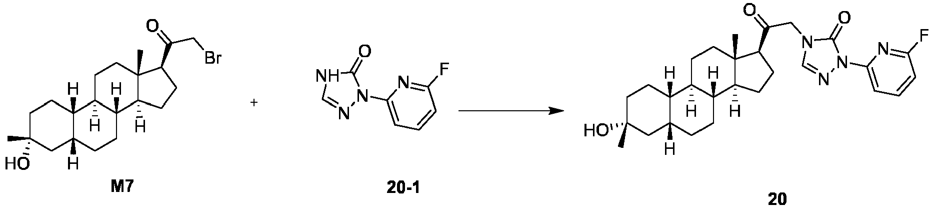

1. K₂CO₃ (580 mg, 4.2 mmol, 10.01 *eq),* **M7** (200 mg, 503.29 µmol, 1.20 *eq),* and **20-1** (75.6 mg, 419.68 µmol, *1.00 eq)* (20-1 was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give compound **20** as a white solid (60 mg, 120.82 µmol, yield: 28.81%). MS (ESI) m/z = 497.3[M + H] ⁺, ¹H NMR (400 MHz, DMSO-*d₆) δ* = 8.18 (s, 1H), 8.12 (q, *J* = 8.4 Hz, 1H), 7.89 (dd, *J* = 8.0, 2.0 Hz, 1H), 7.08 (dd, *J* = 8.00, 2.40 Hz, 1H), 4.75 - 4.67 (m, 1H), 4.61 - 4.53 (m, 1H), 4.25 (s, 1H), 2.80 - 2.72 (m, 1 H), 2.13 - 2.04 (m, 2H), 1.78 - 1.63 (m, 7H), 1.63 - 1.45 (m, 3H), 1.42 - 1.15 (m, 11H), 1.05 (br d, *J* = 8.8 Hz, 3H), 0.59 (s, 3H).

### Example 21

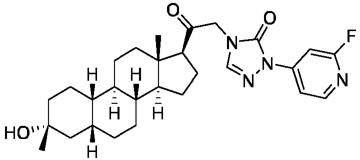

### Synthetic route:

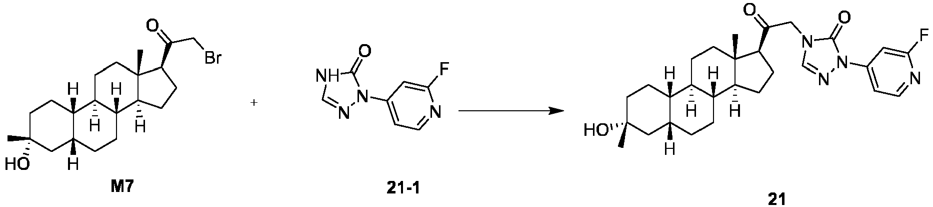

1. K₂CO₃ (1.53 g, 11.1 mmol, 10.0 *eq),* **M7** (485 mg, 1.22 mmol, 1.10 *eq),* and **21-1** (200 mg, 1.11 mmol, 1.00 *eq)* **(21-1** was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 21** as a white solid (155 mg, 304 µmol, yield: 27.3%). MS (ESI) m/z = 497.3[M + H] ⁺, ¹H NMR (400 MHz, DMSO-*d₆)* δ = 8.33 - 8.27 (m, 2H), 7.89 - 7.83 (m, 1H), 7.57 (d, *J* = 1.6 Hz, 1H), 4.77 - 4.56 (m, 2H), 4.25 (s, 1H), 2.81 - 2.73 (m, 1H), 2.13 - 2.02 (m, 2H), 1.78 - 1.59 (m, 7H), 1.53 - 1.03 (m, 17H), 0.59 (s, 3H).

### Example 22

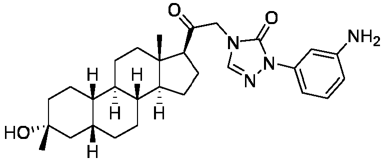

### Synthetic route:

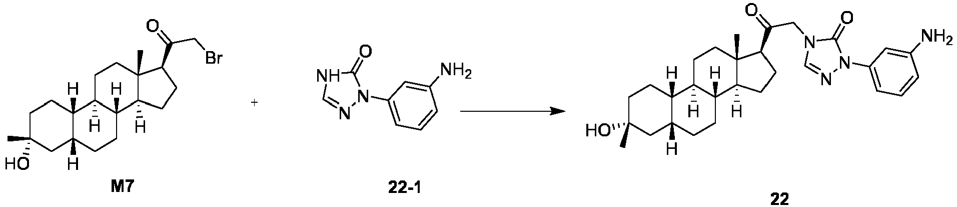

1. K₂CO₃ (1.26 g, 9.08 mmol, 10.0 *eq),* **M7** (200 mg, 503 µmol, 1.10 *eq),* and 22-1 (160 mg, 908 µmol, 1.00 *eq)* **(22-1** was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 22** as a white solid (25 mg, 50.8 µmol, yield: 5.59%). MS (ESI) m/z = 475.3[M-H₂O + H] ⁺, ¹H NMR (400 MHz, DMSO-*d₆)δ* = 8.06 (s, 1H), 7.9 - 7.14 (m, 1H), 7.09 - 6.97 (m, 2H), 6.41 (td, *J* = 2.0, 7.2 Hz, 1H), 5.28 (s, 2H), 4.72 - 4.48 (m, 2H), 4.27 (s, 1H), 2.75 (br t, *J* = 8.8 Hz, 1H), 2.19 - 1.99 (m, 2H), 1.78 - 1.09 (m, 24H), 0.59 (s, 3H).

### Example 23

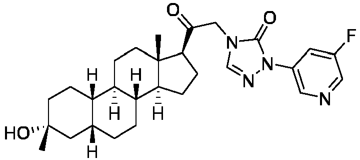

### Synthetic route:

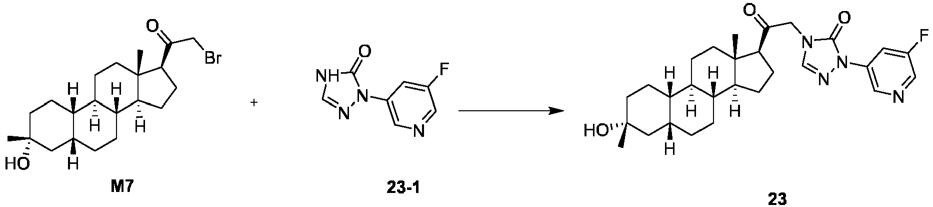

1. K₂CO₃ (844 mg, 6.11 mmol, 10.0 *eq),* **M7** (267 mg, 671.7 µmol, 1.10 *eq),* and **23-1** (110 mg, 610.6 µmol, 1.00 *eq)* **(23-1** was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 23** as a white solid (130 mg, 251 µmol, yield: 41.2%). MS (ESI) m/z = 479.2[M-H₂O + H] ⁺, ¹H NMR (400 MHz, DMSO-*d₆) δ* = 9.03 (s, 1H), 8.51 (d, *J =* 2.4 Hz, 1H), 8.28 (s, 1H), 8.21 - 8.12 (m, 1H), 4.78 - 4.56 (m, 2H), 4.26 (s, 1H), 2.78 (br t, *J* = 8.8 Hz, 1H), 2.15 - 2.04 (m, 2H), 1.76 - 1.23 (m, 22H), 1.05 (br d, *J =* 11.2 Hz, 2H), 0.60 (s, 3H).

### Example 24

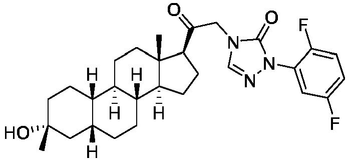

### Synthetic route:

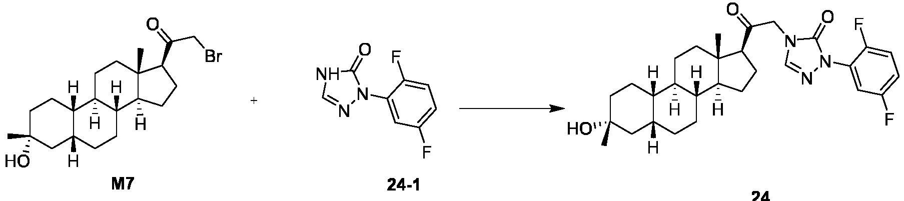

1. K₂CO₃ (350.53 mg, 2.54 mmol, 10.0 *eq),* **M7** (100.8 mg, 256.63 µmol, 1.1 *eq),* and **24-1** (46 mg, 233.3 µmol, *1.0 eq)* **(24-1** was prepared with reference to the preparation method for 17-3) were added to THF (5 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 24** as a white solid (95 mg, 183.1 µmol, yield: 72.2%). MS (ESI) m/z = 496.3[M-H₂O + H] ⁺, ¹H NMR (400 MHz, DMSO-*d₆*).δ = 8.16 (s, 1H), 7.48 (br dd, *J* = 5.2, 9.2 Hz, 2H), 7.40 - 7.26 (m, 1H), 4.76 - 4.48 (m, 2H), 4.27 (s, 1H), 2.74 (br d, *J* = 9.2 Hz, 2H), 2.15 - 2.01 (m, 2H), 1.79 - 1.55 (m, 7H), 1.49 - 1.21 (m, 8H), 1.10 (s, 8H), 0.57 (s, 3H).

### Example 25

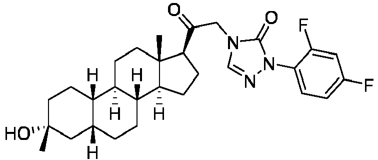

### Synthetic route:

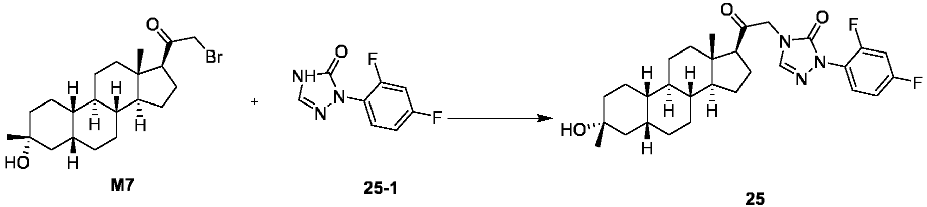

1. K₂CO₃ (350.5 mg, 2.54 mmol, 10.0 *eq),* **M7** (100.79 mg, 256.63 µmol, 1.0 *eq),* and **25-1** (50 mg, 253.63 µmol, *1.0 eq)* **(25-1** was prepared with reference to the preparation method for **17-3)** were added to THF (5 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 25** as a white solid (80 mg, 184.1 µmol, yield: 58.4%). MS (ESI) m/z = 514.0[M+H] ⁺, MS (ESI) m/z = 496.3[M-H₂O + H] ⁺, ¹H NMR (400 MHz, DMSO-*d₆*)δ = 8.13 (s, 1H), 7.65 - 7.57 (m, 1H), 7.55 - 7.48 (m, 1H), 7.24 (br t, *J* = 7.6 Hz, 1H), 4.73 - 4.51 (m, 2H), 4.27 (s, 1H), 2.76 (br t, *J* = 8.4 Hz, 1H), 2.10 - 2.05 (m, 1H), 2.12 - 2.04 (m, 1H), 1.78 - 1.64 (m, 6H), 1.81 - 1.62 (m, 1H), 1.42 - 1.23 (m, 9H), 1.11 (s, 5H), 1.08 - 0.99 (m, 3H), 0.58 (s, 3H).

### Example 26

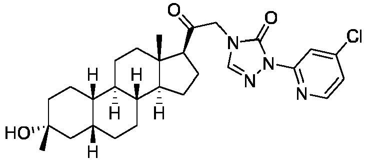

### Synthetic route:

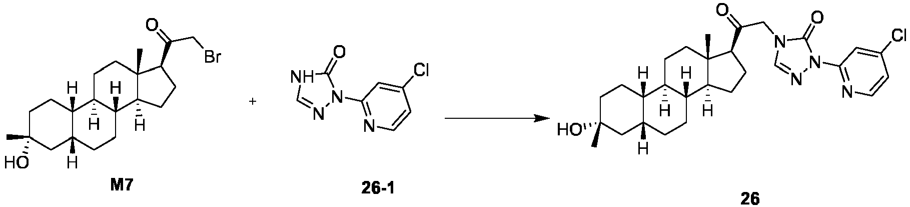

1. K₂CO₃ (562.4 mg, 4.07 mmol, 10.0 *eq),* **M7** (178 mg, 447.6 µmol, 1.1 *eq),* and 26-1 (80 mg, 407 µmol, 1.0 *eq)* **(26-1** was prepared with reference to the preparation method for **17-3)** were added to THF (5 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 26** as a white solid (70 mg, 133 µmol, yield: 32.8%). MS (ESI) m/z = 513.2[M+H] ⁺, ¹H NMR (400 MHz, DMSO-*d₆*) *δ =* 8.48 (d, *J* = 5.6 Hz, 1H), 8.17 - 8.16 (m, 1H), 8.02 (d, *J* = 1.6 Hz, 1H), 7.46 (dd, *J* = 2.0, 5.4 Hz, 1H), 4.73 - 4.54 (m, 2H), 4.42 - 4.37 (m, 1H), 2.79 - 2.73 (m, 1H), 2.11 - 2.04 (m, 2H), 1.79 - 1.64 (m, 7H), 1.41 - 1.23 (m, 9H), 1.11 (s, 8H), 0.58 (s, 3H).

### Example 27

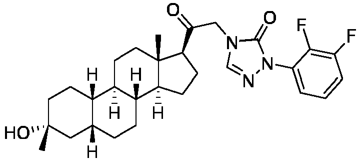

### Synthetic route:

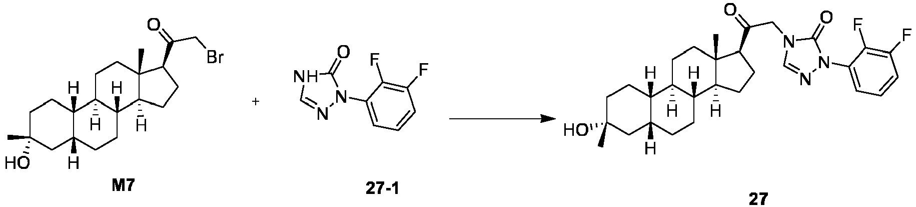

1. K₂CO₃ (701 mg, 5.07 mmol, 10.0 *eq),* **M7** (202 mg, 507 µmol, 1.1 *eq),* and **27-1** (100 mg, 507 µmol, 1.0 *eq)* **(27-1** was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 27** as a white solid (45 mg, 87.18 µmol, yield: 17.19%). MS (ESI) m/z = 496.3[M-H₂O + H] ⁺, ¹H NMR (400 MHz, DMSO-*d₆*) *δ* = 8.18 (s, 1H), 7.60 - 7.46 (m, 1H), 7.45 - 7.31 (m, 2H), 4.81 - 4.64 (m, 1H), 4.61 - 4.52 (m, 1H), 4.27 (s, 1H), 2.76 (br t, *J =* 8.8 Hz, 1H), 2.14 - 2.04 (m, 2H), 1.78 - 1.57 (m, 7H), 1.53 - 1.22 (m, 10H), 1.11 (s, 5H), 1.08 - 0.96 (m, 2H), 0.58 (s, 3H).

### Example 28

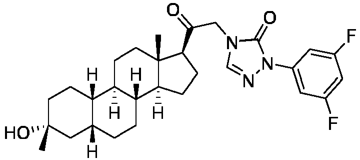

### Synthetic route:

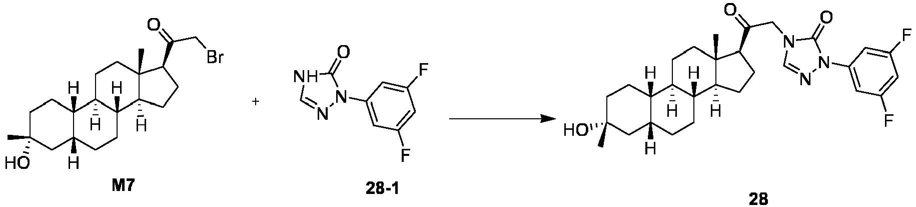

1. K₂CO₃ (347.79 mg, 2.52 mmol, 10.0 *eq),* **M7** (100 mg, 251.64 µmol, 1.1 *eq),* and **28-1** (49.61 mg, 251.64 µmol, *1.0 eq)* **(28-1** was prepared with reference to the preparation method for **17-3)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 28** as a white solid (95 mg, 182.56 µmol, yield: 72.55%). MS (ESI) m/z = 496.3[M-H₂O + H] ⁺, ¹H NMR (400 MHz, DMSO-*d₆*) *δ* = 8.23 (s, 1H), 7.61 (dd, J = 2.4, 9.0 Hz, 2H), 7.14 (br t, J = 2.4 Hz, 1H), 4.81 - 4.46 (m, 2H), 4.26 (s, 1H), 2.76 (s, 1H), 2.17 - 1.96 (m, 2H), 1.84 - 1.19 (m, 17H), 1.16 - 0.96 (m, 7H), 0.59 (s, 3H).

### Example 29

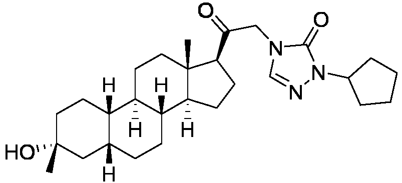

### Synthetic route:

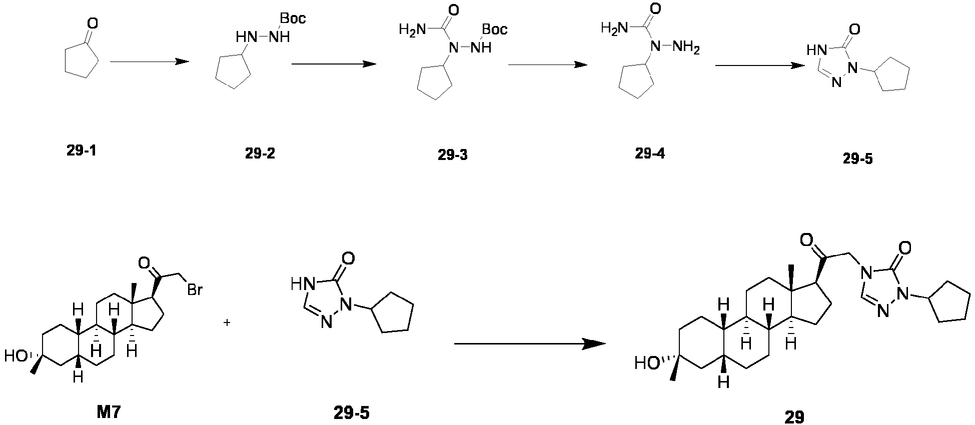

1. **29-1** (10.0 g, 118 mmol, 10.5 mL, 1.00 *eq)* and Boc hydrazine (15.7 g, 118 mmol, 1.00 *eq)* were weighed into a 250 mL reaction flask, and 50 mL of MeOH was added at room temperature. Then NaBH₃CN (14.9 g, 237 mmol, *2.00 eq)* was added at room temperature, and then AcOH (28.1 g, 468 mmol, 26.7 mL, 3.94 *eq)* was added at 0 °C. The mixture was stirred at room temperature for 12 h, and NaHCO₃ (50.0 mL) was added at 0-10 °C. Then 300 mL of DCM was added for extraction, and DCM (50.0 mL × 2) was added to the aqueous phase for extraction. Then the organic phase was washed with 10 mL of brine, then dried over anhydrous Na₂SO₄, and concentrated to give a crude product, and the crude product was purified by column chromatography (petroleum ether/ethyl acetate = 100/1-1 = 1/1) to give **29-2** as a light oily liquid (9.38 g, 46.8 mmol, yield: 39.4%). ¹H NMR (400 MHz, CDCl₃) δ = 6.93 - 6.15 (m, 1H), 4.91 (br s, 1H), 3.45 (br s, 1H), 1.63 (br s, 4H), 1.46 - 1.31 (m, 12H).
2. **29-2** (1.00 g, 4.99 mmol, 1.00 *eq)* was weighed into a 40 mL sealed tube, and 40 mL of i-PrOH (10.0 mL) and TMSNCO (1.73 g, 14.98 mmol, 1.99 mL, 3 *eq)* were added at room temperature. The system was stirred at room temperature for 12 h and concentrated to give a crude product, and the crude product was purified by column chromatography (petroleum ether/ethyl acetate = 100/1-1 = 1/1) to give **29-3** as a white solid (1 g, 4.11 mmol, yield: 82.3%). ¹H NMR (400 MHz, CDCl₃)δ = 6.22 (br s, 1H), 4.61 (quin, *J* = 8.4 Hz, 3H), 1.89 - 1.49 (m, 8H), 1.43 - 1.40 (m, 9H).
3. **29-3** (1.00 g, 4.11 mmol, 1.00 *eq)* was weighed into a 100 mL round-bottom flask, and HCl/dioxane (4.00 M, 1.03 mL, 1.00 *eq)* was added. The system was stirred at room temperature for 2 h and concentrated to give a crude product, and the crude product was purified by column chromatography (petroleum ether/ethyl acetate = 100/1-1 = 1/1) to give **29-4** as a yellow solid (588 mg, yield: 100%). ¹ HNMR (400 MHz, DMSO-*d₆*) δ = 6.53 - 6.51 (m, 1H), 7.57 - 6.06 (m, 1H), 4.44 - 4.28 (m, 1H), 1.81 - 1.29 (m, 8H).
4. **29-4** (700 mg, 4.89 mmol, 1.00 *eq)* was weighed into a 40 mL sealed tube, and EtOH (7 mL), CH(OMe)₃ (1.30 g, 12.2 mmol, 1.34 mL, 2.50 *eq)* was added at room temperature. The system was stirred at room temperature for 12 h and concentrated to give a crude product, and the crude product was purified by column chromatography (petroleum ether/ethyl acetate = 100/1-10/1) to give **29-5** as a white solid (200 mg, 1.27 mmol, yield: 25.9%). ¹H NMR (400 MHz, DMSO-*d₆*)δ = 11.45 (br s, 1H), 7.77 (s, 1H), 4.61 - 4.00 (m, 1H), 1.95 - 1.82 (m, 2H), 1.80 - 1.66 (m, 4H), 1.56 (br d, *J=* 4.4Hz, 2H).
5. K₂CO₃ (902 mg, 6.53 mmol, 10.0 *eq),* **M7** (311 mg, 783 µmol, 1.1 *eq),* and **29-5** (100 mg, 652.82 µmol, 1.0 *eq)* were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 29** as a white solid (150 mg, 312 µmol, yield: 47.8%). MS (ESI) m/z = 470.5[M+ H] ⁺, ¹H NMR(400 MHz, DMSO-*d₆*)δ = 7.80 (s, 1H), 4.65 - 4.36 (m, 3H), 4.24 (s, 1H), 2.70 (br t, *J* = 8.8 Hz, 1H), 2.11 - 1.98 (m, 2H), 1.96 - 1.83 (m, 2H), 1.77 - 1.55 (m, 12H), 1.47 - 1.20 (m, 10H), 1.15 - 0.97 (m, 8H), 0.56 (s, 3H).

### Example 30

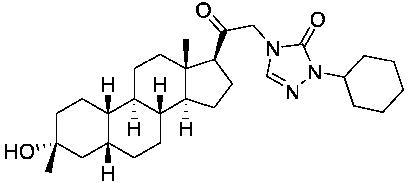

### Synthetic route:

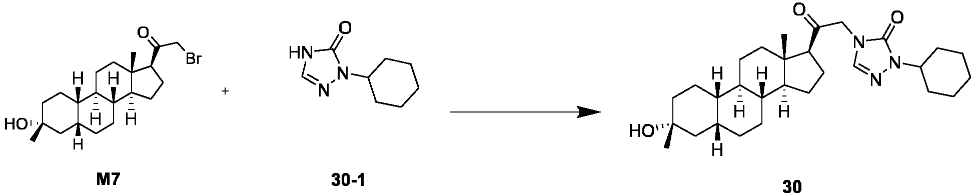

1. K₂CO₃ (827 mg, 5.98 mmol, 10.0 *eq),* **M7** (261 mg, 658 µmol, 1.1 *eq),* and **30-1** (100 mg, 598 µmol, 1.0 *eq)* (30-1 was prepared with reference to the preparation method for **29-5)** were added to THF (10 mL). Then the system was stirred at 50 °C for 16 h, quenched with water (30 mL), and extracted with ethyl acetate (40.0 mL × 2). The organic phases were combined and then washed with saturated brine (40 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 30** as a white solid (40 mg, 82.8 µmol, yield: 13.8%). MS (ESI) m/z = 484.3[M+ H] ⁺, ¹H NMR(400 MHz, DMSO-*d₆*) *δ* = 7.79 (s, 1H), 4.61 - 4.37 (m, 2H), 4.24 (s, 1H), 3.86 (br t, *J* = 11.6 Hz, 1H), 2.74 - 2.65 (m, 1H), 2.12 - 1.98 (m, 2H), 1.81 - 1.53 (m, 14H), 1.46 - 0.99 (m, 20H), 0.56 (s, 3H).

### Example 31

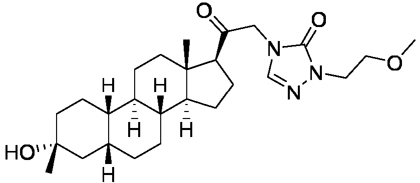

### Synthetic route:

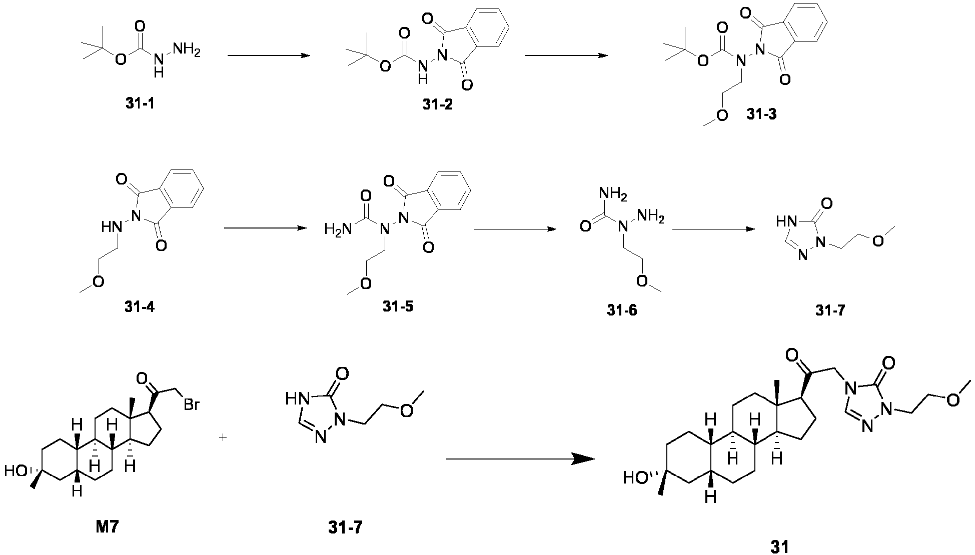

1. Phthalic anhydride (10.0 g, 67.51 mmol, 1.00 *eq)* was weighed into a 1 L three-necked reaction flask, and 200 mL of toluene was added. **31-1** (8.92 g, 67.51 mmol, 1.00 *eq)* was weighed into the system. The system was stirred at 120 °C for 2.5 h and concentrated, and the resulting filter cake was slurried with 100 mL of petroleum ether to give 31-2 as a white solid (17.1 g, 60.64 mmol, yield: 89.82%). ¹H NMR(400 MHz, CDCl₃)*δ* = 7.97 - 7.63 (m, 4H), 6.56 (br s, 1H), 1.44 (br s, 9H).
2. 2-Bromoethyl methyl ether (5.30 g, 38.13 mmol, 3.58 mL, 2.0 *eq)* and **31-2** (5.00 g, 19.06 mmol, 1.00 *eq)* were weighed into a 250 mL three-necked reaction flask. Then 100 mL of acetonitrile was added to the system, and benzyl(triethyl)ammonium chloride (1.74 g, 7.63 mmol, 0.4 *eq)* and K₂CO₃ (7.90 g, 57.19 mmol, 3.0 *eq)* were added at room temperature. The system was stirred at 55 °C for 12 h, quenched with water (100 mL), and extracted with ethyl acetate (100.0 mL × 2). The organic phases were combined and then washed with saturated brine (50 mL), dried over anhydrous sodium sulfate, and filtered. The organic phase was concentrated under reduced pressure, and the crude product was purified by column chromatography (petroleum ether/ethyl acetate = 100/1-3/1) to give the product **31-3** as a white solid (4.7 g, 14.67 mmol, yield: 76.96%). ¹H NMR(400 MHz, CDCl₃)*δ* = 8.05 - 7.56 (m, 4H), 3.98 - 3.72 (m, 2H), 3.68 - 3.46 (m, 2H), 3.35 - 3.07 (m, 3H), 1.56 - 1.30 (m, 10H).
3. **31-3 (4.7** g, 14.67 mmol, 1 *eq)* was weighed into a 100 mL round-bottom reaction flask, and HCl/EtOAc (4 M, 3.67 mL, *1 eq)* was added. The system was stirred at room temperature for 2 h and concentrated to give **31-4** (4.07 g, crude). ¹H NMR(400 MHz, DMSO-*d₆*)δ = 7.85 (s, 4H), 3.44 (t, *J* = 5.6 Hz, 2H), 3.17 (s, 3H), 3.07 (t, *J* = 5.6 Hz, 2H).
4. **31-4** (3.07 g, 13.94 mmol, 1 *eq)* was weighed into a 40 mL sealed tube, and 30 mL of isopropanol was added. Trimethylsilyl isocyanate (4.82 g, 41.82 mmol, 5.56 mL, 3 *eq)* was added. The system was stirred at room temperature for 12 h and concentrated to give **31-5** as a white solid (4.8 g, crude). ¹H NMR (400 MHz, DMSO-*d₆)δ = 7.90* (br d, *J=* 6.0 Hz, 4H), 6.84 - 6.21 (m, 2H), 3.70 (s, 2H), 3.46 - 3.37 (m, 2H), 3.00 (br s, 3H).
5. 50 mL of ethanol was added to a 100 mL three-necked flask, **31-5** (4.80 g, 18.2 mmol, 1.00 *eq)* was weighed into the system, and hydrazine hydrate (1.86 g, 36.47 mmol, 1.81 mL, 98% purity, 2 *eq)* was weighed into the system. The system was stirred at 70 °C for 2 h, washed with 1 M HCl (10 mL), and then extracted with EtOAc (10 mL × 2). The aqueous phase was kept and lyophilized to give **31-6** as a yellow solid (199 mg, crude). ¹H NMR (400 MHz, DMSO-*d₆*)δ = 10.80 - 8.87 (m, 1H), 7.10 - 6.51 (m, 1H), 3.70 (br t, *J* = 5.6 Hz, 2H), 3.51 (br t, *J* = 5.6 Hz, 2H), 3.28 (s, 3H).
6. **31-6** (120 mg, 901.25 µmol, *1 eq)* was weighed into a 40 mL sealed tube, 5 mL of ethanol was added to the system, and trimethoxymethane (239 mg, 2.25 mmol, 247 µL, 2.5 *eq)* was weighed into the system. The system was purified by Pre HPLC (column: Phenomenex C18 75 × 30 mm × 3 µm; mobile phase: [water (HCl)-ACN]; B%: 0%-24%, 36 min) to give **31-7** as a colorless oil (50 mg, 349.30 µmol, yield: 38.76%). ¹H NMR(400 MHz, DMSO-*d₆*)δ = 11.83 - 11.33 (m, 1H), 7.85 (s, 1H), 3.70 (br s, 2H), 3.65 - 3.54 (m, 2H), 3.28 (s, 3H).
7. K₂CO₃ (482.75 mg, 3.49 mmol, 10.0 *eq),* **M7** (138.81 mg, 349.30 µmol, *1 eq),* and **31-7** (50 mg, 349 µmol, *1.0 eq)* were added to THF (5 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 31** as a white solid (105 mg, 222.74 µmol, yield: 63.77%). MS (ESI) m/z = 460.5[M+ H] ⁺, ¹H NMR (400 MHz, DMSO-*d₆*).δ = 7.82 (s, 1H), 4.65 - 4.39 (m, 2H), 3.83 - 3.75 (m, 2H), 3.58 - 3.55 (m, 2H), 3.21 (s, 3H), 2.74 - 2.70 (m, 1H), 2.08 - 1.99 (m, 2H), 1.72 - 1.06 (m, 25H), 0.56 (s, 3H).

### Example 32

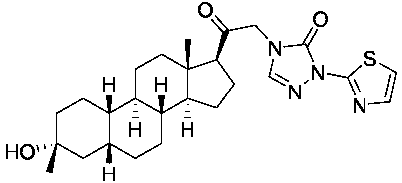

### Synthetic route:

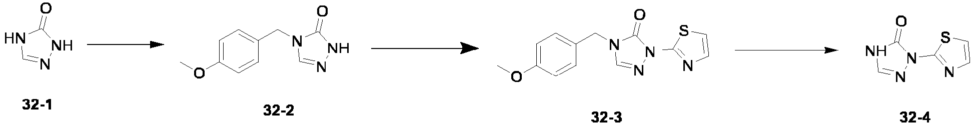

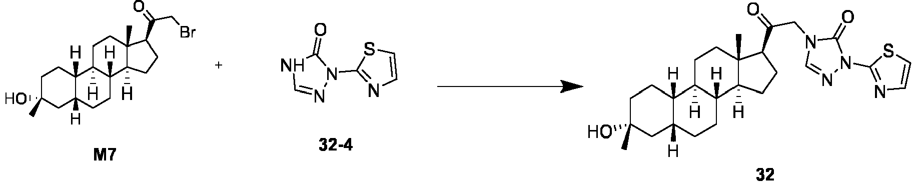

1. **32-1** (20.0 g, 235 mmol, 1.00 *eq*) was weighed into a 250 mL round-bottom flask, and 100 mL of DMF was added. 4-Methoxybenzyl chloride (18.4 g, 117 mmol, 16.0 mL, 0.50 *eq*) was weighed into the system, and K₂CO₃ (48.7 g, 353 mmol, 1.50 *eq)* was weighed into the system. The system was stirred at room temperature for 3 h, and the pH of the system was adjusted to 5. The system was extracted with 100 mL of ethyl acetate, and the aqueous phase was washed with 200 mL of ethyl acetate. The organic phases were combined and then washed with saturated brine (100 mL), dried over anhydrous sodium sulfate, and filtered. The organic phase was concentrated under reduced pressure to give a crude product, and the crude product was purified by column chromatography (petroleum ether/ethyl acetate = 100/0-50/50) to give **32-2** as a white solid (6.20 g, 28.91 mmol, yield: 24.60%). ¹H NMR (400 MHz, DMSO-*d*₆) *δ*= 11.67 (br s, 1H), 7.92 (s, 1H), 7.24 (d, *J* = 8.8 Hz, 2H), 6.94 - 6.89 (m, 2H), 4.66 (s, 2H), 3.74 (s, 3H).
2. **32-2** (1.00 g, 4.87 mmol, 1.00 *eq*) was weighed into a 100 mL three-necked reaction flask. The system was protected with nitrogen gas, and dioxane (50 mL) was added. 2-Bromothiazole (799 mg, 4.87 mmol, 439 µL, 1.00 *eq*) was weighed into the system, and Cs₂CO₃ (3.18 g, 9.75 mmol, 2.00 *eq),* CuI (464 mg, 2.44 mmol, 0.50 *eq*), and KI (647 mg, 3.90 mmol, 0.80 *eq)* were sequentially weighed into the system. The system was stirred at 100 °C for 12 h and concentrated. The filter cake was washed with 30 mL of ethyl acetate and concentrated. The crude product was purified by column chromatography (petroleum ether/ethyl acetate = 100/0-60/40) to give **32-3** as a white solid (170 mg, 590 µmol, yield: 12.1%). ¹H NMR (400 MHz, DMSO-*d6) δ* = 8.45 (s, 1H), 7.60 (d, *J* = 3.6 Hz, 1H), 7.51 (d, *J =* 3.6 Hz, 1H), 7.33 (d, *J =* 8.4 Hz, 2H), 6.99 - 6.87 (m, 2H), 4.83 (s, 2H), 3.74 (s, 3H).
3. **32-3** (170 mg, 590 µmol, 1.00 *eq*) was weighed into a 40 mL sealed tube, and 0.5 mL of water and 5 mL of acetonitrile were added to the system. Ammonium cerium(IV) nitrate (970 mg, 1.77 mmol, 882 µL, 3.00 *eq*) was weighed into the system. The system was stirred at room temperature for 12 h, concentrated, and filtered, and then the filter cake was washed with 10 mL of ethyl acetate. The filtrate was concentrated to give a crude product, and the crude product was purified by column prep-HPLC (column: Welch Xtimate C18 150 × 30 mm × 5 µm; mobile phase: [water (NH4HCO3)-ACN]; B%: 0%-16%, 28 min) to give **32-4** (30 mg, 178.38 µmol, yield: 30.25%). MS (ESI) m/z = 169 [M+H] ⁺, ¹H NMR (400 MHz, DMSO-*d6*) *δ* = 7.85 - 7.77 (m, 1H), 7.37 (d, *J* = 3.6 Hz, 1H), 7.08 (d, *J* = 3.6 Hz, 1H), 6.86 (d, *J =* 8.8 Hz, 1H).
4. K₂CO₃ (246.54 mg, 1.78 mmol, 10.0 *eq*), **M7** (71 mg, 178.38 µmol, 1 *eq*), and **32-4** (30 mg, 178 µmol, 1.0 *eq)* were added to THF (4 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 32** as a white solid (20 mg, 38.4 µmol, yield: 21.51%). MS (ESI) m/z = 485.1[M+ H] ⁺, ¹H NMR (400 MHz, DMSO-*d₆) δ* = 8.25 (s, 1H), 7.61 (d, *J =* 3.6 Hz, 1H), 7.51 (d, *J =* 3.6 Hz, 1H), 4.83 - 4.68 (m, 1H), 4.64 - 4.54 (m, 1H), 4.30 (s, 1H), 2.76 (br t, *J* = 8.8 Hz, 1H), 2.09 - 2.03 (m, 2H), 1.74 - 1.01 (m, 24H), 0.58 (s, 3H).

### Example 33

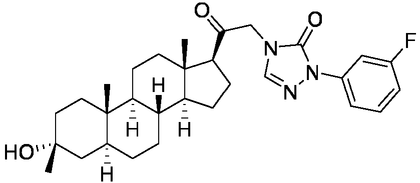

### Synthetic route:

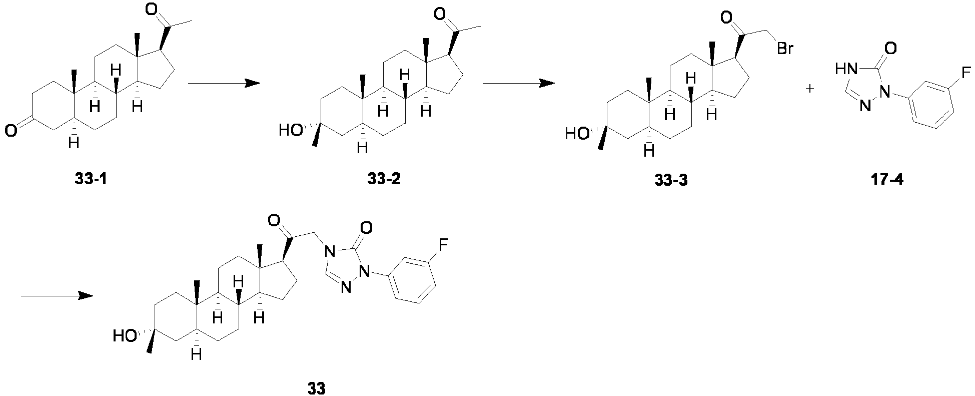

1. 15 mL of THF was added to a 100 mL three-necked flask, and the system was protected with N₂ gas. **33-1** (1 g, 3.16 mmol, 1.00 *eq*) was weighed into the system, and MeMgBr (3 M, 1.05 mL, 1 *eq*) was weighed into the system at 0 °C. The system was left to react at 0 °C for 0.5 h, quenched with an aqueous ammonium chloride solution (500 mL), and extracted with ethyl acetate (200 mL × 2). The organic phases were combined and then washed with saturated brine (10 mL), dried over anhydrous sodium sulfate, and filtered. The organic phase was concentrated under reduced pressure, and the crude product was purified by Pre-HPLC (column: Phenomenex C18 75 × 30 mm × 3 µm; mobile phase: [water(HCl)-ACN]; B%: 36%-76%, 36 min) to give **33-2** as a red solid (150 mg, 451.10 µmol, yield: 14.28%). ¹H NMR(400 MHz, CDCl₃)*δ* = 2.54 (t, *J* = 8.8 Hz, 1H), 2.21 - 2.09 (m, 4H), 2.01 (td, *J =* 3.2, 12.0 Hz, 1H), 1.63 - 1.49 (m, 10H), 1.43 - 1.22 (m, 12H), 0.96 (dd, *J* = 5.6, 12.3 Hz, 1H), 0.82 - 0.79 (m, 1H), 0.76 (s, 3H), 0.61 (s, 3H).
2. **33-2** (150 mg, 451.10 µmol, 1.00 *eq*) was weighed into a 40 mL sealed tube, and the system was protected with N₂ gas. 1.5 mL of methanol was added at room temperature, and HBr (14.75 mg, 87.51 µmol, 9.90 µL, 0.194 *eq*) and Br₂ (72.09 mg, 451.10 µmol, 23.25 µL, 1 *eq*) were added at 0 °C. Then the system was stirred at room temperature for 12 h, quenched with an aqueous sodium bicarbonate solution (5 mL), and extracted with ethyl acetate (5 mL × 2). The organic phases were combined and then washed with saturated brine (10 mL), dried over anhydrous sodium sulfate, and filtered. The organic phase was concentrated under reduced pressure, and the crude product was purified by column chromatography (petroleum ether/ethyl acetate = 100/1-1/1) to give **33-3** as a white solid (150 mg, 364.60 µmol, yield: 80.82%). ¹H NMR (400 MHz, CDCl₃)*δ* = 3.84 (d, *J* = 2.9 Hz, 2H), 2.75 (t, *J* = 8.8 Hz, 1H), 2.17 - 2.04 (m, 1H), 1.71 - 1.57 (m, 4H), 1.55 (br d, *J* = 3.2 Hz, 5H), 1.43 (br d, *J* = 2.4 Hz, 3H), 1.39 (br s, 5H), 1.25 - 1.13 (m, 4H), 1.12 - 1.04 (m, 2H), 0.89 (br dd, *J* = 5.2, 12.1 Hz, 1H), 0.74 (br s, 1H), 0.68 (s, 3H), 0.56 (s, 3H).
3. K₂CO₃ (335.93 mg, 2.43 mmol, 10.0 *eq*), **33-4** (43.55 mg, 243.06 µmol, 1 *eq*), and **33-3** (100 mg, 243.06 µmol, 1.0 *eq*) were added to THF (5 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 33** as a white solid (20 mg, 38.93 µmol, yield: 16.02%). MS (ESI) m/z = 510.1 [M + H] ⁺, ¹H NMR (400 MHz, DMSO-*d₆*)*δ* = 8.21 (s, 1H), 7.84 - 7.69 (m, 2H), 7.64 - 7.43 (m, 1H), 7.11 (br d, *J =* 2.0 Hz, 1H), 4.81 - 4.55 (m, 2H), 3.88 (s, 1H), 2.85 - 2.67 (m, 1H), 2.22 - 1.90 (m, 2H), 1.80 -0.75 (m, 23H), 0.74 (s, 3H), 0.60 (s, 3H).

### Example 34

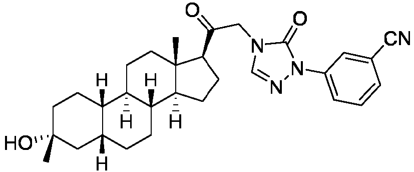

### Synthetic route:

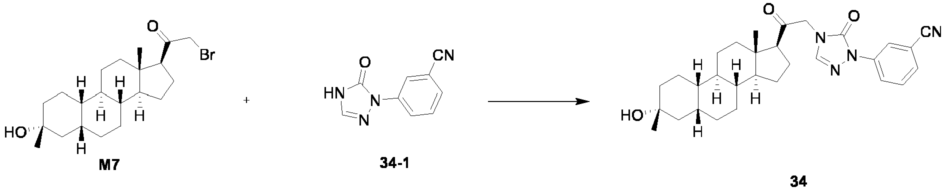

1. K₂CO₃ (742 mg, 5.37 mmol, 10.0 *eq*), **M7** (100 mg, 537 µmol, 1 *eq*), and **34-1** (235 mg, 591 µmol, 1.1 *eq*) were added to THF (15 mL). Then the system was stirred at 50 °C for 16 h, quenched with water (20 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and the crude product was separated by SFC (column: DAICEL CHIRALCEL OD (250 mm × 30 mm, 10 µm); mobile phase: [0.1% NH₃H₂O ETOH]; B%: 60%-60%, min) to give a crude product. The crude product was separated and purified by column chromatography (dichloromethane/methanol (100/0-95/5)) to give **compound 34** as a white solid (63 mg, 122 µmol, yield: 22.8%). MS (ESI) m/z = 485.3[M-H₂O+H] ⁺, ¹H NMR (400 MHz, DMSO-*d₆) δ* = 8.35 - 8.11 (m, 3H), 7.81 - 7.60 (m, 2H), 4.79 - 4.52 (m, 2H), 4.26 (s, 1H), 2.77 (br t, *J* = 8.8 Hz, 1H), 2.19 - 1.95 (m, 2H), 1.81 - 1.46 (m, 9H), 1.43 - 0.99 (m, 15H), 0.59 (s, 3H).

### Example 35

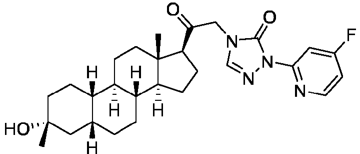

### Synthetic route:

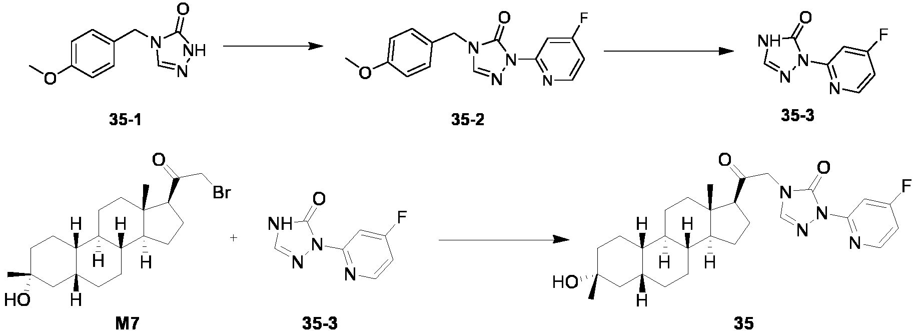

Compound **M7** was synthesized using the same method as that in Example 1.
1. Compound **35-1** (1.03 g, 5.00 mmol, 0.80 *eq*) was added to a 100 mL three-necked flask with nitrogen gas (R1), and dibromotetrafluoropyridine (1.10 g, 6.25 mmol, 1.00 *eq*) was added to the reaction system. Cs₂CO₃ (4.07 g, 12.5 mmol, 2.00 *eq*) was added to R1 at 25 °C. Potassium iodide (830.08 mg, 5.00 mmol, 0.80 *eq*) was added to R1. CuI (595.20 mg, 3.13 mmol, 0.50 *eq*) was added to R1 at 25 °C. 1,10-Phenanthroline (788 mg, 4.38 mmol, 0.70 *eq*) was added to R1 at 25 °C. Dioxane (20.0 mL) was added to R1 at 25 °C. R1 was stirred at 100 °C for 2 h. Thin-layer chromatography analysis (petroleum ether/ethyl acetate = 1/3) indicated that compound **35-1** was consumed, and a new spot appeared. The mixture was quenched with water (10 mL) and extracted with ethyl acetate (10 mL × 2). The combined organic phases were washed with brine (10 mL), dried over Na₂SO₄, filtered, and concentrated to give a crude product, and the crude product was purified by silica gel column chromatography (petroleum ether/ethyl acetate = 100/1-1/3) to give compound **35-2** as a yellow solid (490 mg, 1.63 mmol, yield: 26.11%). ¹H NMR (400 MHz, DMSO-*d₆)*δ = 8.56 - 8.47 (m, 1H), 8.37 (s, 1H), 7.82 (dd, *J* = 2.4, 11.2 Hz, 1H), 7.32 (s, 2H), 7.29 - 7.22 (m, 1H), 6.94 (d, *J =* 8.8 Hz, 2H), 4.81 (s, 2H), 3.74 (s, 3H).
2. Compound **35-2** (100 mg, 333.01 µmol, 1.00 *eq*) was added to a 40 mL sealed tube (R1). With the temperature controlled at 25 °C, acetonitrile (10 mL) was added to R1. With the temperature controlled at 25 °C, ceric ammonium nitrate (547.70 mg, 999.04 µmol, 497.91 µL, 3.00 *eq*) was added to R1. The mixture was stirred at 60 °C for 12 h and concentrated to give a crude product, and the crude product was purified by column chromatography to give compound **35-3** as a white solid (40 mg, 83.49 µmol, yield: 25.07%). ¹H NMR (400 MHz, DMSO-*d₆)*δ = 12.11 (br s, 1H), 8.51 (dd, *J =* 5.6, 9.1 Hz, 1H), 8.16 (s, 1H), 7.84 (dd, *J =* 2.4, 11.1 Hz, 1H), 7.24 (br t, *J =* 2.4 Hz, 1H).
3. K₂CO₃ (306 mg, 2.22 mmol, 10.0 *eq*), **M7** (88.2 mg, 222 µmol, 1.00 *eq),* and **35-3** (40 mg, 222.05 µmol, 1.00 *eq)* were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 35** as a white solid (30.0 mg, 60.23 µmol, yield: 27.12%). MS (ESI) m/z = 497 [M+H] ⁺, ¹H NMR (400 MHz, DMSO-*d*₆)δ = 8.53 (dd, *J* = 5.6, 9.2 Hz, 1H), 8.18 (s, 1H), 7.77 (dd, *J* = 2.4, 10.8 Hz, 1H), 7.28 (ddd, *J* = 2.4, 5.6, 8.4 Hz, 1H), 4.87 - 4.43 (m, 2H), 2.76 (br t, *J =* 8.8 Hz, 1H), 2.17 - 1.97 (m, 2H), 1.89 - 1.17 (m, 18H), 1.14 - 0.86 (m, 7H), 0.58 (s, 3H).

### Example 36

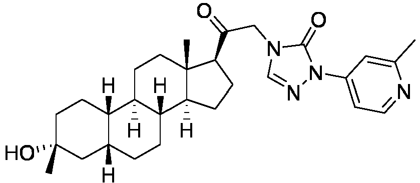

### Synthetic route:

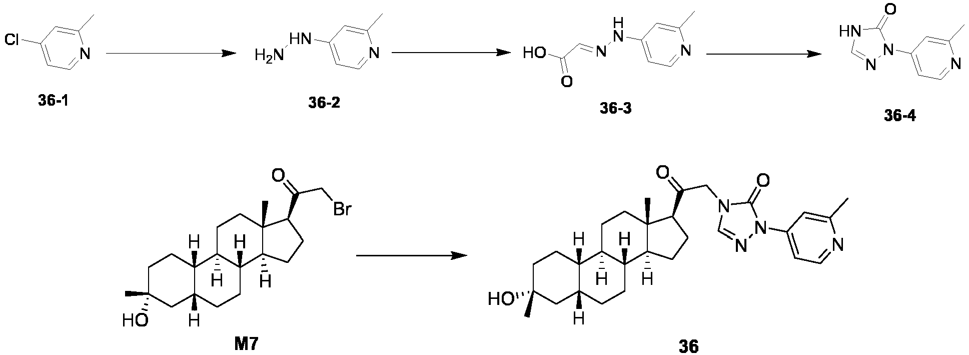

1. Compound **36-1** (5 g, 39.1 mmol, 1.00 *eq)* was added to a 40 mL sealed tube (R1). With the temperature controlled at 25 °C, N2H4·H2O (20.0 g, 391 mmol, 19.4 mL,10.0 *eq*) was added to R1. R1 was stirred at 100 °C for 12 h, and the mixture was concentrated to give crude compound **36-2** as a white solid (2.10 g, 17.05 mmol, yield: 43.51%).
2. Water (12.0 mL) was poured into a 250 mL round-bottom flask (R1). With the temperature controlled at 25 °C, hydrochloric acid (12 M, 1.00 mL, 2.79 *eq*) was added to R1. With the temperature controlled at 25 °C, compound **36-2** (530 mg, 4.30 mmol, 1.00 *eq*) was added to R1 at 25 °C. Glyoxylic acid (955 mg, 6.46 mmol, 718 µL, 1.50 *eq)* was added to R1 at 25 °C. With the temperature controlled at 25 °C, R1 was stirred for 2 h to give **36-3.** The mixture was concentrated by lyophilization to give compound **36-3** as a yellow solid (910 mg). MS (ESI) m/z = 180 [M+H] ⁺, 1H NMR (400 MHz, DMSO-d₆)δ = 13.52 (br s, 1H), 8.34 (br d, J = 6.4 Hz, 1H), 7.70 (s, 1H), 7.52 - 7.00 (m, 2H), 2.59 (s, 3H).
3. Compound **36-3** (1.00 g, 5.58 mmol, 1.00 *eq*) was added to a 40 mL sealed tube (R1). DPPA (1.54 g, 5.58 mmol, 1.20 mL, 1.00 *eq*) was added to R1. TEA (1.13 g, 11.2 mmol, 1.55 mL, 2.00 *eq*) was added to R1. Toluene (5.00 mL) was added to R1. The mixture was stirred for 12 h, and from the crude product, compound **36-4** was obtained as a yellow solid (30 mg, 170.29 µmol, yield: 3.05%). MS (ESI) m/z = 177 [M+H] ⁺, ¹H NMR (400 MHz, DMSO-*d*₆) δ = 12.62 (br s, 1H), 8.73 (d, *J* = 6.6 Hz, 1H), 8.45 (s, 1H), 8.33 - 8.17 (m, 2H), 2.74 (s, 3H).
4. K₂CO₃ (156.90 mg, 1.14 mmol, 10 *eq*), **M7** (45.11 mg, 113.52 µmol, 1 *eq*), and **36-4** (20 mg, 113.52 µmol, 1.00 *eq)* were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 36** as a white solid (15 mg, 30.17 µmol, yield: 27.12%). MS (ESI) m/z = 493[M+H] ⁺, ¹H NMR (400 MHz, DMSO-*d*₆) δ = 8.75 *(d, J = 7.2* Hz, 1H), 8.45 (s, 1H), 8.33 - 8.11 (m, 2H), 4.92 - 4.46 (m, 2H), 2.83 - 2.69 (m, 4H), 2.06 (br d, *J* = 9.6 Hz, 2H), 1.83 - 1.18 (m, 18H), 1.14 - 0.85 (m, 7H), 0.59 (s, 3H).

### Example 37

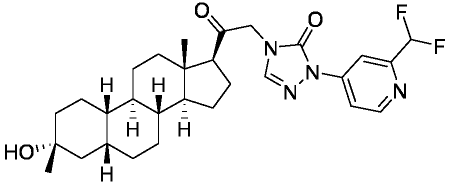

### Synthetic route:

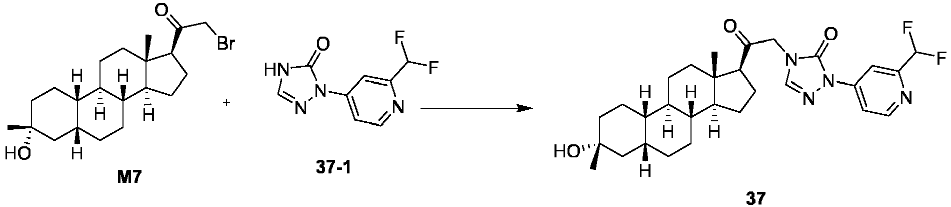

1. K₂CO₃ (195.43 mg, 1.41 mmol, 10.0 *eq*), **M7** (56.19 mg, 141.41 µmol, 1.00 *eq),* and **37-1** (prepared with reference to the preparation method for **36-4)** (30 mg, 141.41 µmol, 1.00 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 37** as a white solid (26 mg, 49.18 µmol, yield: 34.78%). MS (ESI) m/z = 529[M+H] ⁺, ¹H NMR (400 MHz, DMSO-*d*₆) δ = 8.66 (d, *J =* 5.6 Hz, 1H), 8.30 (d, *J =* 2.0 Hz, 1H), 8.10 (d, 1H), 7.67 (s, 1H), 6.66 (s, 1H), 4.76 - 4.28 (m, 2H), 2.73 - 2.54 (m, 1H), 2.14 (s, 2H), 1.89 - 1.62 (m, 9H), 1.54 - 1.20 (m, 16H), 0.69 (s, 3H).

### Example 38

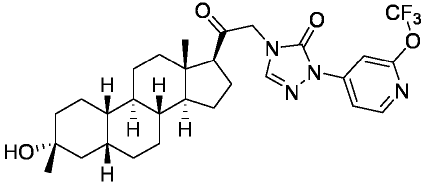

### Synthetic route:

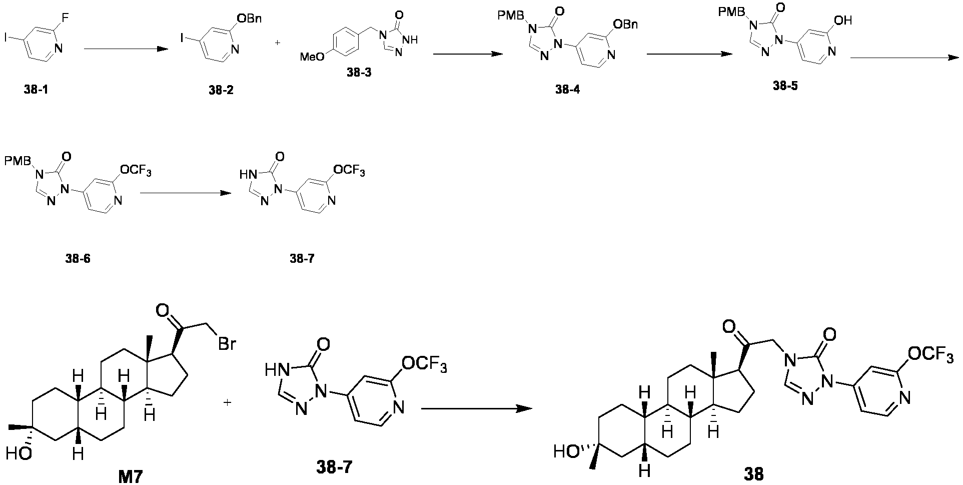

1. Sodium hydride (2.69 g, 67.3 mmol, 60.0% purity, 1.50 *eq*) was dissolved in 25.0 mL of dimethylformamide, and then the solution was added to a 250 mL three-necked flask (R1) protected with a nitrogen balloon. Benzyl alcohol (4.85 g, 44.9 mmol, 4.65 mL, 1.00 *eq*) was added to R1 at room temperature, and the reaction mixture was stirred at room temperature for half an hour. Compound **38-1**(10.0 g, 44.9 mmol, 1.00 *eq*) was dissolved in 30.0 mL of dimethylformamide, and then the solution was added to R1 at 0 °C. R1 was stirred at 0 °C for half an hour. Saturated ammonium chloride (50.0 mL) was added to R1 at 10 °C-20 °C. After the reaction mixture was extracted with ethyl acetate (50.0 mL), the organic phase was separated from the aqueous phase. The aqueous phase was further extracted twice with ethyl acetate (30.0 mL). The organic phase was washed with water (50.0 mL) 5 times and then with saturated brine (100 mL), then dried over a proper amount of anhydrous sodium sulfate, and finally filtered and concentrated to give a crude product. The crude product (petroleum ether:ethyl acetate = 10:1, R_{f} = 0.48) was purified by silica gel column chromatography (ethyl acetate/petroleum ether = 0-10%) to give compound **38-2** as a colorless oil (11.9 g, 38.3 mmol, yield: 85.3%).
2. Compound **38-3** (989 mg, 4.82 mmol, 1.00 *eq*) was added to a 40.0 mL sealed tube (R1). Degassed dioxane (20.0 mL) was added to R1 at room temperature. Compound **38-2** (1.50 g, 4.82 mmol, 1.00 *eq*) was added to R1 at room temperature. Cesium carbonate (3.14 g, 9.64 mmol, 2.00 *eq)* was added to R1 at room temperature. Potassium iodide (640 mg, 3.86 mmol, 0.80 *eq*) was added to R1 at room temperature. 1,10-Phenanthroline (608 mg, 3.37 mmol, 0.70 *eq*) was added to R1 at room temperature. Cuprous iodide (459 mg, 2.41 mmol, 0.50 *eq)* was added to R1 at room temperature. R1 was stirred at 100 °C for 2 h, and the reaction mixture in R1 was filtered. Ethyl acetate (20.0 mL) was added to R1 at room temperature. The organic phase was separated from the aqueous phase. The aqueous phase was further extracted twice with ethyl acetate (20.0 mL). Then the organic phase was washed with saturated brine (20.0 mL), dried over a proper amount of anhydrous sodium sulfate, and finally filtered and concentrated to give a crude product. The crude product (petroleum ether/ethyl acetate = 3/1, R_{f} = 0.28) was purified by silica gel column chromatography (ethyl acetate/petroleum ether = 0-10%) to give compound **38-4** as a yellow solid (952 mg, 2.45 mmol, yield: 17.0%).
3. Compound **38-4** (950 mg, 2.45 mmol, 1.00 *eq*) was added to a 50.0 mL single-neck flask (R1) protected with a hydrogen balloon (15.0 Psi). Tetrahydrofuran (20.0 mL) was added to R1 at room temperature, and Pd/C (26.0 mg, 24.5 µmol, 0.01 *eq*) was added to R1 at room temperature. R1 was stirred at 25 °C for 10 h and stirred at 40 °C for 12 h. Pd/C (52.1 mg, 48.9 µmol, 0.02 *eq)* was added to R1 at room temperature, and R1 was stirred at 25 °C for 1 h. The reaction mixture was filtered, and the filtrate was concentrated to give compound **38-5** as a yellow solid (185 mg, 620 µmol, yield: 25.4%).
4. Compound **38-5** (185 mg, 620 µmol, 3.00 *eq*) was added to a 40.0 mL AK flask (R1). Nitromethane (17.2 g, 282 mmol, 15.3 mL, 1365 *eq)* was added to R1 at room temperature, and 1-(trifluoromethyl)-1,2-benziodoxol-3(1H)-one (65.3 mg, 207 µmol, 1.00 *eq*) was added to R1 at room temperature. R1 was stirred at 100 °C for 16 h. After the reaction mixture was concentrated, the resulting crude product was separated using a conventional reversed-phase column to give compound **38-6** as a yellow solid (25.0 mg, 60.1 µmol, yield: 88.0%).
5. Compound **38-6** (25.0 mg, 68.3 µmol, 1.00 *eq*) was added to a 40.0 mL AK flask (R1). Acetonitrile (10.0 mL) was added to R1 at room temperature, and ceric ammonium nitrate (112 mg, 205 µmol, 102 µL, 3.00 *eq*) was added to R1 at room temperature. R1 was stirred at 50 °C for 12 h. Ceric ammonium nitrate (112 mg, 205 µmol, 102 µL, *3.00 eq*) was added to R1 at room temperature, and R1 was stirred at 60 °C for 10 h. Water (20.0 mL) was added to R1 at room temperature. After the reaction mixture was extracted with ethyl acetate (20.0 mL), the organic phase was separated from the aqueous phase. The aqueous phase was further extracted twice with ethyl acetate (10.0 mL). The organic phase was washed with saturated brine (20.0 mL), then dried over a proper amount of anhydrous sodium sulfate, and finally filtered and concentrated to give compound **38-7** as a yellow solid (20.0 mg, crude). MS (ESI) m/z = 247.0 [M+H]⁺.
6. K₂CO₃ (127 mg, 918 µmol, 10.0 *eq*), **M7** (36.5 mg, 91.8 µmol, 1.00 *eq*), and **38-7** (20.0 mg, 81.3 µmol, 8.85 *eq*-1 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 38** as a pale yellow solid (8.2 mg, 13.7 µmol, yield: 15%). MS (ESI) m/z = 563.1[M+H] ⁺, ¹H NMR (400 MHz, CHLOROFORM-d) *δ* = 8.33 (d, *J* = 5.6 Hz, 1H), 7.93 (dd, *J* = 1.6, 5.6 Hz, 1H), 7.81 - 7.59 (m, 2H), 4.72 - 4.29 (m, 2H), 2.74 - 2.51 (m, 1H), 2.25 - 2.05 (m, 2H), 1.87 - 1.75 (m, 5H), 1.55 - 1.39 (m, 7H), 1.36 - 1.07 (m, 13H), 0.69 (s, 3H).

### Example 39

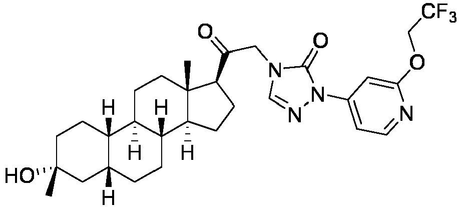

### Synthetic route:

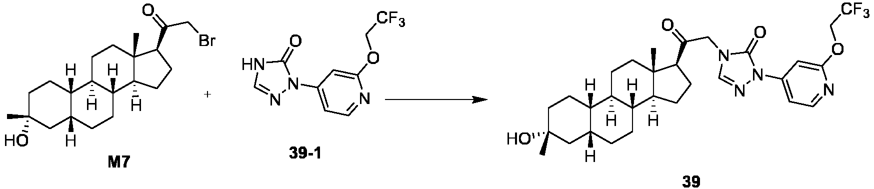

1. K₂CO₃ (483 mg, 3.49 mmol, 10.0 *eq*), **M7** (139 mg, 349 µmol, 1.00 *eq),* and **39-1** (prepared with reference to the preparation method for **35-3)** (100 mg, 384 µmol, 1.10 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 39** as a white solid (136 mg, 222 µmol, yield: 63.1%). MS (ESI) m/z = 577.3[M+H] ⁺, ¹H NMR (400 MHz, DMSO-*d*₆) *δ* = 8.31 - 8.21 (m, 2H), 7.64 (dd, *J =* 1.6, 5.6 Hz, 1H), 7.42 (d, *J* = 1.6 Hz, 1H), 5.03 (q, *J* = 9.2 Hz, 2H), 4.80 - 4.53 (m, 2H), 2.77 (br t, *J* = 8.8 Hz, 1H), 2.16 - 2.01 (m, 2H), 1.78 - 1.56 (m, 8H), 1.47 - 1.22 (m, 10H), 1.13 - 1.00 (m, 7H), 0.59 (s, 3H).

### Example 40

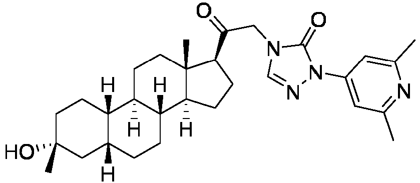

### Synthetic route:

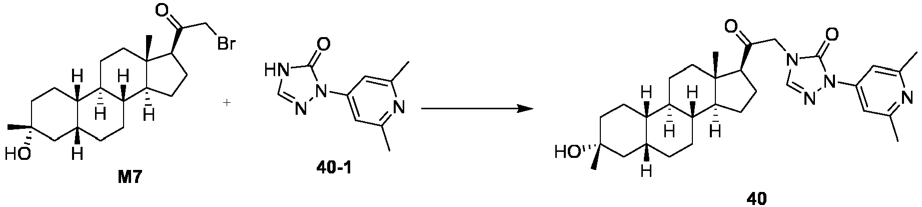

1. K₂CO₃ (509 mg, 3.68 mmol, 10.0 *eq*), **M7** (146 mg, 368 µmol, 1.00 *eq*), and **40-1** (prepared with reference to the preparation method for **36-4)** (70.0 mg, 368 µmol, 1.00 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 40** as a pale yellow solid (65.4 mg, 129.1 µmol, yield: 35.1%). MS (ESI) m/z = 507.4[M+H] ⁺, ¹H NMR (400 MHz, DMSO-*d*₆) *δ* = 8.44 (s, 1H), 8.09 (s, 2H), 4.83 - 4.53 (m, 2H), 2.79 (br t, *J =* 8.8 Hz, 1H), 2.70 (s, 6H), 2.06 (br d, *J =* 9.6 Hz, 2H), 1.81 - 0.94 (m, 25H), 0.58 (s, 3H).

### Example 41

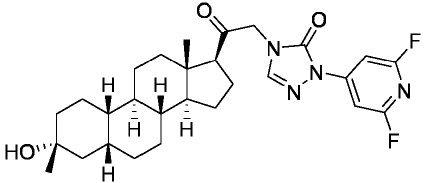

### Synthetic route:

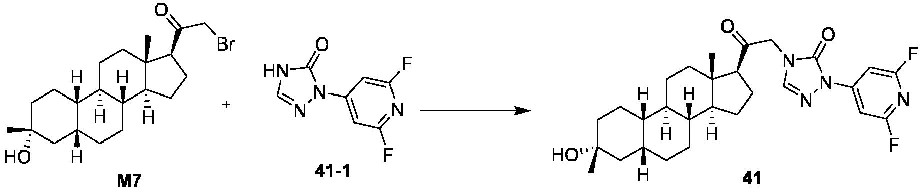

1. K₂CO₃ (634 mg, 4.59 mmol, 10.0 *eq*), **M7** (182 mg, 459 µmol, 1.00 *eq*), and **41-1** (prepared with reference to the preparation method for **36-4)** (100 mg, 505 µmol, 1.10 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 41** as a pale yellow solid (153 mg, 185.2 µmol, yield: 62.3%). MS (ESI) m/z = 497.2[M+H] ⁺, ¹H NMR (400 MHz, DMSO-*d*₆) *δ* = 8.35 (s, 1H), 7.55 (s, 2H), 4.84 - 4.49 (m, 2H), 4.25 (s, 1H), 4.03 (q, *J* = 7.2 Hz, 1H), 2.77 (br t, *J =* 8.8 Hz, 1H), 2.06 (br d, *J =* 9.6 Hz, 2H), 1.78 - 0.98 (m, 23H), 0.59 (s, 3H).

### Example 42

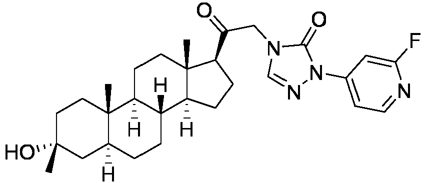

### Synthetic route:

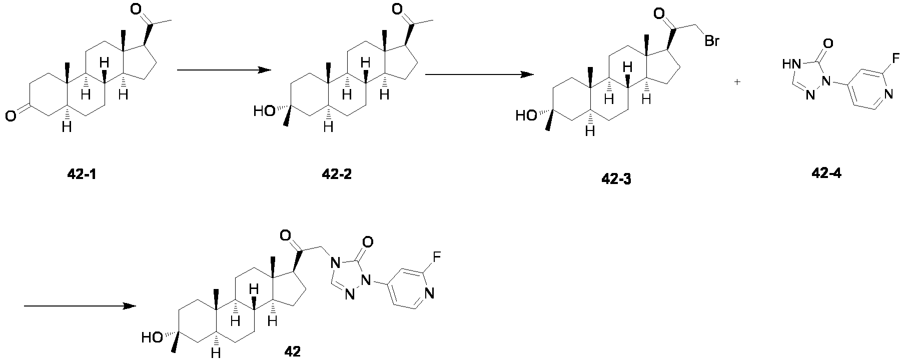

1. Under nitrogen protection, anhydrous tetrahydrofuran (15 mL) was added to a 100 mL three-necked flask (R1), compound **42-1** (1 g, 3.16 mmol, 1.00 *eq*) was added to R1 at 25 °C, and MeMgBr (3 M, 1.05 mL, 1.00 *eq*) was added to R1 at 0 °C. R1 was stirred at 0 °C for 0.5 h. Saturated ammonium chloride (5 mL) was added to the mixture, and the mixture was filtered. The filter cake was washed with ethyl acetate (5 mL × 2). The organic layer was separated from the filtrate and washed with brine (10 mL). The organic phase was dried over anhydrous sodium sulfate and concentrated to give a crude product. The crude product was purified by high performance liquid chromatography to give white compound **42-2** (165 mg, 496.21 µmol, yield: 15.70%).
2. Compound **42-2** (165 mg, 496 µmol, 1.00 *eq*) was added to a 40 mL sealed tube (R1) filled with nitrogen gas. MeOH (1.50 mL) was added to R1 at 25 °C. HBr (16.23 mg, 96.27 µmol, 10.89 µL, 0.194 *eq*) was added to R1 at 0 °C. Br₂ (79.30 mg, 496.21 µmol, 25.58 µL, 1.00 *eq*) was added to R1 at 0 °C. R1 was stirred at 25 °C for 5 h, and the mixture was quenched with saturated sodium bicarbonate (5 mL) and extracted with ethyl acetate (5 mL × 2). The combined organic layers were washed with brine (5 mL), dried over Na₂SO₄, filtered, and concentrated to give a crude product. The crude product was purified by column chromatography on silica gel (petroleum ether/ethyl acetate = 100/1-1/1) to give compound **42-3** as a white solid (140 mg, 340.29 µmol, yield: 68.58%). ¹H NMR (400 MHz, CDCl₃)δ = 4.05 - 3.74 (m, 2H), 2.82 (t, *J =* 8.8 Hz, 1H), 2.31 - 2.06 (m, 1H), 1.81 - 1.62 (m, 4H), 1.61 - 1.48 (m, 5H), 1.47 - 1.40 (m, 2H), 1.39 - 1.28 (m, 4H), 1.27 - 1.11 (m, 9H), 0.96 (br dd, *J* = 5.2, 12.0 Hz, 1H), 0.82 (dd, *J =* 3.6, 11.6 Hz, 1H), 0.76 (s, 3H), 0.64 (s, 3H).
3. K₂CO₃ (470.30 mg, 3.40 mmol, 10.0 *eq*), **42-3** (140 mg, 340.29 µmol, 1.00 *eq*), and **42-4** (61.30 mg, 340.29 µmol, 1.00 *eq*) were added to THF (4.0 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure, and then the crude product was separated and purified by HPLC to give **compound 42** as a pale yellow solid (110 mg, 211.54 µmol, yield: 62.16%). MS (ESI) m/z = 511[M+H] ⁺, ¹H NMR (400 MHz, CDCl₃)δ = 8.23 (d, *J =* 5.8 Hz, 1H), 7.88 (d, *J=* 5.6 Hz, 1H), 7.71 - 7.53 (m, 2H), 4.69 - 4.33 (m, 2H), 2.63 (t, *J* = 8.8 Hz, 1H), 2.33 - 2.15 (m, 1H), 2.11 (br d, *J =* 11.4 Hz, 1H), 1.81 - 1.63 (m, 4H), 1.55 - 1.39 (m, 6H), 1.37 - 1.13 (m, 12H), 1.05 - 0.95 (m, 1H), 0.83 (br d, *J =* 2.1 Hz, 1H), 0.77 (s, 3H), 0.68 (s, 3H).

### Example 43

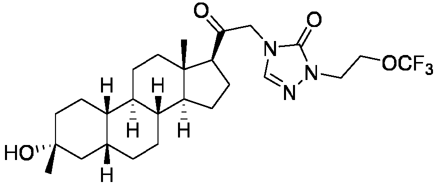

### Synthetic route:

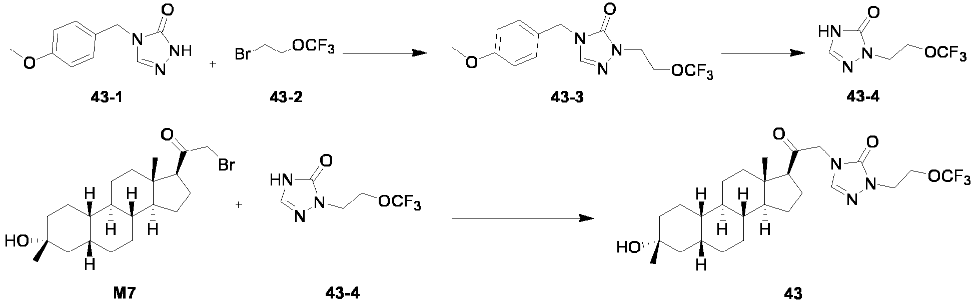

1. Compound **43-1** (255 mg, 1.24 mmol, 1.20 *eq*) was added to a 40.0 mL AK flask (R1). Dimethyl sulfoxide (5.00 mL) was added to R1 at room temperature. Potassium carbonate (286 mg, 2.07 mmol, 2.00 *eq)* was added to R1 at room temperature. Compound **43-2** (200 mg, 1.04 mmol, 1.00 *eq*) was added to R1 at room temperature. The mixture was stirred at 80 °C for 2 h, and TLC analysis (dichloromethane/methanol = 10/1) showed that compound **43-1** was completely consumed and a new spot appeared. After the reaction mixture was filtered, the filtrate was concentrated to give a white solid crude product. The crude product was separated using a conventional reversed-phase column to give compound **43-3** as a white solid (100 mg, 315 µmol, yield: 30.4%). ¹H NMR (400 MHz, DMSO-*d₆)* δ = 8.06 (s, 1H), 7.23 (d, J = 8.8 Hz, 2H), 6.91 (d, J = 8.8 Hz, 2H), 4.71 (s, 2H), 4.31 (t, J = 5.2 Hz, 2H), 3.99 (t, J = 5.2 Hz, 2H), 3.73 (s, 4H).
2. Compound **43-3** (50.0 mg, 157 µmol, 1.00 *eq)* was added to a 40.0 mL AK flask (R1). Acetonitrile (0.50 mL) was added to R1 at room temperature. Potassium carbonate (286 mg, 2.07 mmol, 2.00 eq) was added to R1 at room temperature. Ceric ammonium nitrate (259 mg, 473 µmol, 236 µL, 3.00 eq) was added to R1 at room temperature. R1 was stirred at room temperature for 4 h and stirred at 60 °C for 12 h. The reaction mixture was concentrated to give crude **43-4.** MS (ESI) m/z = 198.1 [M+H] ⁺. The crude product was directly used in the next step.
3. K₂CO₃ (701 mg, 5.07 mmol, 10.0 *eq*), **M7** (202 mg, 507 µmol, 1 *eq*), and **43-4** (100 mg, 507 µmol, 1.0 *eq*) were added to THF (4 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure to give compound **43** as a white solid (160 mg, 309 µmol, yield: 60.9%). MS (ESI) m/z = 514.4[M+H] ⁺, ¹H NMR (400 MHz, CDCl₃) *δ* = 7.46 (s, 1H), 4.55 - 4.33 (dd, 2H), 4.27-4.25 (d, 2H), 4.14-4.09 (m, 2H), 2.58-2.63 (m, 1H), 2.32 - 1.09 (m, 27H), 0.67 (s, 3H).

### Example 44

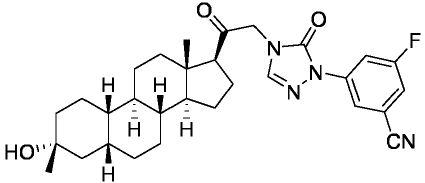

### Synthetic route:

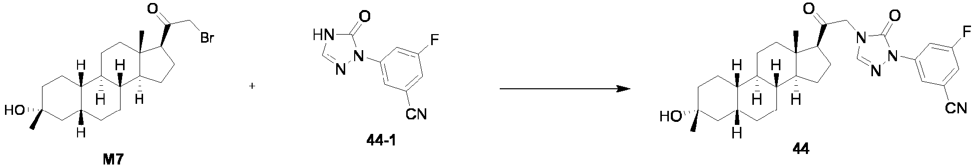

1. K₂CO₃ (615 mg, 4.45 mmol, 10.0 *eq*), **M7** (177 mg, 445 µmol, 1.0 *eq*), and **44-1** (100 mg, 489.81 µmol, 1.1 *eq*) **(44-1** was prepared with reference to the preparation method for **43-4)** were added to THF (4 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure to give a crude product, and the crude product was purified by silica gel column chromatography (dichloromethane:methanol = 0-10%) to give a yellow solid. The yellow solid was transferred to a 50.0 mL single-neck flask (R2), and 10.0 mL of methyl t-butyl ether was added to R2. R2 was stirred at room temperature for 2 h. The mixture in R2 was filtered, and the resulting solid was concentrated to give compound **44** as a yellow solid (144 mg, 270 µmol, yield: 60.7%). MS (ESI) m/z = 503.2[M-OH+H] ⁺, ¹H NMR (400 MHz, DMSO-*d₆*) *δ* = 8.28 (s, 1H), 8.16 (s, 1H), 8.05 (td, *J* = 2.4, 10.5 Hz, 1H), 7.80 - 7.74 (m, 1H), 4.83 - 4.47 (m, 2H), 4.25 (br s, 1H), 2.77 (br t, *J =* 8.8 Hz, 1H), 2.12 - 2.01 (m, 2H), 1.78 - 1.01 (m, 24H), 0.59 (s, 3H).

### Example 45

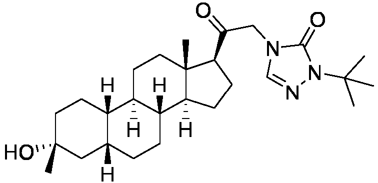

### Synthetic route:

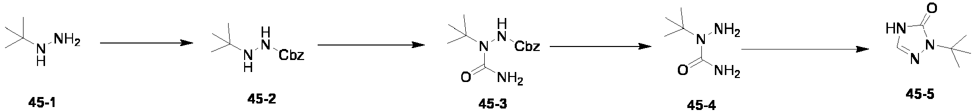

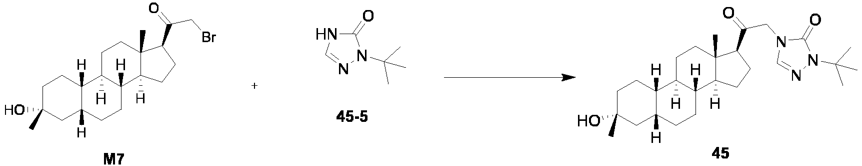

1. Compound **45-1** (2.00 g, 16.1 mmol, 1.00 *eq*, HCl) was added to a 500 mL single-neck flask (R1). Toluene (25.0 mL) was added to R1 at room temperature, and NaOH (6.42 g, 16.1 mmol, 1.00 *eq*) was added to R1 at room temperature. R1 was stirred at room temperature for 15 min. Benzyl chloroformate (2.63 g, 15.4 mmol, 2.20 mL, 0.96 *eq*) was dissolved in toluene (10.0 mL), and the solution was added to R1 at -15 °C. NaOH (6.16 g, 15.4 mmol, 10.0% purity, 0.96 eq) was added to R1 at -15 °C. R1 was stirred at room temperature for 2 h. Chloroform (30.0 mL) was added to R1 at room temperature. The organic phase was separated from the aqueous phase, and the aqueous phase was further extracted three times with chloroform (30.0 mL). The organic phase was washed with saturated brine (100 mL), dried over a proper amount of anhydrous sodium sulfate, and finally filtered and concentrated to give a crude product. The crude product (petroleum ether:ethyl acetate = 1:1, Rf = 0.49) was purified by silica gel column chromatography (petroleum ether:ethyl acetate = 0-20%) to give compound **45-2** as a colorless oil (1.46 g, 6.57 mmol, yield: 40.9%). MS (ESI) m/z = 223.1 [M+H] ⁺, ¹H NMR(400 MHz, CHLOROFORM-d) *δ* = 7.27 (s, 5H), 5.06 (s, 2H), 0.99 (s, 9H).
2. Compound **45-2** (960 mg, 4.32 mmol, 1.00 *eq*) was added to a 100 mL single-neck flask (R1). Isopropanol (20.0 mL) was added to R1 at room temperature, and trimethylsilyl isocyanate (498 mg, 4.32 mmol, 574 µL, 1.00 *eq*) was added to R1 at room temperature. R1 was stirred at room temperature for 6 h, and trimethylsilyl isocyanate (249 mg, 2.16 mmol, 287 µL, 0.50 *eq*) was added to R1 at room temperature. R1 was stirred at room temperature for 18 h, and the reaction mixture was directly concentrated to give a crude product. The crude product (dichloromethane:methanol = 10:1) was purified by silica gel column chromatography (methanol:dichloromethane = 0-10%) to give compound **45-3** as a white solid (550 mg, 2.07 mmol, yield: 48.0%). MS (ESI) m/z = 265.9 [M+H] ⁺, ¹H NMR (400 MHz, DMSO-*d*₆) *δ* = 9.27 (s, 1H), 7.47 - 7.20 (m, 5H), 5.94 - 5.70 (m, 2H), 5.30 - 4.81 (m, 2H), 1.32 - 1.22 (m, 9H).
3. Compound **45-3** (300 mg, 1.13 mmol, 1.00 *eq)* was added to a 50.0 mL single-neck flask (R1) protected with a hydrogen balloon (15.0 Psi). Ethanol (15.0 mL) was added to R1 at room temperature, and Pd/C (12.0 mg, 11.3 µmol, 10.0% purity, 0.01 *eq)* was added to R1 at room temperature. R1 was stirred at room temperature for 12 h. The reaction mixture was filtered, and the filtrate was concentrated to give compound **45-4** as a white solid (145 mg, 1.11 mmol, yield: 97.8%). ¹H NMR (400 MHz, DMSO-*d*₆) *δ* = 6.19 - 5.62 (m, 2H), 4.35 (s, 2H), 1.31 (s, 9H).
4. Compound **45-4** (95.0 mg, 724 µmol, 1.00 *eq)* was added to a 40.0 mL AK flask (R1). Ethanol (5.00 mL) was added to R1 at room temperature. Trimethyl orthoformate (192 mg, 1.81 mmol, 198 µL, 2.50 *eq)* was added to R1 at room temperature. p-Toluenesulfonic acid (12.5 mg, 72.4 µmol, 0.10 *eq)* was added to R1 at room temperature. R1 was stirred at 60 °C for 12 h, and the reaction mixture was concentrated to give a crude product. The crude product (petroleum ether:ethyl acetate = 1:1) was purified by silica gel column chromatography (petroleum ether:ethyl acetate = 0-100%) to give compound **45-5** as a white solid (110 mg, crude). MS (ESI) m/z = 142.3 [M+H] ⁺, ¹H NMR (400 MHz, DMSO-*d*₆) *δ* = 11.31 (br s, 1H), 7.69 (s, 1H), 1.44 (s, 9H).
5. K₂CO₃ (890 mg, 6.44 mmol, 10.0 *eq*), **M7** (256 mg, 644 µmol, 1.0 *eq),* and **45-5** (100 mg, 708 µmol, 1.1 *eq*) were added to THF (4 mL). Then the system was stirred at 50 °C for 12 h, quenched with water (5 mL), and extracted with ethyl acetate (10.0 mL × 2). The organic phases were combined and then washed with saturated brine (10 mL), dried over anhydrous sodium sulfate, and filtered. The organic phase was concentrated under reduced pressure to give a crude product, and the crude product was purified by silica gel column chromatography (methanol:dichloromethane = 0-10%) to give compound **45** as a white solid (210 mg, 456 µmol, yield: 70.8%). MS (ESI) m/z = 458.3[M +H] ⁺, ¹H NMR(400 MHz, DMSO-*d₆)δ* = 7.74 (s, 1H), 4.54 - 4.38 (m, 2H), 4.24 (s, 1H), 2.73 - 2.64 (m, 1H), 2.09 - 2.00 (m, 2H), 1.75 - 1.59 (m, 7H), 1.45 (s, 9H), 1.39 - 1.23 (m, 9H), 1.10 (s, 8H), 0.56 (s, 3H).

### Bioactivity Assays

### Test Example 1. In Vitro Evaluation of Intrasynaptic and Extrasynaptic Functional Activities in Cells

### 1.1. Experimental materials

| Material name | Manufacturer | Cat. No. |
|---|---|---|
| α4β3δ-HEK-FlpIn-TRex | Beijing Institute of Materia Medica | / |
| α1β2γ2-CHO-TRex | Beijing Institute of Materia Medica | / |
| GABA | Sigma | A2129 |
| DMEM culture medium | Invitrogen | C11995500BT |
| Fetal bovine serum | Shanghai Yeasen | 40130ES76 |
| Blasticidin | Shanghai Yeasen | 60218ES10 |
| Zeocin | Invitrogen | R25001 |
| Hygromycin | Shanghai Yeasen | 60225ES10 |
| Puromycin | Solarbio | P8230 |
| Tetracycline | Sigma | 87128 |
| Polylysine | Sigma | P1149 |
| Trypsin | Gibco | 27250018 |

### 1.2. Experimental method

The α4β3δ-HEK-FlpIn-TRex stable cell strain was cultured in DMEM + 10% FBS complete culture medium, with the selective antibiotics being 10 µg/mL Blasticidin, 100 µg/mL Zeocin, 100 µg/mL Hygromycin, and 0.2 µg/mL Puromycin. The α1β2γ2-CHO-TRex stable cell strain was cultured in DMEM/F12 + 10% FBS complete culture medium, with the selective antibiotics being 10 µg/mL Blasticidin, 100 µg/mL Zeocin, 300 µg/mL Hygromycin, and 1 µg/mL Puromycin.

The day before the patch clamp experiment, cells were seeded onto 12 mm polylysine-coated coverslips and then cultured in 35 mm culture dishes, and tetracycline (1 µg/mL) was added to induce the expression of GABA receptors. The extracellular fluid had the following composition (in mM): 140 NaCl, 3 KCl, 1.5 MgCl₂, 2 CaCl₂, 10 HEPES, and 10 Glucose. After thorough mixing, NaOH was added to adjust the pH to 7.4, and sucrose was added to adjust the osmotic pressure to 300-320 mOsm. The fluid was stored at 4 °C. The electrode internal solution had the following composition (in mM): 145 KCl, 1 MgCl₂, 5 EGTA, 10 HEPES, and 5 MgATP. After thorough mixing, KOH was added to adjust the pH to 7.3, and sucrose was added to adjust the osmotic pressure to 290-300 mOsm. The solution was filtered through a 0.22 µM filter before use.

Whole-cell patch clamp recording was mainly employed in this experiment. The membrane potential was clamped at -60 mV, and receptors were activated using GABA at an EC10-20 concentration (~1 µM). The test compounds were dissolved in 0.1% DMSO, and perfusion was performed for 30 s prior to GABA stimulation. Finally, the positive allosteric regulating effects of the test compounds on α1β2γ2 or α4β3δ were determined.

The experimental result for each concentration was expressed as a fold increase in the GABA-induced current caused by the compound. Initially, the minimum concentration at which the compound is just able to enhance the GABA-induced current was determined, and then the test concentration was gradually increased in 3-fold increments until the activating effect of the compound reached the EC₈₀ effect or higher. At least 5 concentrations are tested to obtain a complete EC₅₀ curve and EC₅₀ values. All experiments were repeated on no less than 3 cells, and data processing was completed using GraphPad prism software. The dose-response curves were fitted using the Hill equation, and the half-maximal activating concentration EC₅₀ and Eₘₐₓ were finally obtained. The results are shown in Table 1.

**Table 1. Results for the functional activity of the compounds on intrasynaptic and extrasynaptic receptors**

| **Name of the compounds** | **Intrasynaptic functional activity (α1β2γ2) (EC₅₀, nM/Eₘₐₓ, %)** | **Extrasynaptic functional activity (α4β3δ) (EC₅₀, nM/Eₘₐₓ, %)** |
|---|---|---|
| Compound **1** | 56/565.8 | 10/440 |
| Compound **2** | 373/773 | 61/391 |
| Compound **3** | 90/430 | 29/490 |
| Compound **4** | 220/390 | 170/410 |
| Compound **5** | 26/410 | 21/570 |
| Compound **6** | 444/242 | 305/497 |
| Compound **8** | 371/1265 | 112/334 |
| Compound **9** | 12.4/294 | 148/428 |
| Compound **11** | 241/594 | 139/552 |
| Compound **13** | 31/305 | 55/470 |
| Compound **17** | 167/389 | 134/474 |
| Compound **18** | 171/710 | 107/541 |
| Compound **19** | 153/764 | 159/386 |
| Compound **20** | 368/724 | 360/966 |
| Compound **21** | 153/1066 | 116/1246 |
| Compound **22** | 349/786 | 213/1069 |
| Compound **23** | 145/618 | 150/601 |
| Compound **24** | 340/347 | 79/743 |
| Compound **25** | 784/692 | 89/542 |
| Compound **26** | 109/624 | 72/589 |
| Compound **27** | 126/445 | 42/526 |
| Compound **28** | 154/1044 | 35/441 |
| Compound **29** | 192/691 | 203/830 |
| Compound **30** | 585/873 | 237/685 |
| Compound **31** | 227/735 | 86/609 |
| Compound **32** | 330/763 | 126/633 |
| Compound **33** | 598/495 | 96/936 |
| Compound **34** | 112/1048 | 50/683 |
| Compound **35** | 340/446 | 68/720 |
| Compound **36** | 119/871 | 99/745 |
| Compound **37** | 83/600 | 49/432 |
| Compound **38** | 55/700 | 115/625 |
| Compound **39** | 50/675 | 34/967 |
| Compound **40** | 171/691 | 41/563 |
| Compound **41** | 145/762 | 67/754 |
| Compound **42** | 280/736 | 143/507 |
| Compound **43** | 770/576 | 460/889 |
| Compound **44** | 72/518 | 37/546 |
| Compound **45** | 121/806 | 113/749 |

The results show that: compounds **1-45** all have relatively good intrasynaptic and extrasynaptic functional activities.

### Test Example 2. In Vitro Liver Microsomal Stability Assay

### 2.1. Solution preparation

1) Preparation of working solutions of test samples: The test samples were diluted with acetonitrile to 100 µM.
2) Preparation of a working solution of liver microsomes: liver microsomes were diluted with 100 mM phosphate-buffered saline to 0.56 mg/mL.
3) Preparation of a working solution of reduced nicotinamide adenine dinucleotide (NADPH): A proper amount of NADPH was weighed out and diluted with phosphate-buffered saline to 20 mM, and an equal volume of a 60 mM MgCl₂ solution was added.
4) Preparation of a stop solution: Tolbutamide was diluted with acetonitrile to 20 ng/mL, serving as a stop solution containing an internal standard.

### 2.2. Incubation process

1) Incubation anti-adsorption EP tubes were prepared and labeled with species, test/control sample, time points (0, 5, 10, 20, 30, 60 min, Blank60, and NCF60), etc.
2) 2 µL of test or control sample working solution, diluted with acetonitrile to 100 µM, and 178 µL of liver microsome working solution were added to each tube. For the Blank60 tube, 2 µL of acetonitrile was added instead of a test sample. The tubes were pre-incubated in a 37 °C water bath for about 10 min, with each sample tested in triplicate.
3) After the pre-incubation was complete, 20 µL of NADPH working solution was added to each tube except for the 0 min and NCF60 tubes to start reactions, and 20 µL of phosphate-buffered saline (containing 30 mM MgCl₂) was added to the NCF60 tube. In the incubation system, the final concentrations of the test and control samples were 1 µM, the final concentration of liver microsomes was 0.5 mg/mL, the final concentration of NADPH was 1 mM, and the final concentration of MgCl₂ was 3 mM.
4) For the 0 min sample, 600 µL of stop solution was added before adding the NADPH working solution. After each sample was incubated for its respective time, 600 µL of stop solution was added to stop the reaction.
5) After the reaction was stopped, each sample was vortexed for 30 s and then centrifuged at 13,500 rpm for 10 min. 100 µL of the supernatant was transferred to an EP tube, and 100 µL Milli-Q water was added. The mixture was well mixed by vortexing and analyzed by LC-MS/MS.
6) Testosterone and dextromethorphan were used as positive control samples under the same conditions to assess the stability and reliability of the system.

### 2.3. Data analysis

Parameters of the test samples, including their half-lives, intrinsic clearance rates in liver microsomes, and hepatic intrinsic clearance rates, were calculated based on the percentage of test sample remaining. The results are shown in Table 2.

**Table 2. The half-lives, intrinsic clearance rates in liver microsomes, and hepatic intrinsic clearance rates of the compounds**

| **Compound No.** | **Species** | **Remaining amount% at 60 min** | **T₁₂(min)** | **CLint(mic) µL/min/mg protein** | **Clint(liver) mL/min/kg** |
|---|---|---|---|---|---|
| Compound **1** | Rat | 44.5 | 57.3 | 24.2 | 43.6 |
| | Human | 89.0 | 330 | 4.20 | 3.97 |
| Compound **2** | Rat | 11.25 | 11.3 | 104 | 187 |
| | Human | 18.57 | 24.1 | 57 | 52 |
| Compound **3** | Rat | 51.77 | 26.5 | 52 | 94 |
| | Human | 70.25 | 117.5 | 12 | 11 |
| Compound **4** | Rat | 37.09 | 27.0 | 51 | 93 |
| | Human | 64.0 | 96.3 | 14 | 13 |
| Compound **5** | Rat | 14.19 | 14.1 | 98 | 176 |
| | Human | 43.53 | 52.5 | 26 | 24 |
| Compound **6** | Rat | 15.45 | 21.3 | 65 | 117 |
| | Human | 72.92 | 126 | 11 | 10 |
| Compound **8** | Rat | 5.61 | 2.9 | 486 | 875 |
| | Human | 53.59 | 69.3 | 20 | 28 |
| Compound **9** | Rat | 4.19 | 10.3 | 133 | 239 |
| | Human | 24.73 | 32.8 | 42 | 38 |
| Compound **11** | Rat | 9.24 | 9.0 | 153 | 276 |
| | Human | 28.02 | 19.8 | 70 | 63 |
| Compound **17** | Rat | 6.6 | 15.5 | 90 | 161 |
| | Human | 57.7 | 72.9 | 19 | 17 |
| Compound **18** | Rat | 9.7 | 9.0 | 154 | 278 |
| | Human | 38.3 | 41.7 | 33 | 30 |
| Compound **19** | Rat | 9.5 | 17.6 | 79 | 141 |
| | Human | 42.2 | 28.5 | 29 | 26 |
| Compound **20** | Rat | 8.40 | 7.80 | 178 | 320 |
| | Human | 52.3 | 70.7 | 20 | 28 |
| Compound **21** | Rat | 31.9 | 36.3 | 38 | 69 |
| | Human | 47.9 | 53.3 | 26 | 23 |
| Compound **22** | Rat | 8.9 | 17.1 | 81 | 146 |
| | Human | 8.6 | 7.8 | 178 | 320 |
| Compound **23** | Rat | 31.7 | 34.7 | 40 | 72 |
| | Human | 53.4 | 60.3 | 23 | 21 |
| Compound **24** | Rat | 14.7 | 21.1 | 66 | 118 |
| | Human | 56.1 | 71.4 | 19 | 17 |
| Compound **25** | Rat | 16.2 | 24.1 | 58 | 104 |
| | Human | 42 | 49.5 | 28 | 25 |
| Compound **26** | Rat | 12.7 | 20.8 | 67 | 120 |
| | Human | 53.7 | 68.6 | 20 | 18 |
| Compound **27** | Rat | 14.60 | 20.4 | 68 | 122 |
| | Human | 64.13 | 91.2 | 15 | 14 |
| Compound **28** | Rat | 48.8 | 57.8 | 24 | 43 |
| | Human | 61.6 | 58.2 | 24 | 21 |
| Compound **29** | Rat | 36.8 | 38.7 | 36 | 64 |
| | Human | 45.8 | 20.7 | 67 | 60 |
| Compound **30** | Rat | 7.4 | 7.6 | 183 | 329 |
| | Human | 9.5 | 17.5 | 79 | 91 |
| Compound **31** | Rat | 2.1 | 10.7 | 130 | 234 |
| | Human | 28.5 | 34.1 | 41 | 37 |
| Compound **32** | Rat | 7.7 | 15.4 | 85 | 152 |
| | Human | 66.8 | 64.8 | 21 | 19 |
| Compound **33** | Rat | 62.2 | 85.6 | 16 | 29 |
| | Human | 85.8 | >186 | <7.5 | <6.7 |
| Compound **34** | Rat | 20.5 | 25.6 | 54 | 98 |
| | Human | 79.9 | 177.7 | 8 | 7 |
| Compound **36** | Rat | 12.2 | 29 | 69 | 125 |
| | Human | 15.9 | 23 | 59 | 53 |
| Compound **38** | Rat | 82.9 | 210 | 7 | 12 |
| | Human | 97.3 | >186 | <7.50 | <6.75 |
| Compound **39** | Rat | 90.6 | >186 | <7.50 | <13.5 |
| | Human | 76.2 | 154 | 9 | 8 |
| Compound **41** | Rat | 68.3 | 108.3 | 13 | 23 |
| | Human | 98.2 | >186 | <7.50 | <6.75 |
| Compound **43** | Rat | 15.74 | 23 | 60 | 108 |
| | Human | 25.97 | 30.7 | 45 | 41 |
| Compound **44** | Rat | 64.82 | 93.6 | 15 | 27 |
| | Human | 66.67 | 101.9 | 14 | 12 |
| Compound **45** | Rat | 7.24 | 8.9 | 156 | 281 |
| | Human | 48.81 | 56.3 | 25 | 22 |

As shown in the Table above, the compounds provided by the present disclosure have, for the most part, relatively good stability in liver microsomes of rats and humans, indicating that they have good metabolic stability.

### Test Example 3. Loss of Righting Reflex in SD Rats Injected with Compound 41

### 3.1. Objective

To determine the threshold for the loss of righting reflex in SD rats after injection of compound 41 in order to evaluate the sedative effect and therapeutic safety window of the compound.

### 3.2. Test process

Male SD rats weighing 180-200 g, as test animals, were acclimatized for 7 days after arriving at the animal facility and were randomly divided into groups based on weight one day before testing. The rats were fasted for half a day before testing but had access to water. Doses of compound 41 were designed in a 0.8-fold gradient, and the doses were set at 23.5 mg/kg, 18.8 mg/kg, 15 mg/kg, 12 mg/kg, and 9.6 mg/kg. Observations were made on the loss of righting reflex within 4 h of intraperitoneally injecting compound 41 into the rats. The criterion for determining if righting reflex was lost was that: after compound 41 was injected, if a rat in a supine position did not right itself within 30 s, it was considered a positive loss of righting reflex; otherwise, it was considered a negative loss of righting reflex.

### 3.3. Test results

The test results show that at doses of 23.5 mg/kg, 18.8 mg/kg, 15 mg/kg, 12 mg/kg, and 9.6 mg/kg, the loss of righting reflex occurred in rats at rates of 80%, 50%, 30%, 0, and 0, respectively. Under the test conditions, the threshold dose of compound 41, when intraperitoneally injected, for the loss of righting reflex in SD rats was 12 mg/kg, indicating that compound 41 has a relatively wide therapeutic safety window.

### Test Example 4. Loss of Righting Reflex in SD Rats Intragastrically Given Compound 41

### 4.1. Objective

To determine the threshold for the loss of righting reflex in SD rats after intragastric administration of compound 41 in order to evaluate the sedative effect and therapeutic safety window of compound 41.

### 4.2. Test method

Male SD rats weighing 180-200 g, as experimental animals, were acclimatized for 7 days after arriving at the animal facility and were randomly divided into groups based on weight one day before experimentation. The rats were fasted overnight before experimentation but had access to water. Doses of compound 41 were designed in a 0.8-fold gradient, and the doses were set at 39 mg/kg, 31.25 mg/kg, 25 mg/kg, 20 mg/kg, and 16 mg/kg, with 10 rats in each group. Observations were made on the loss of righting reflex within 4 h of intragastrically administering the compound to the rats. The criterion for determining if righting reflex was lost was that: after the test compound was administered, if a rat in a supine position did not right itself within 30 s, it was considered a positive loss of righting reflex; otherwise, it was considered a negative loss of righting reflex.

### 4.3. Test results

The results show that at doses of 39 mg/kg, 31.25 mg/kg, 25 mg/kg, 20 mg/kg, and 16 mg/kg, the loss of righting reflex occurred in rats at rates of 100%, 90%, 50%, 0%, and 0%, respectively. Under the experimental conditions, the threshold dose of compound 41, when intragastrically administered, for the loss of righting reflex in SD rats was 20 mg/kg. The threshold of intragastrically administering compound 41 for the loss of righting reflex in rats (20 mg/kg) is 5.7 times the effective dose for the antiepileptic convulsion effect (3.5 mg/kg), indicating a relatively wide therapeutic safety window.

### Test Example 5. Pharmacokinetic Study of Compound 41 in CD-1 Mice

### 4.1. Test animals and vehicle

Male CD-1 mice weighing about 25 g were randomly divided into groups of 3.

DMSO: 30% SBECD (10%: 90%)

### 4.2. Test method

The intravenous (IV) dose was 1 mg/kg, and the intragastric (IG) dose was 5 mg/kg. Animals were fasted for 12 h before administration, with free access to water, and were fed together 3 h post-administration. Blood samples of about 0.15 mL were collected at 5 min (intravenous),15 min, 30 min, 1 h, 2 h, 4 h, 8 h, 12 h, and 24 h post-administration into heparinized EP tubes and centrifuged at 13,500 rpm for 10 min to separate the plasma. Mouse brain tissue samples were collected after cardiac perfusion at 30 min, 2 h, and 8 h. After pretreatment, the samples were analyzed by LC-MS/MS to determine the concentration of the test compound in the plasma and brain, and pharmacokinetic parameters were calculated.

### 4.3. Test results

**Table 3. Pharmacokinetic parameters in CD-1 mice after intravenous (1 mg/kg) and intragastric (5 mg/kg) administration of compound 41**

| Route of administration | Tₘₐₓ | C₀/Cₘₐₓ | AUCₗₐₛₜ | AUC_{inf} | T_{1/21/2} | MRTₗₐₛₜ | F |
|---|---|---|---|---|---|---|---|
| | hour | ng/mL | h*ng/mL | h*ng/mL | hour | hour | % |
| IV | / | 490 | 3661 | 4316 | 9.0 | 7.9 | / |
| IG | 1 | 1673 | 20016 | 31341 | 16.5 | 9.8 | 109 |

**Table 4. The concentration in the brain of CD-1 mice after intragastric (5 mg/kg) administration of compound 41**

| | 30min | 2h | 8h |
|---|---|---|---|
| Brain (ng/g) | 847±36 | 1917±978 | 1589±233 |
| Plasma (ng/mL) | 936±119 | 1284±345 | 891±72 |
| Brain-to-blood ratio | 0.9 | 1.5 | 1.8 |

The data in Table 3 show that both intravenous administration and intragastric administration of compound 41 achieved relatively high plasma exposure (AUC) and relatively long half-lives in CD-1 mice, and the absolute bioavailability for intragastric administration reached 109%, indicating that compound 41 has good *in vivo* metabolic stability and exhibits high levels of *in vivo* absorption. The data in Table 4 show that compound 41 has a strong brain penetration capability, which results in high brain exposure and a relatively high concentration in the brain.

### Test Example 6. Pharmacokinetic Study of Compound 41 in SD Rats

### 5.1. Test animals and vehicle

Male SD rats weighing about 250 g were randomly divided into groups of 3.

DMSO: 30% SBECD (10%: 90%)

### 5.2. Test method

The intravenous dose was 6.1 µmol/kg, and the intragastric dose was 24.4 µmol/kg. Animals were fasted for 12 h before administration, with free access to water, and were fed together 3 h post-administration. Blood samples of about 0.3 mL were collected at 5 min (intravenous),15 min, 30 min, 1 h, 2 h, 3 h, 4 h, 6 h, 8 h, 12 h, and 24 h post-administration into heparinized EP tubes and centrifuged at 13,500 rpm for 10 min to separate the plasma. Rat brain tissue samples were collected after cardiac perfusion at 30 min, 2 h, and 6 h. After pretreatment, the samples were analyzed by LC-MS/MS to determine the concentration of the test compound in the plasma and brain, and pharmacokinetic parameters were calculated.

### 5.3. Test results

**Table 5. Pharmacokinetic parameters in SD rats after intravenous (6.1 µmol/kg) and intragastric (24.4 µmol/kg) administration of compound 41**

| Route of administration | Tₘₐₓ | C₀/Cₘₐₓ | AUCₗₐₛₜ | AUC_{inf} | T_{1/2} | MRTₗₐₛₜ | F |
|---|---|---|---|---|---|---|---|
| | hour | nmol/L | hour*nmol/L | hour*nmol/L | hour | hour | % |
| IV | / | 2112±537 | 9710±2259 | 10050±2377 | 5.17±0.55 | 6.3±0.83 | / |
| IG | 1.67±0.58 | 3277±324 | 32520±5197 | 35123±7031 | 6.11±1.44 | 7.61±0.6 | 84 |

**Table 6. The concentration in the brain of SD rats after intragastric (24.4 µmol/kg) administration of compound 41**

| | 30min | 2h | 6h |
|---|---|---|---|
| Brain (nmol/kg) | 2565±452 | 4533±720 | 2692±259 |
| Plasma (nmol/L) | 3203±1159 | 3813±670 | 1475±261 |
| Brain-to-blood ratio | 0.8 | 1.2 | 1.8 |

The data in Table 5 show that both intravenous administration and intragastric administration of compound 41 achieved relatively high plasma exposure (AUC) and relatively long half-lives in SD rats, and the absolute bioavailability for intragastric administration was 84%, indicating that compound 41 has good *in vivo* metabolic stability and exhibits high levels of *in vivo* absorption. The data in Table 5 show that compound 41 has a strong brain penetration capability, which results in high brain exposure and a relatively high concentration in the brain.

**Test Example 7. Evaluation of Anticonvulsant Effect of Compound 41 in SD Rats**

### 6.1. Objective

To establish an epileptic convulsion SD rat animal model by intraperitoneally injecting 50 mg/kg of pentylenetetrazol (PTZ) into SD rats, and evaluate the anticonvulsant effect of compound 41.

### 6.2. Test method

Male SD rats weighing 300+10 g, as test animals, were acclimatized for 7 days after arriving at the animal facility and were randomly divided into groups based on weight one day before administration. The rats were fasted for half a day before testing but had access to water. During the test, compound 41 was orally administered to the animals at 3.5 mg/kg in advance. Two hours after the oral administration, a PTZ solution was intraperitoneally injected at 50 mg/kg. Observations were made on the rats' epileptic convulsion behavior within 1 h of performing PTZ induction. The latency period of clonic convulsion and the latency period of tonic-clonic behavior were measured.

### 6.3. Test results

Behavioral raw data are finally expressed as mean ± standard error (Mean + S.E.M.) and statistically analyzed using One-way ANOVA Dunnett post hoc. *P* < 0.05 indicates the presence of a significant difference, *P* < 0.01 indicates the presence of a very significant difference, and *P* < 0.001 indicates the presence of an extremely significant difference.

The results from the observations of epileptic convulsion behavior show that within 1 h of intraperitoneally injecting PTZ at 50 mg/kg to induce epileptic convulsion behavior, the latency period of clonic convulsion behavior of the animals in the administration group (2764.6±1330.9 s) was significantly greater than the latency period of the solvent control group (114.5+36.9 s) (*P* < 0.001); the latency period of tonic-clonic behavior of the animals in the administration group (3600+0 s) was significantly greater than the latency period of the solvent control group (115.8437.1 s) (*P* < 0.001).

## Claims

1. A compound of formula (I) or a pharmaceutically acceptable salt thereof, 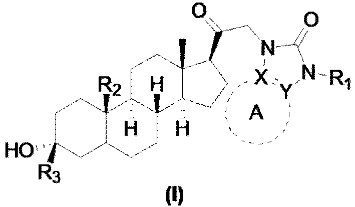 wherein,
(1) X is C, and Y is N; or X is N, and Y is C; or X and Y are simultaneously C;
R₁ is selected from the group consisting of H, C₁₋₆ alkyl, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl; or
(2) X and Y are simultaneously C, and X and Y, together with the atoms to which they are attached, form a ring A;
the ring A is 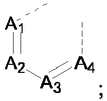
wherein A₁, A₂, A₃, and A₄ are each independently selected from the group consisting of CR₄ or N; R₁ is selected from the group consisting of H, C₁₋₆ haloalkyl, C₃₋₆ cycloalkyl, C₃₋₆ cycloalkyl C₁₋₃ alkyl, C₁₋₆ alkoxy C₁₋₃ alkyl, C₁₋₆ haloalkoxy C₁₋₃ alkyl, 6-10 membered aryl, or 5-10 membered heteroaryl, and the 6-10 membered aryl and 5-10 membered heteroaryl may be optionally substituted with one or more halogen, C₁₋₆ alkyl, C₁₋₆ haloalkyl, C₁₋₆ alkoxy, C₁₋₆ haloalkoxy, -(C=O)NH₂, -NH₂, or cyano groups;
R₂ is selected from the group consisting of H, halogen, C₁₋₆ alkyl, or C₁₋₆ alkoxy C₁₋₃ alkyl;
R₃ is selected from the group consisting of H, C₁₋₆ alkyl, or C₁₋₆ alkoxy C₁₋₃ alkyl;
each R₄ is independently selected from the group consisting of H, halogen, C₁₋₆ alkyl, C₁₋₆ alkoxy, C₁₋₆ haloalkoxy, C₁₋₆ alkoxy C₁₋₃ alkyl, or halogenated C₁₋₆ alkoxy C₁₋₃ alkyl.

2. The compound or the pharmaceutically acceptable salt thereof according to claim 1, wherein the compound or the pharmaceutically acceptable salt thereof has a structure of formula (IA) or formula (IB): 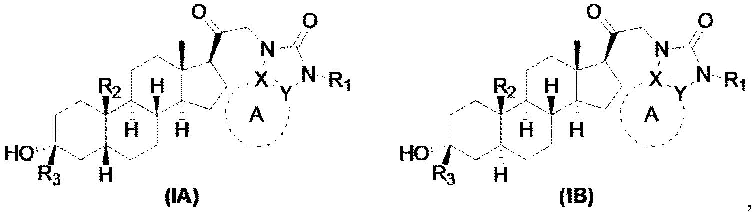 wherein X, Y, ring A, R₁, R₂, R₃, R₄, A₁, A₂, A₃, and A₄ are as defined in claim 1.

3. The compound or the pharmaceutically acceptable salt thereof according to claim 1, wherein the compound or the pharmaceutically acceptable salt thereof has a structure of formula (IIA), (IIB), or (IIC): 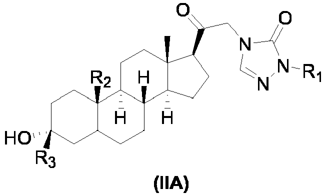 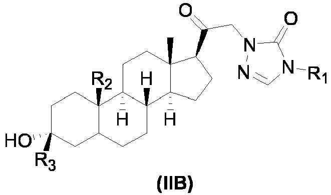 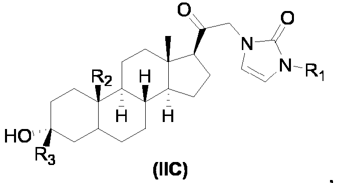 wherein R₁, R₂, and R₃ are as defined in claim 1.

4. The compound or the pharmaceutically acceptable salt thereof according to claim 1, wherein the compound or the pharmaceutically acceptable salt thereof has a structure of formula (IIA-1), (IIA-2), (IIB-1), (IIB-2), (IIC-1), or (IIC-2): 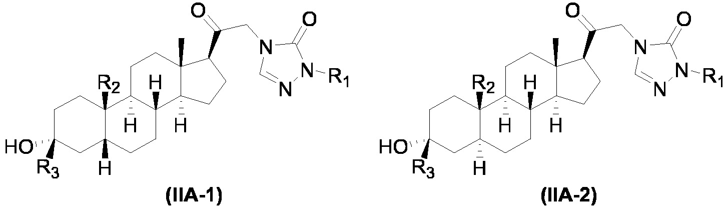 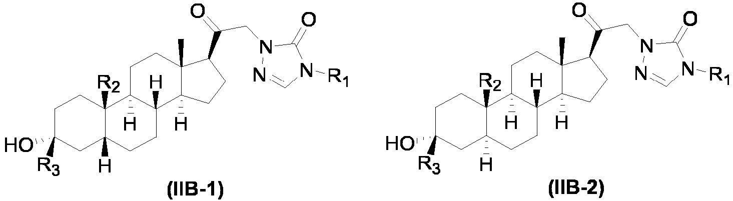 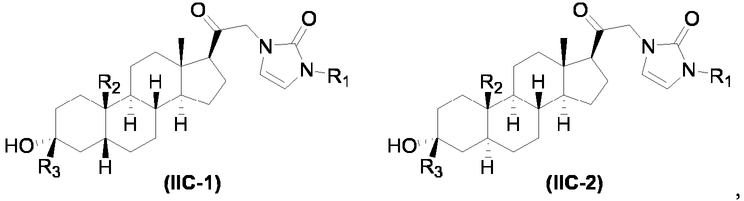 wherein R₁, R₂, and R₃ are as defined in claim 1.

5. The compound or the pharmaceutically acceptable salt thereof according to any one of claims 1-4, wherein R₁ is selected from the group consisting of H, methyl, ethyl, n-propyl, isopropyl, n-butyl, isobutyl, s-butyl, t-butyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, pentafluoroethyl, pentachloroethyl, cyclopentyl, cyclohexyl, cyclopropylmethyl, cyclopropylethyl, cyclobutylmethyl, cyclobutylethyl, cyclopentylmethyl, cyclopentylethyl, cyclohexylmethyl, cyclohexylethyl, methoxymethyl, ethoxymethyl, methoxyethyl, ethoxyethyl, trifluoromethoxymethyl, trifluoromethoxyethyl, phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl; the phenyl, naphthyl, pyrrolyl, furanyl, thienyl, imidazolyl, pyrazolyl, oxazolyl, isoxazolyl, thiazolyl, isothiazolyl, pyridinyl, pyridazinyl, pyrimidinyl, pyrazinyl, indolyl, benzopyrazolyl, isoindolyl, indazolyl, benzotriazolyl, benzothienyl, isobenzothienyl, benzofuranyl, benzoisofuranyl, benzodioxolyl, or benzimidazolyl is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups;
R₂ is selected from the group consisting of H, F, Cl, Br, I, methyl, ethyl, n-propyl, isopropyl, methoxymethyl, or ethoxymethyl;
R₃ is selected from the group consisting of H, methyl, ethyl, n-propyl, isopropyl, methoxymethyl, or ethoxymethyl.

6. The compound or the pharmaceutically acceptable salt thereof according to any one of claims 1-5, wherein R₁ is selected from the group consisting of H, methyl, ethyl, t-butyl, cyclopropylmethyl, 2,2,2-trifluoroethyl, cyclopentyl, cyclohexyl, methoxymethyl, ethoxymethyl, methoxyethyl, ethoxyethyl, trifluoromethoxymethyl, trifluoromethoxyethyl, 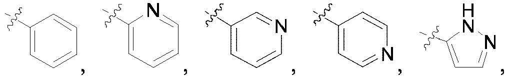 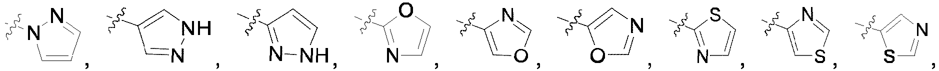 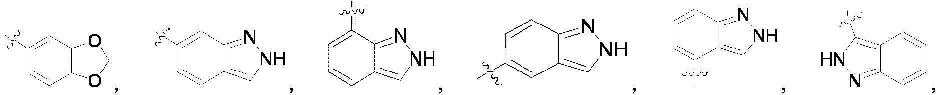 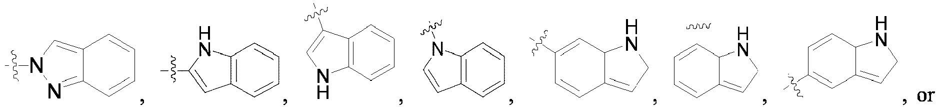 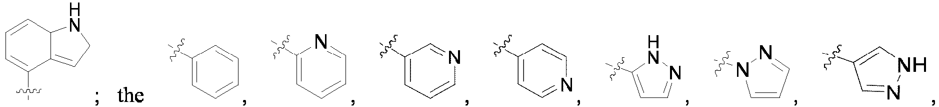 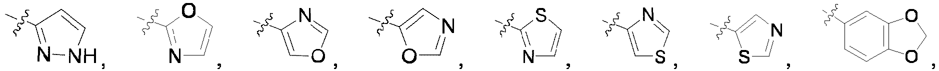 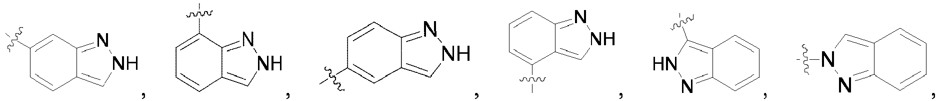 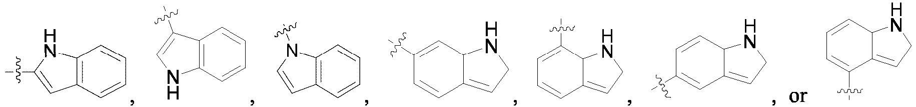 is optionally substituted with one or more F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano groups.

7. The compound or the pharmaceutically acceptable salt thereof according to any one of claims 1-6,
wherein R₁ is selected from the group consisting of 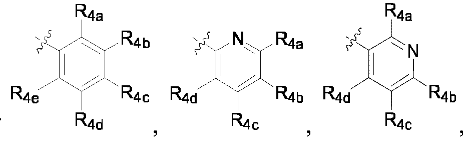 and 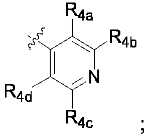
R₄ₐ, R_{4b}, R_{4c}, R_{4d}, and R₄ₑ are each independently selected from the group consisting of H, F, Cl, Br, I, methyl, ethyl, n-propyl, isopropyl, fluoromethyl, chloromethyl, difluoromethyl, dichloromethyl, trifluoromethyl, trichloromethyl, 2,2-difluoroethyl, 2,2-dichloroethyl, 2,2,2-trifluoroethyl, 2,2,2-trichloroethyl, methoxy, ethoxy, n-propoxy, isopropoxy, fluoromethoxy, chloromethoxy, difluoromethoxy, dichloromethoxy, trifluoromethoxy, trichloromethoxy, 2,2-difluoroethoxy, 2,2-dichloroethoxy, 2,2,2-trifluoroethoxy, 2,2,2-trichloroethoxy, -(C=O)NH₂, -NH₂, or cyano;
R₂ is H;
R₃ is methyl.

8. The compound or the pharmaceutically acceptable salt thereof according to claim 1, wherein the compound is selected from the group consisting of:

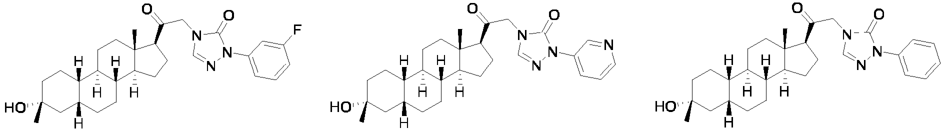

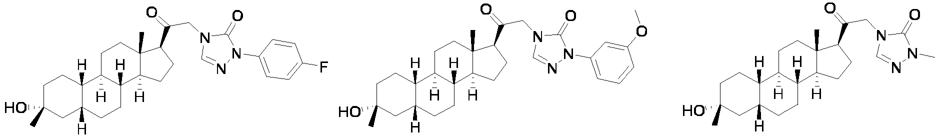

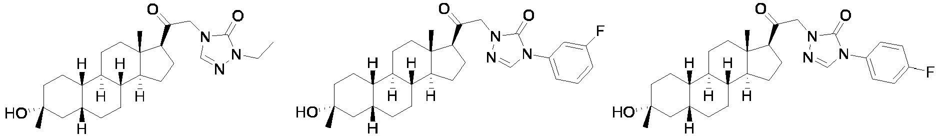

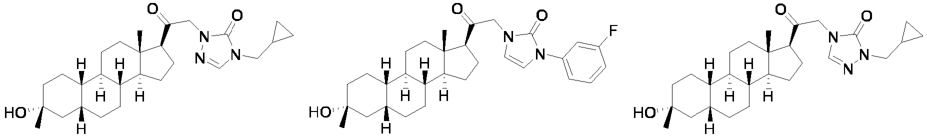

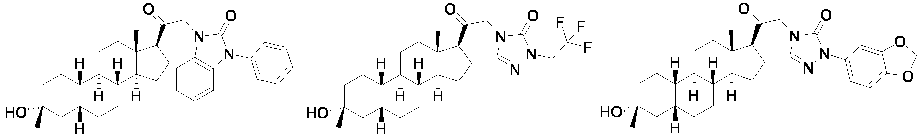

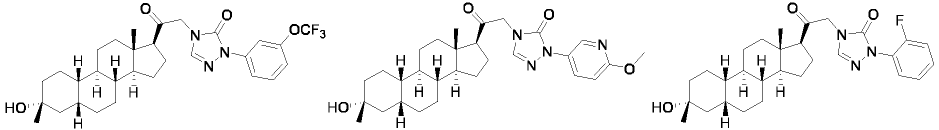

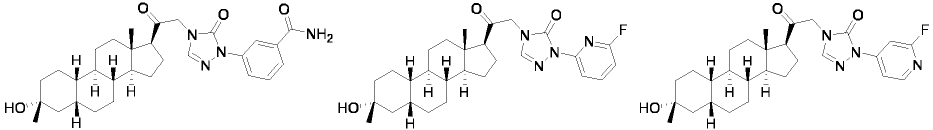

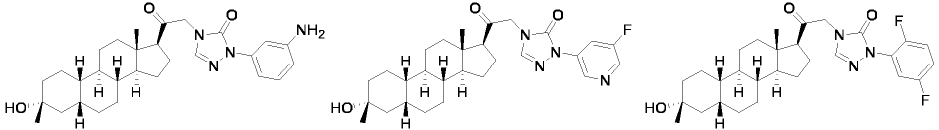

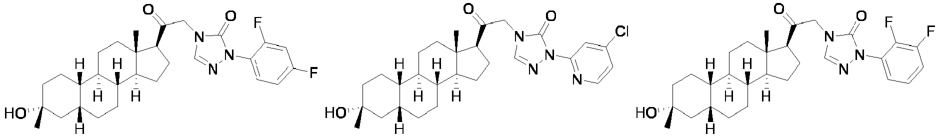

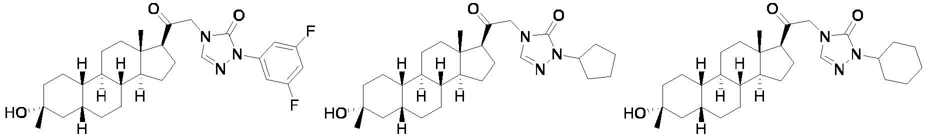

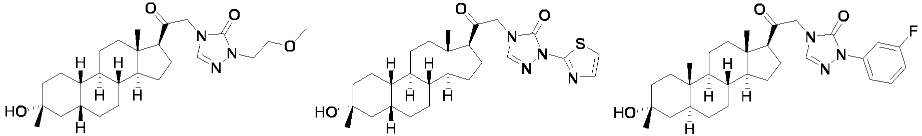

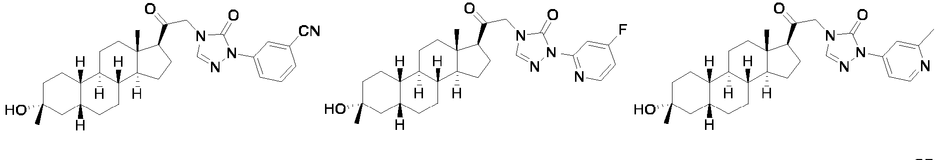

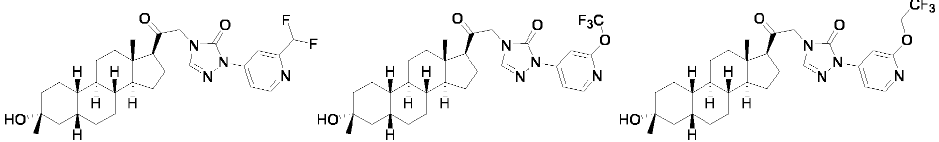

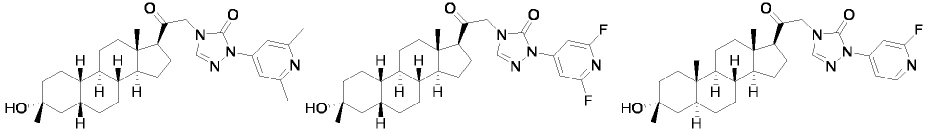

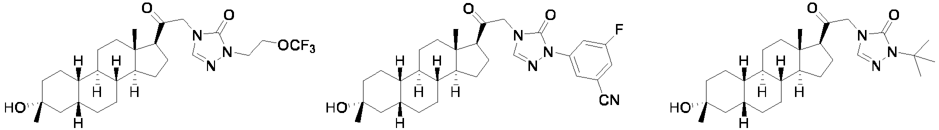

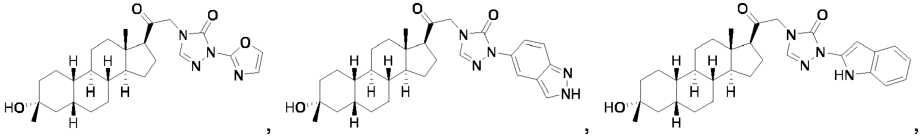

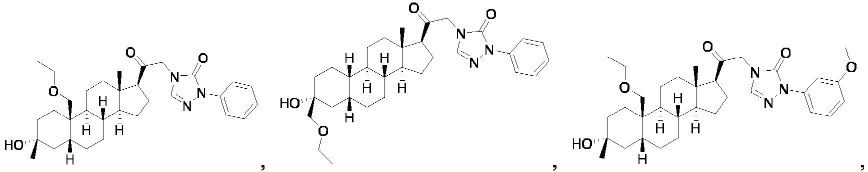

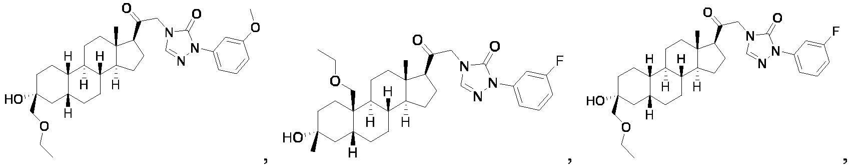

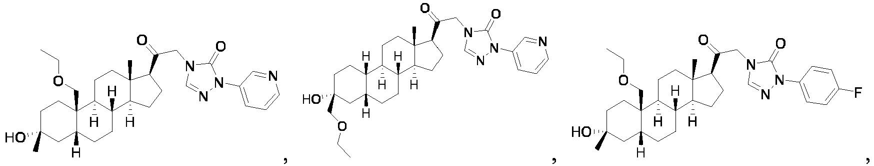

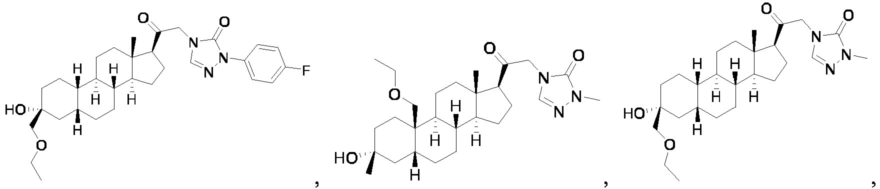

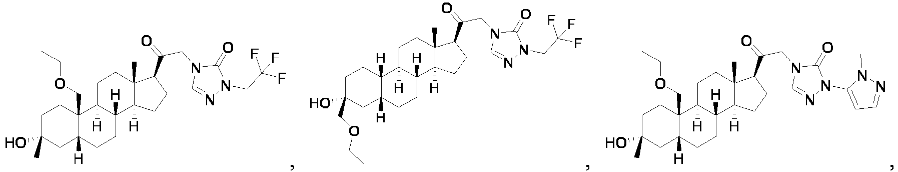

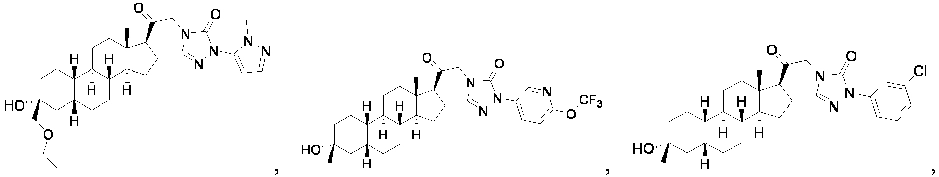

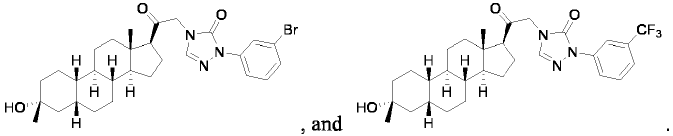

9. A pharmaceutical composition, comprising the compound or the pharmaceutically acceptable salt thereof according to any one of claims 1-8 and a pharmaceutically acceptable carrier.

10. Use of the compound or the pharmaceutically acceptable salt thereof according to any one of claims 1-8 or the pharmaceutical composition according to claim 9 in the manufacture of a GABAA receptor regulator medicament.

11. Use of the compound or the pharmaceutically acceptable salt thereof according to any one of claims 1-8 or the pharmaceutical composition according to claim 9 in the manufacture of a medicament for treating CNS-related diseases.

12. The use according to claim 11, wherein the CNS-related diseases include: sleep disorder, mood disorder, mania, dysthymic disorder, bipolar disorder, anxiety disorder, stress, post-traumatic stress disorder (PTSD), compulsive disorder, schizophrenia spectrum disorder, convulsive disorder, disorders of memory and/or cognition, dementia, movement disorder, personality disorder, autism, autism spectrum disorder (ASD), pain, traumatic brain injury (TBI), vascular diseases, substance abuse disorder and/or withdrawal syndrome, and tinnitus.

13. The use according to claim 12, wherein the CNS-related diseases include depression.

14. The use according to claim 13, wherein the depression includes minor depression, major depressive disorder, persistent depressive disorder, psychotic depression, postpartum depression, or seasonal affective disorder.

15. The use according to claim 12, wherein the CNS-related diseases include insomnia, bipolar I disorder, bipolar II disorder, generalized anxiety disorder (GAD), social anxiety disorder, schizophrenia, schizoaffective disorder, attention disorder, dementia of the Alzheimer type, dementia with Lewy bodies, vascular dementia, Huntington's disease, Parkinson's disease, essential tremor, antisocial personality disorder, obsessive-compulsive personality disorder, autism, monogenetic causes of autism, synaptophathy, Rett syndrome, fragile X syndrome, Angelman syndrome, neuropathic pain, injury-related pain syndrome, acute pain, chronic pain, stroke, ischemia, vascular malformation, and addiction to opiates, cocaine, and/or alcohol.
